(19) Europäisches Patentamt / European Patent Office / Office européen des brevets

(11) **EP 4 186 895 A1**

(12) **EUROPEAN PATENT APPLICATION**
published in accordance with Art. 153(4) EPC

(43) Date of publication:
**31.05.2023 Bulletin 2023/22**

(21) Application number: **21845747.1**

(22) Date of filing: **26.07.2021**

(51) International Patent Classification (IPC):
***C07D 401/14*** (2006.01) ***C07D 403/14*** (2006.01)
***C07D 413/14*** (2006.01) ***C07D 487/04*** (2006.01)
***C07F 9/6558*** (2006.01) ***C07F 9/6561*** (2006.01)
***C07F 9/6568*** (2006.01) ***C07F 9/6574*** (2006.01)
***A61P 3/00*** (2006.01) ***A61P 9/00*** (2006.01)
***A61P 29/00*** (2006.01) ***A61P 31/00*** (2006.01)
***A61P 35/00*** (2006.01) ***A61P 35/02*** (2006.01)
***A61P 37/02*** (2006.01) ***A61K 31/675*** (2006.01)
***A61K 31/5025*** (2006.01) ***A61K 31/506*** (2006.01)

(52) Cooperative Patent Classification (CPC):
**A61K 31/5025; A61K 31/506; A61K 31/675; A61P 3/00; A61P 9/00; A61P 29/00; A61P 31/00; A61P 35/00; A61P 35/02; A61P 37/02; C07D 401/14; C07D 403/14; C07D 413/14; C07D 487/04; C07F 9/6558;** (Cont.)

(86) International application number:
**PCT/CN2021/108429**

(87) International publication number:
**WO 2022/017533 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2020 CN 202010722999**
**29.01.2021 CN 202110129797**

(71) Applicant: **TYK Medicines Inc.**
**Huzhou, Zhejiang 313100 (CN)**

(72) Inventors:
- **LI, Jun**
  **Huzhou, Zhejiang 313100 (CN)**
- **LIANG, Apeng**
  **Huzhou, Zhejiang 313100 (CN)**
- **WU, Yusheng**
  **Huzhou, Zhejiang 313100 (CN)**
- **DONG, Shengli**
  **Huzhou, Zhejiang 313100 (CN)**
- **LI, Meihua**
  **Huzhou, Zhejiang 313100 (CN)**
- **NIU, Chengshan**
  **Huzhou, Zhejiang 313100 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **COMPOUND USEFUL AS CDK7 KINASE INHIBITOR AND USE THEREOF**

(57) The present invention relates to a compound useful as a CDK7 kinase inhibitor and use thereof. Specifically, the compound of the present invention has a structure shown in formula I, wherein the definition of each group and substituent is as described in the description. The compound of the present invention can be used as an inhibitor of cyclin-dependent kinase 7 (CDK7) for the treatment or prevention of proliferative diseases (such as cancers), and in particular for the regulation and treatment of related diseases caused by abnormal activity of cyclin-dependent kinase 7 (CDK7).



Processed by Luminess, 75001 PARIS (FR)

(Cont. next page)

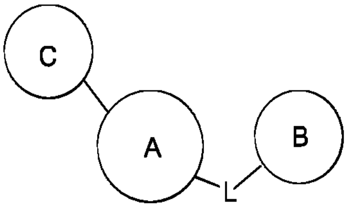


I

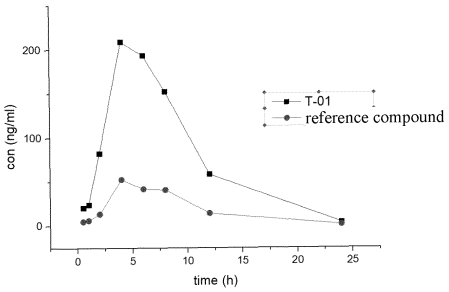


Figure 1

(52) Cooperative Patent Classification (CPC): (Cont.)
**C07F 9/6561; C07F 9/6568; C07F 9/6574**

## Description

### TECHNICAL FIELD

The invention relates to the technical field of medicine, in particular to compounds used as CDK7 kinase inhibitors, and its application in regulating CDK7 kinase activity or treating CDK7 related diseases, especially cancer.

### BACKGROUND ART

CDKs (Cyclin-dependent kinases) belong to the serine/threonine kinase family, and they exert physiological functions by combining with the corresponding cyclins to form an active dimer complex, causing cell growth and proliferation. At present, more than 20 kinds of CDKs have been found, which are divided into two categories according to their functions: CDKs that regulate cell cycle and CDKs that regulate cell transcription, wherein CDKs 1-6 and 14-18 participate in cell cycle regulation, and CDKs 7-13 and 19-20 participate in cell transcription regulation.

CDK7 is an important member of CDKs family, and its main physiological function is to regulate cell cycle and transcription. In the cell sap, CDK7 together with cyclin H and Mat1 form CAK(CDKs activating kinase), which participates in the regulation of cell cycle by phosphorylation of CDK1/2/4/6. In the nucleus, CDK7, as a component of the universal transcription factor TF II H(Transcription factor II human), participates in the gene transcription process of cells through the phosphorylation of CTD domain (carboxy-terminal domain) of RNA polymerase II at the most important initial stage of gene transcription. Because CDK7 has the dual functions of CAK and CTD phosphorylation, it plays an important role in cell proliferation, cell cycle and gene transcription.

Due to the dual unique function of CDK7 in transcription and cell cycle progression, CDK7 is widely expressed in various types of cancer, down-regulation of CDK7 activity can lead to a decrease in cell proliferation. More importantly, it is now agreed that targeted transcription can selectively restrict the synthesis of mRNA involved in tumor growth without causing interruption of housekeeping genes transcription. Therefore, CDK7 is considered to be a feasible and very promising target for tumor therapy, which has attracted widespread attention. Many small molecules, such as THZ1, THZ2, CT7001, SY-1365, etc., have shown very good inhibitory effects on tumor growth in preclinical studies. Especially in small cell lung cancer, triple-negative breast cancer, pancreatic cancer and other unsatisfied major diseases that lack effective treatment methods at current. Therefore, the development of specific CDK7 inhibitors is expected to be used in the above clinically unmet areas.

### SUMMARY OF THE INVENTION

The invention provides a new compound with CDK7 kinase inhibitory activity, better pharmacodynamics and pharmacokinetic properties.

In the first aspect of the present invention, it provides a compound used as a CDK7 kinase inhibitor, the compound is a compound of formula I, or its pharmaceutically acceptable salt, stereoisomer, tautomer, hydrate, solvate, isotope compound or prodrug,

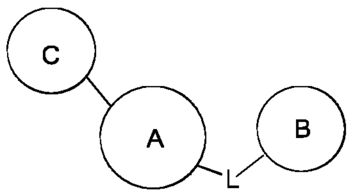

I

wherein:

ring A is selected from the group consisting of

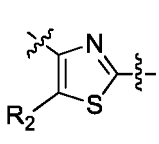 , 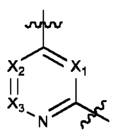 , 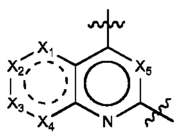 ,

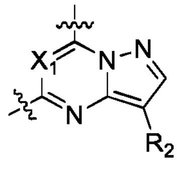, and 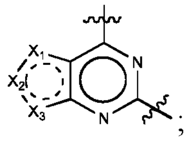;

ring B is selected from the substituted or unsubstituted group consisting of C3-C8 cycloalkyl, 4-7 membered heterocyclyl containing 1-3 groups selected from N, O, S, S(O) or $S(O)_2$, 5-9 membered bridged heterocycloalkyl containing 1-3 groups selected from N, O, S, S(O) or $S(O)_2$, 5-9 membered bridged cycloalkyl, 6-10 membered spiro heterocycloalkyl containing 1-3 groups selected from N, O, S, S(O) or $S(O)_2$, 6-10 membered spiro cycloalkyl, 6-10 membered fused heterocycloalkyl containing 1-3 groups selected from N, O, S, S(O) or $S(O)_2$, 6-10 membered fused cycloalkyl, 5-6 membered heteroaryl containing 1-3 groups selected from N, O, S, S(O) or $S(O)_2$ and 5-6 membered aryl;

ring C is selected from the group consisting of

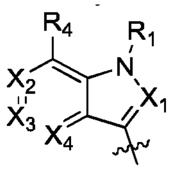, 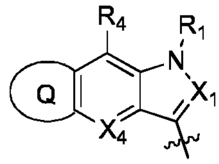,

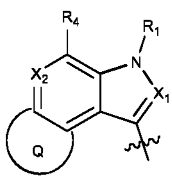, 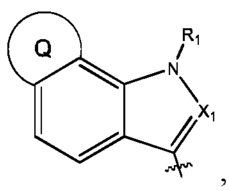, and 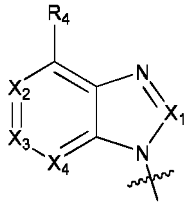;

L is selected from the group consisting of O, S(O), $S(O)_2$, $NR_1$, $-NR_1$-(C1-C6 alkylene)-, $CR_2R_3$, C=NH, and C=NOH;

each $X_1$, $X_2$, $X_3$, $X_4$ and $X_5$ is independently selected from the group consisting of chemical bonds, N, O, S, $NR_1$, $CR_2$, and $CR_2R_3$;

$X_1$, $X_2$, $X_3$ or $X_1$, $X_2$, $X_3$, $X_4$ together with the pyrimidine ring to which they are fused form aromatic rings or non-aromatic rings;

each $R_1$ is independently selected from the substituted or unsubstituted group consisting of hydrogen, amino, -C(=O)-O-(C1-C6 alkyl), C1-C6 alkyl, C2-C6 alkenyl, C2-C6 alkynyl, C1-C6 haloalkyl, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, C6-C10 aryl-C1-C6 alkylene, and 3-8 membered heterocycloalkyl containing 1, 2 or 3 heteroatoms selected from N, O or S;

each $R_2$ and $R_3$ is independently selected from the substituted or unsubstituted group consisting of hydrogen, halogen, amino, hydroxyl, cyano, oxo (= O), carboxyl, -C(=O)-O-(C1-C6 alkyl), -NH-C(=O)-O-(C1-C6 alkyl), -O-C(=O)-NH-(C1-C6 alkyl), -O-C(=O)-NR5-(C1-C6 alkyl), carbamate, C1-C6 alkyl, C1-C6 haloalkyl, C2-C6 alkenyl, C2-C6 alkynyl, C1-C6 alkoxy, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 5-10 membered heteroaryl-NH- containing 1, 2 or 3 heteroatoms selected from N, O or S, C6-C10 aryloxy, C6-C10 aryl-C1-C6 alkylene, 3-8 membered heterocycloalkyl containing 1, 2 or 3 heteroatoms selected from N, O or S, C1-C6 alkyl-NH-, and C3-C6 cycloalkyl-NH-;

$R_4$ is selected from the group consisting of H,

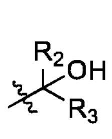, 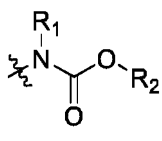, 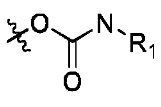,

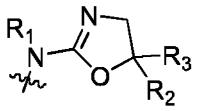, 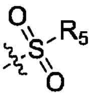, 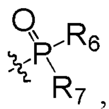, and 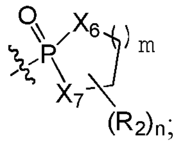;

each $R_5$, $R_6$, and $R_7$ is independently selected from the group consisting of C1-C6 alkyl, C1-C6 haloalkyl, C3-C6 cycloalkyl, C3-C6 halocycloalkyl, 3-8 membered heterocycloalkyl containing 1, 2 or 3 heteroatoms selected from N, O or S, C6-C10 aryl, and 5-10 membered heteroaryl containing 1, 2 or 3 heteroatoms selected from N, O or S;
each $X_6$ and $X_7$ is independently selected from the group consisting of O, $CR_2R_3$ and $NR_1$;
ring Q is a substituted or unsubstituted 4-7 membered ring containing 0, 1, 2 or 3 heteroatoms selected from O, N, S or P;
m is selected from the group consisting of 0, 1, 2 and 3;
each n is independently selected from the group consisting of 0, 1, 2, 3, 4 and 5;
the substituted independently means to be substituted by a group selected from the group consisting of -NH-C(=O)-O-(C1-C6 alkyl), amino, halogenated or unsubstituted C1-C6 alkyl and =O;
provided that: when ring A is

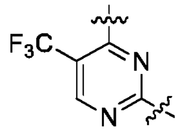

and ring B is piperidine ring or piperidine ring substituted by methyl, carboxyl or amido-, then ring C is not

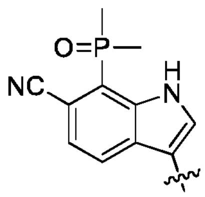.

In another preferred embodiment,

ring A is selected from the group consisting of

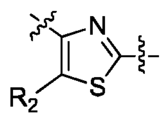, 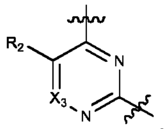, 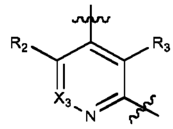,

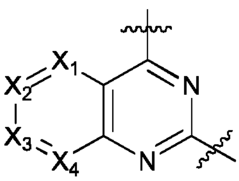, 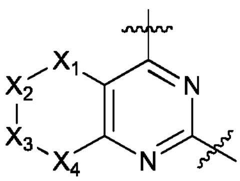, 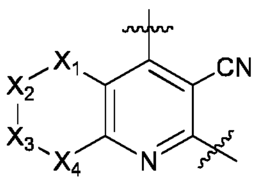, 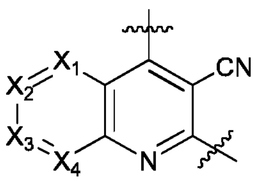,

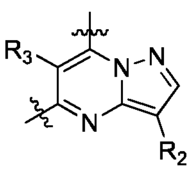, 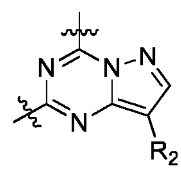, 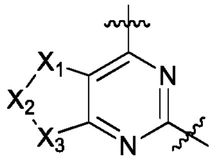, 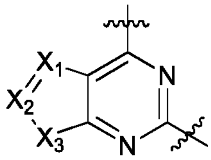, and 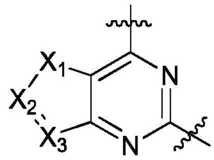;

ring B is selected from the group consisting of

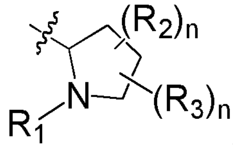 , 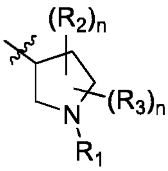 ,

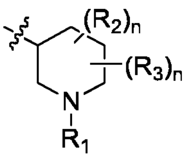 , 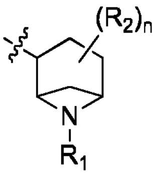 , 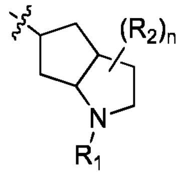 , 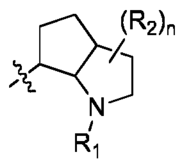 , 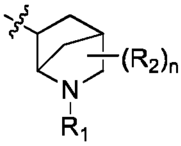 , 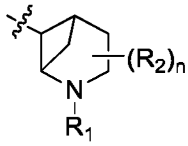 ,

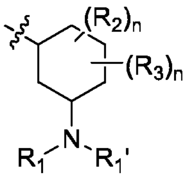 , 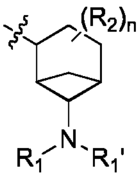 , 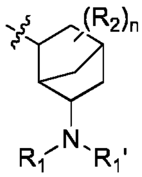 , 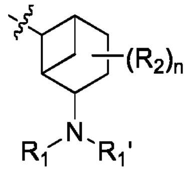 , 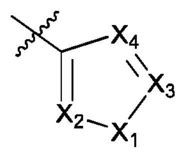 , 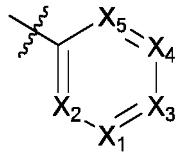 ,

and

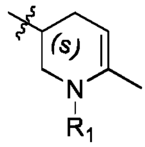 ;

ring C is selected from the group consisting of

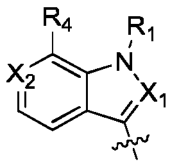 , 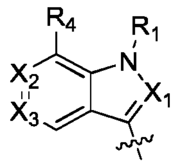 ,

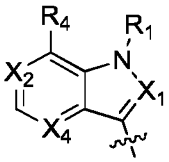 , 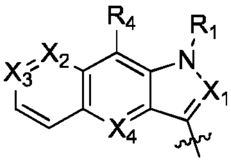 , 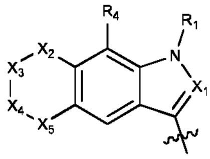 , 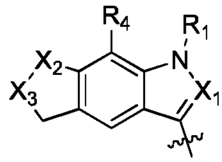 , 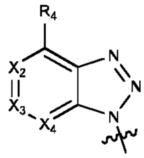 ,

and

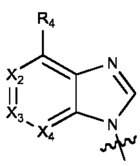 ;

each $X_1$, $X_2$, $X_3$, $X_4$ and $X_5$ is independently selected from the group consisting of chemical bonds, N, O, S, $NR_1$, $CR_2$, and $CR_2R_3$;

$X_1$, $X_2$, $X_3$ or $X_1$, $X_2$, $X_3$, $X_4$ together with pyrimidine rings to which they are fused form aromatic rings or non-aromatic rings;

each $R_1$ is independently selected from the substituted or unsubstituted group consisting of hydrogen, amino, -C(=O)-O-(C1-C6 alkyl), C1-C6 alkyl, C2-C6 alkenyl, C2-C6 alkynyl, C1-C6 haloalkyl, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, C6-C10 aryl-C1-C6 alkylene, and 3-8 membered heterocycloalkyl containing 1, 2 or 3 heteroatoms selected from N, O or S;

each $R_2$ and $R_3$ is independently selected from the substituted or unsubstituted group consisting of hydrogen, halogen, amino, hydroxyl, cyano, oxo (= O), carboxyl, -C(=O)-O-(C1-C6 alkyl), -NH-C(=O)-O-(C1-C6 alkyl), -O-C(=O)-NH-(C1-C6 alkyl), -O-C(=O)-NR5-(C1-C6 alkyl), carbamate, C1-C6 alkyl, C1-C6 haloalkyl, C2-C6 alkenyl, C2-C6 alkynyl, C1-C6 alkoxy, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 5-10 membered heteroaryl-NH- containing 1, 2 or 3 heteroatoms selected from N, O or S, C6-C10 aryloxy, C6-C10 aryl-C1-C6 alkylene, 3-8 membered heterocycloalkyl containing 1, 2 or 3 heteroatoms selected from N, O or S, C1-C6 alkyl-NH-, and C3-C6 cycloalkyl-NH-;

or $R_2$ and $R_3$ together with the carbon atoms on the ring form a 3-7 membered ring;

$R_1$' is selected from the group consisting of hydrogen, amino, -C(=O)-O-C1-C6 alkyl, C1-C6 alkyl, C2-C6 alkenyl, C2-C6 alkynyl, C1-C6 haloalkyl, C1-C6 heteroalkyl, C3-C6 cycloalkyl, C3-C6 halocycloalkyl, C6-C10 aryl, 6-10 membered heteroaryl containing 1, 2 or 3 heteroatoms selected from N, O or S, C6-C10 aryl-C1-C6 alkylene, and 3-8 membered heterocycloalkyl containing 1, 2 or 3 heteroatoms selected from N, O or S;

each n is independently selected from the group consisting of 0, 1, 2, 3, 4 and 5;

$R_4$ is defined as above.

In another preferred embodiment,

ring A is selected from the group consisting of

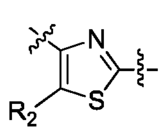, 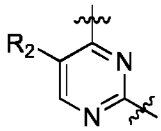, 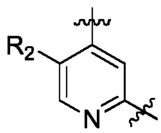,

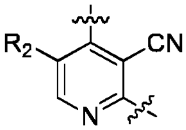, 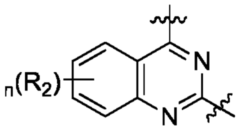, 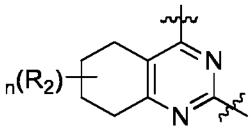, 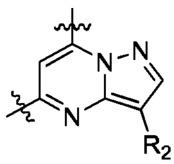, 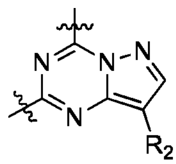,

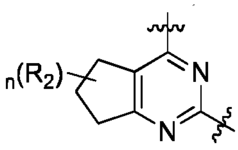, 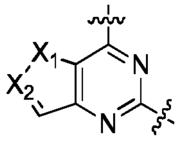, 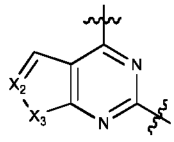, and 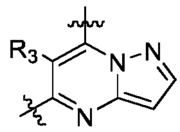;

ring B is selected from the group consisting of

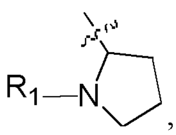, 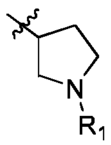, 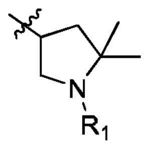,

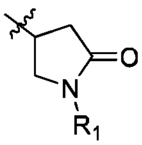, 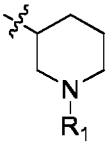, 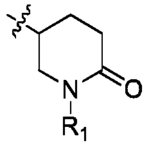, 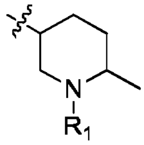, 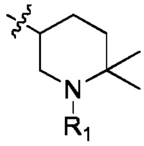, 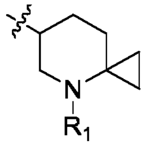, 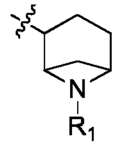,

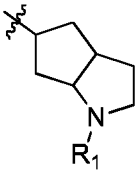, 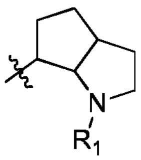, 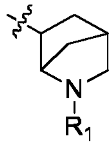, 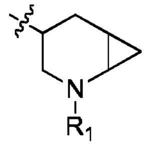, 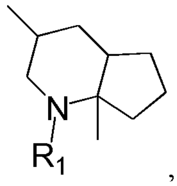, 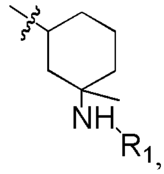, 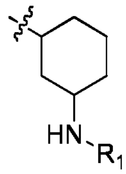,

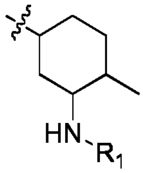, 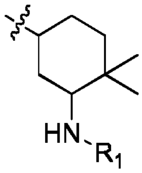, 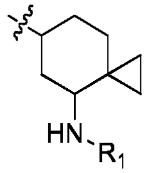, 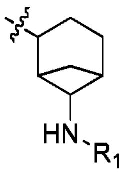, 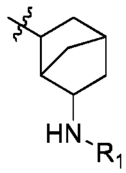, 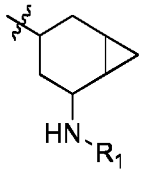, 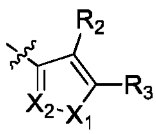,

and

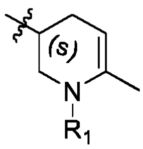;

each n is independently selected from the group consisting of 0, 1, 2, 3, 4 and 5.

In another preferred embodiment, ring A is selected from the group consisting of

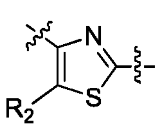, 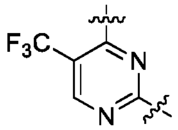, 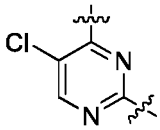,

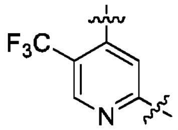, 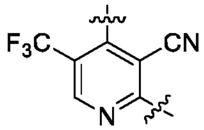, 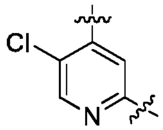, 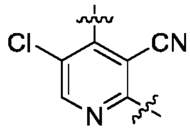, 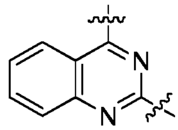, 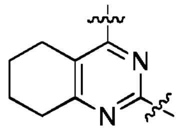,

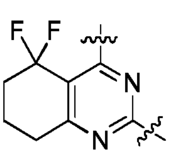, 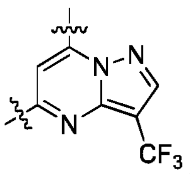, 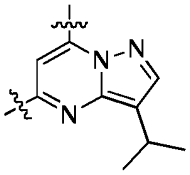, 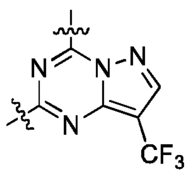, 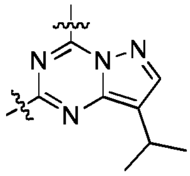, 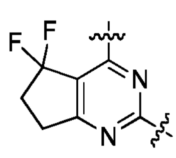,

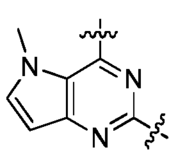, 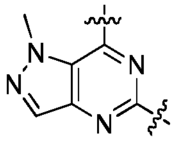, 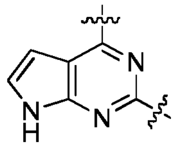, 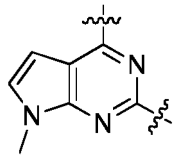, 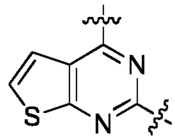, 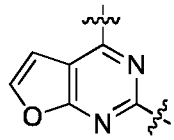,

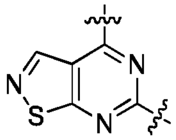, 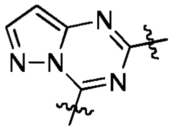, and 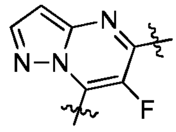.

In another preferred embodiment, ring A is

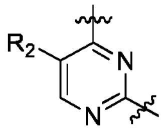,

wherein $R_2$is selected from the group consisting of halogen, C1-C6 alkyl, and C1-C6 haloalkyl;

ring B is selected from the substituted or unsubstituted group consisting of C3 -C8 cycloalkyl, 4-7 membered heterocyclyl containing 1-3 groups selected from N, O, S, S(O) or $S(O)_2$, 5-9 membered bridged heterocycloalkyl containing 1-3 groups selected from N, O, S, S(O) or $S(O)_2$, 5-9 membered bridged cycloalkyl, 6-10 membered spiro heterocycloalkyl containing 1-3 groups selected from N, O, S, S(O) or $S(O)_2$, 6-10 membered spiro cycloalkyl, 6-10 membered fused heterocycloalkyl containing 1-3 groups selected from N, O, S, S(O) or $S(O)_2$, 6-10 membered fused cycloalkyl, 5-6 membered heteroaryl containing 1-3 groups selected from N, O, S, S(O) or $S(O)_2$ and C6-C10 aryl;

L is selected from the group consisting of O, $NR_1$ and $-NR_1$-(C1-C6 alkylene)-;

ring C is selected from the group consisting of

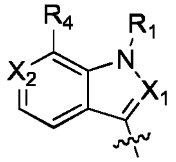 and 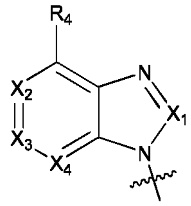;

wherein

$X_1$ is selected from the group consisting of N and CH;

each $X_2$, $X_3$ and $X_4$ is independently selected from the group consisting of N and $CR_2$';

each $R_2$' is independently selected from the substituted or unsubstituted group consisting of hydrogen, amino, -NH-C(=O)-O-(C1-C6 alkyl), $-NR_1$-C(=O)-O-(C1-C6 alkyl), $-NR_1$-C(=O)-$R_5$, $-NR_1$-C(=O)-O-C1-C6 alkylene-$X_8$-C1-C6 alkyl, -O-C(=O)-$R_5$, -O-C(=O)-NH-(C1-C6 alkyl), -O-C(=O)-NR5-(C1-C6 alkyl),

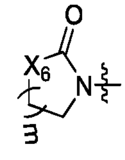, 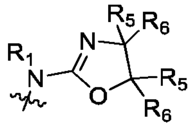, 3-8 membered heterocycloalkyl (linked to C by N) containing 1, 2 or 3 heteroatoms selected from N, O or S, C1-C6 alkyl-NH-, C3-C6 cycloalkyl-NH-, and hydroxyl;

$R_4$ is selected from the substituted or unsubstituted group consisting of H, halogen, C1-C6 alkyl, C1-C6 haloalkyl,

C2-C6 alkenyl, C2-C6 alkynyl, C1-C6 alkoxy, C1-C6 haloalkoxy, C1-C6 heteroalkyl, C3-C6 cycloalkyl, C3-C6 cycloalkoxy, C3-C6 halocycloalkyl, C6-C10 aryl, 5-10 membered heteroaryl containing 1, 2 or 3 heteroatoms selected from N, O or S, 3-8 membered heterocycloalkyl containing 1, 2 or 3 heteroatoms selected from N, O or S,

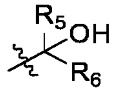 ,

$-NR_1-C(=O)-O-$(C1-C6 alkyl), -O-C(=O)-Rs, $-C(=O)-R_5$,

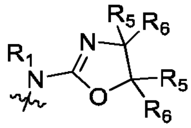 ,

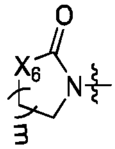 , 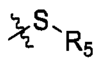 , 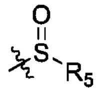 , 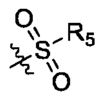 , 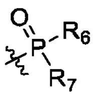 , 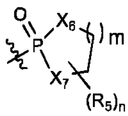 ,

and cyano;

$R_1$ is independently selected from the substituted or unsubstituted group consisting of hydrogen, amino, -C(=O)-O-(C1-C6 alkyl), C1-C6 alkyl, C1-C6 haloalkyl, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1, 2 or 3 heteroatoms selected from N, O or S, and $-C(=O)-NR_8R_9$;

each $R_5$, $R_6$ and $R_7$ is independently selected from the group consisting of hydrogen, C1-C6 alkyl, C1-C6 haloalkyl, C1-C6 alkyl containing 1, 2 or 3 heteroatoms selected from N, O or S, C3-C6 cycloalkyl, C3-C6 halocycloalkyl, C1-C6 alkylamino, C3-C6 cycloalkylamino, 3-8 membered heterocycloalkyl containing 1, 2 or 3 heteroatoms selected from N, O or S, C6-C10 aryl, 5-10 membered heteroaryl containing 1, 2 or 3 heteroatoms selected from N, O or S, and $NR_8R_9$;

each $R_8$ and $R_9$ is independently selected from the group consisting of hydrogen and C1-C6 alkyl;

each $X_6$ and $X_7$ is independently selected from the group consisting of O, $CR_5R_6$ and $NR_1$;

each $X_8$ is independently selected from the group consisting of N, O and S;

m is selected from the group consisting of 0, 1, 2 and 3;

each n is independently selected from the group consisting of 0, 1, 2, 3, 4 and 5;

the substituted independently means to be substituted by a group selected from the group consisting of -NH-C(=O)-O-(C1-C6 alkyl), amino, halogenated or unsubstituted C1-C6 alkyl, =O, hydroxyl, and -NH(C1-C6 alkyl).

In another preferred embodiment, ring A is selected from the group consisting of

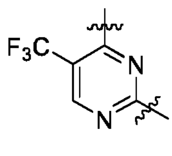 , 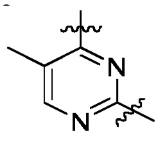 , 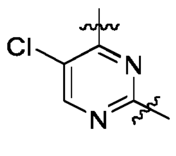 , 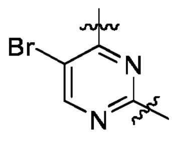 , 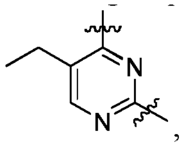 , and 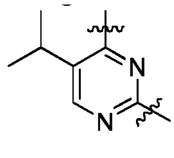 ;

ring B is selected from the substituted or unsubstituted group consisting of C3 -C8 cycloalkyl, 4-7 membered heterocyclyl containing 1-3 groups selected from N, O, S, S(O) or $S(O)_2$, 5-9 membered bridged heterocycloalkyl containing 1-3 groups selected from N, O, S, S(O) or $S(O)_2$, 5-9 membered bridged cycloalkyl, 6-10 membered spiro heterocycloalkyl containing 1-3 groups selected from N, O, S, S(O) or $S(O)_2$, 6-10 membered spiro cycloalkyl, 6-10 membered fused heterocycloalkyl containing 1-3 groups selected from N, O, S, S(O) or $S(O)_2$, 6-10 membered fused cycloalkyl, 5-6 membered heteroaryl containing 1-3 groups selected from N, O, S, S(O) or $S(O)_2$ and 5-6 membered aryl;

L is selected from the group consisting of O, $NR_1$ and $-NR_1-$(C1-C6 alkylene)-;

ring C is selected from the group consisting of

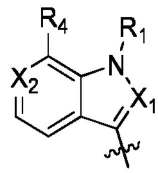 and 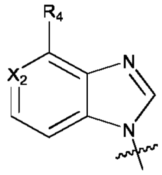;

wherein, $R_1$, $R_4$, $X_1$, and $X_2$ are as defined in claim 1.

In another preferred embodiment, ring B is selected from the group consisting of

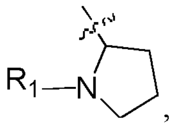, 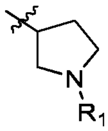, 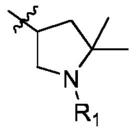, 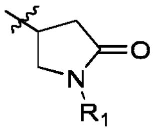, 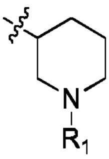, 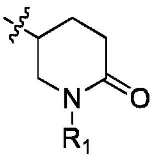, 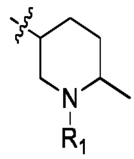,

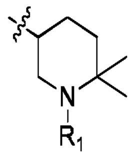, 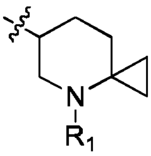, 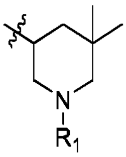, 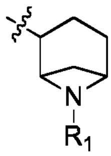, 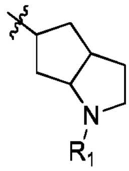, 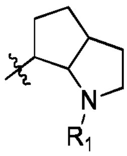, 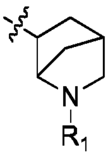, 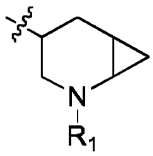,

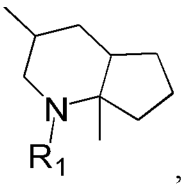, 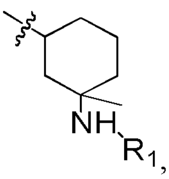, 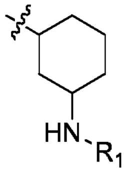, 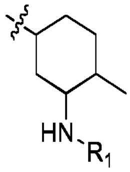, 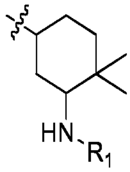, 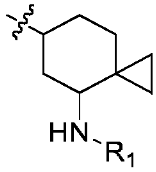, 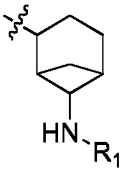,

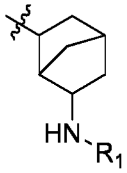, 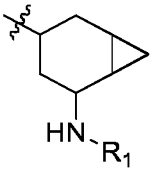, 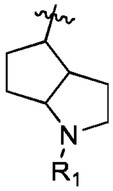, 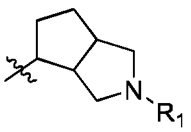, 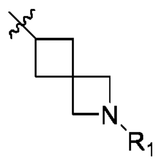, 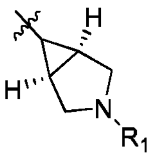, 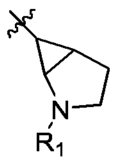,

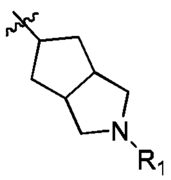, 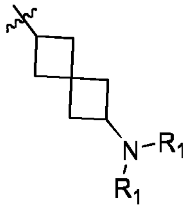, 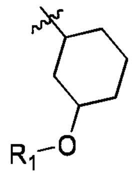, 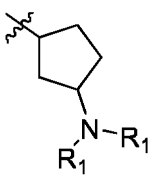, 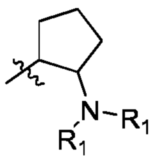, and 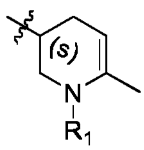;

ring C is selected from the group consisting of

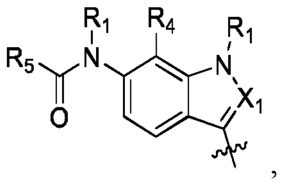,

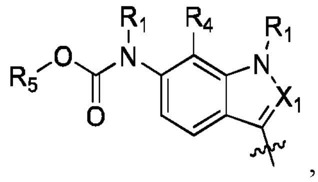, 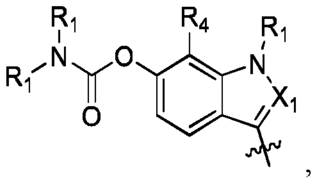, 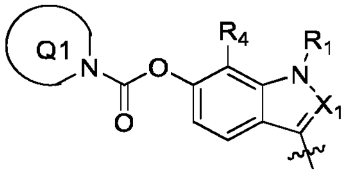,

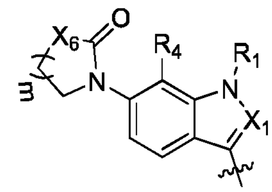, 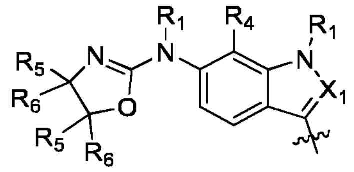, and 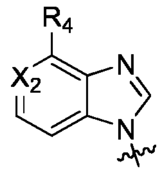;

wherein, $R_1$, $R_4$, $R_5$, $R_6$, $X_1$, $X_2$, $X_6$, and m are as defined in claim 1, and $Q_1$ is a 3-7 membered ring.

In another preferred embodiment, ring A is selected from the group consisting of

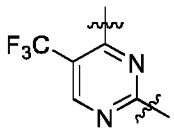, 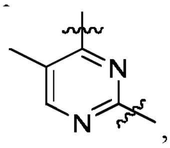, 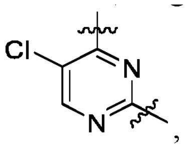, and 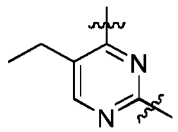;

ring C is selected from the group consisting of

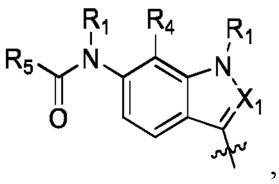,

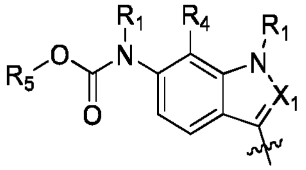, 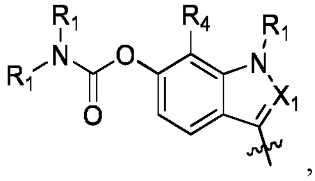, 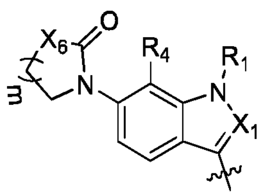;

and

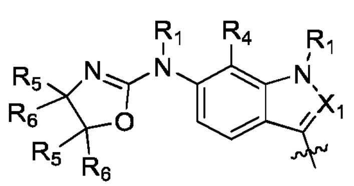;

R4is selected from the group consisting of H, halogen, C1-C6 alkyl, C1-C6 haloalkyl, C2-C6 alkenyl, C1-C6 alkoxy,

C1-C6 haloalkoxy, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1 2 or 3 heteroatoms selected from N, O or S,

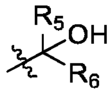, 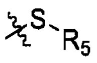, 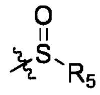, 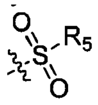, 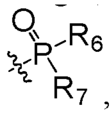,

and

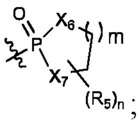;

wherein, $R_1$, $R_5$, $R_6$, $R_7$, $X_1$, $X_6$, $X_7$, m, and n are as defined in claim 1. In another preferred embodiment, ring A is selected from the group consisting of

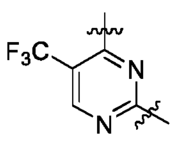, 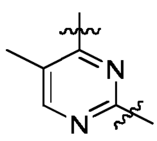, and 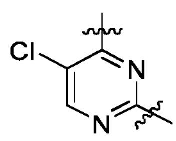;

ring C is selected from the group consisting of

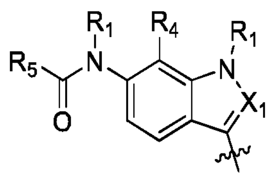,

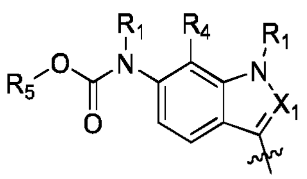, 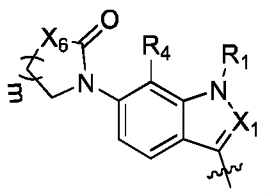, and 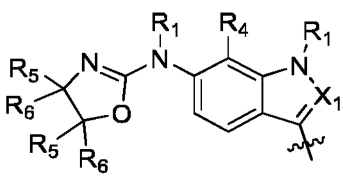;

$R_4$ is selected from the group consisting of H, halogen, C1-C6 alkyl, C1-C6 haloalkyl, C2-C6 alkenyl, C1-C6 alkoxy, C1-C6 haloalkoxy, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1 2 or 3 heteroatoms selected from N, O or S,

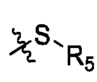, 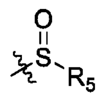, 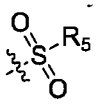, and 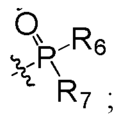;

L is selected from the group consisting of O and $NR_1$;
wherein $R_1$, $R_5$, $R_6$, $R_7$, $X_1$, $X_6$, and m are as defined above.

In another preferred embodiment, $R_4$ is selected from the group consisting of H, halogen, C1-C6 alkyl, C1-C6 haloalkyl, C2-C6 alkenyl, C1-C6 alkoxy, C1-C6 haloalkoxy, C3-C6 cycloalkyl, C3-C6 halocycloalkyl, C6-C10 aryl, 5-10

membered heteroaryl containing 1, 2 or 3 heteroatoms selected from N, O or S, and 3-8 membered heterocycloalkyl containing 1, 2 or 3 heteroatoms selected from N, O or S;

L is NH.

In another preferred embodiment, the compound is selected from the group consisting of:

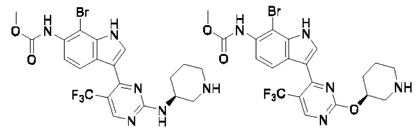
T-01 T-02

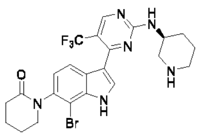
T-03

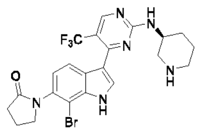
T-04

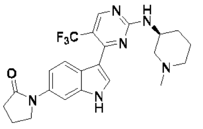
T-05

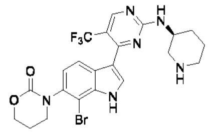
T-06

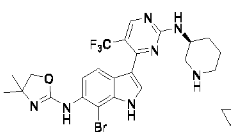
T-07

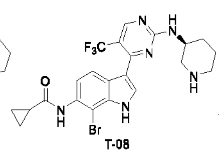
T-08

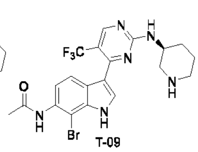
T-09

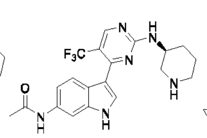
T-10

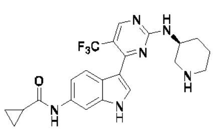
T-11

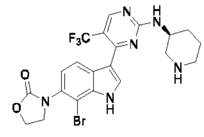
T-12

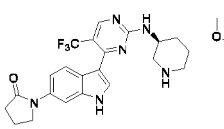
T-13

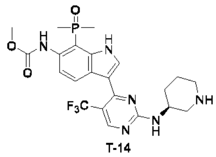
T-14

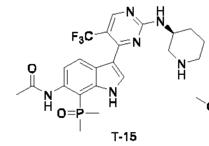
T-15

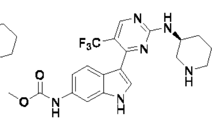
T-16

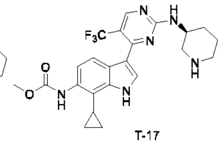
T-17

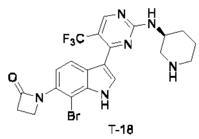
T-18

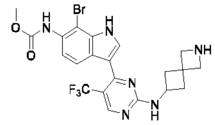
T-19

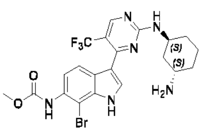
T-20

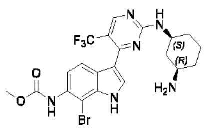
T-21

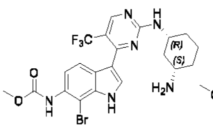
T-22

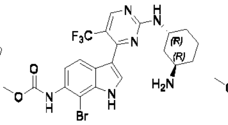
T-23

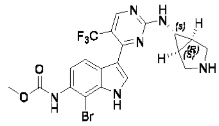
T-24

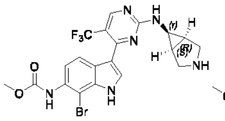
T-25

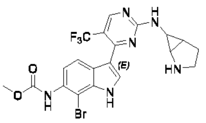
T-26

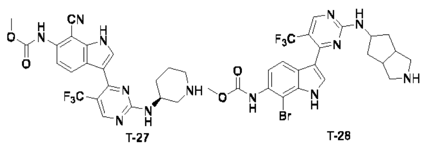
T-27 T-28

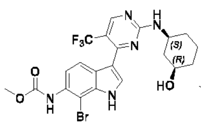
T-29

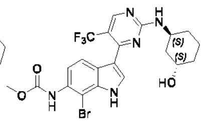
T-30

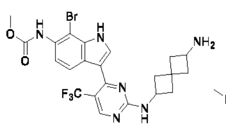
T-31

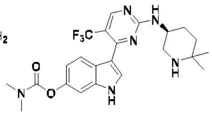
T-32

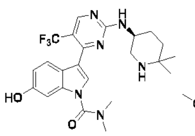
T-33

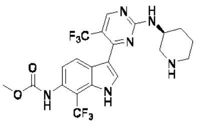
T-34

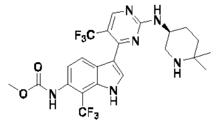
T-35

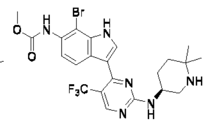
T-36

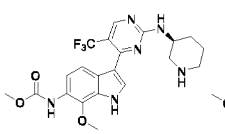
T-37

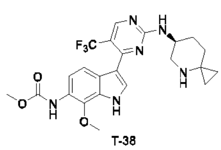
T-38

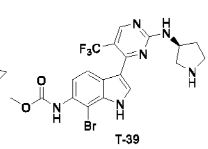
T-39

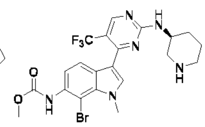
T-40

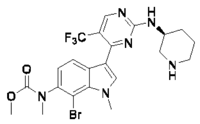
T-41

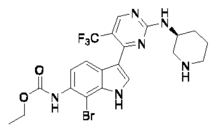
T-42

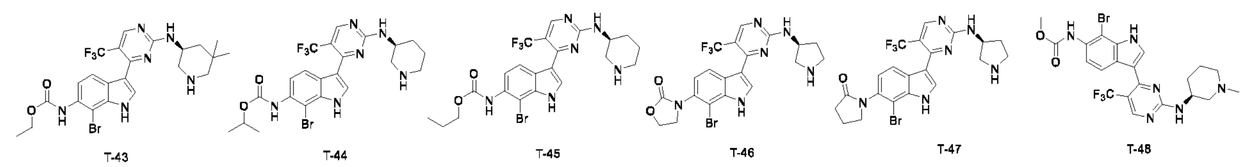
T-43 T-44 T-45 T-46 T-47 T-48

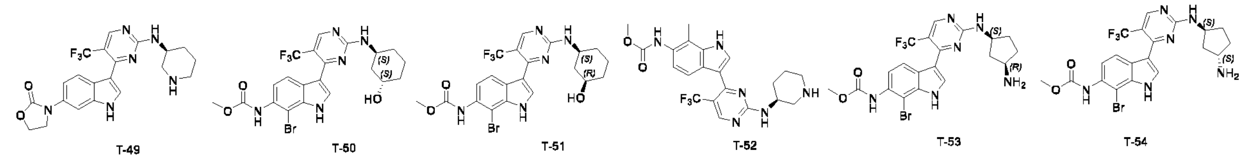
T-49 T-50 T-51 T-52 T-53 T-54

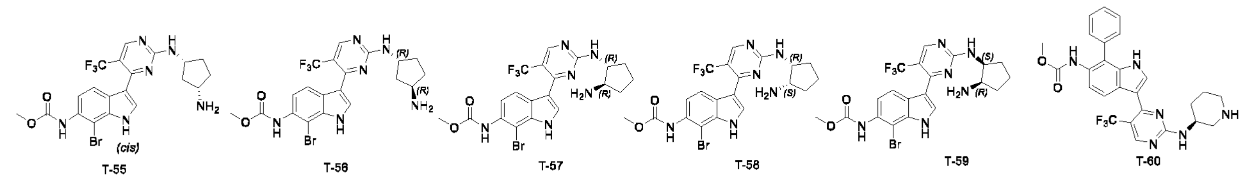
T-55 T-56 T-57 T-58 T-59 T-60

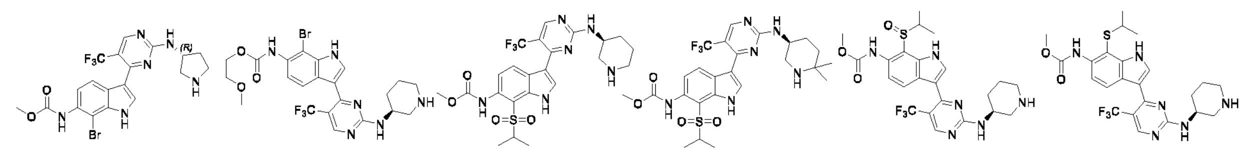
T-61 T-62 T-63 T-64 T-65 T-66

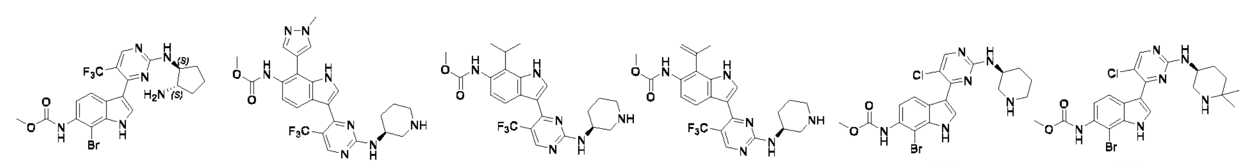
T-67 T-68 T-69 T-70 T-71 T-72

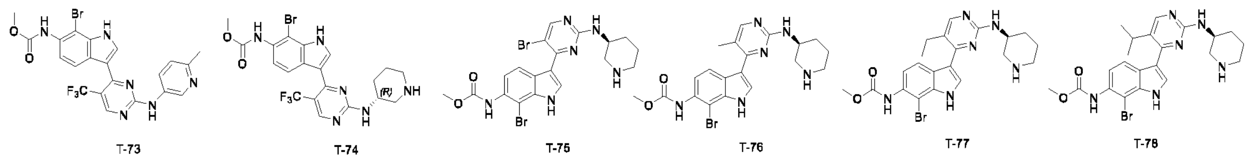
T-73 T-74 T-75 T-76 T-77 T-78

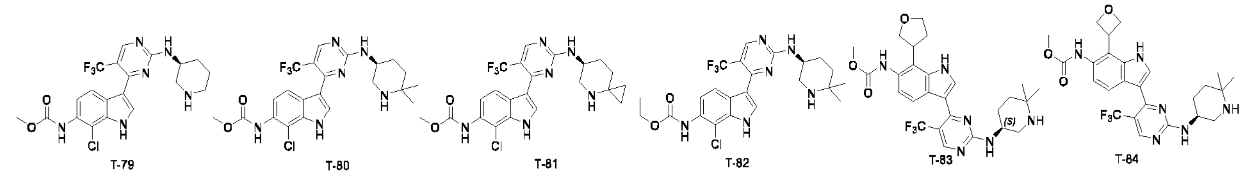
T-79 T-80 T-81 T-82 T-83 T-84

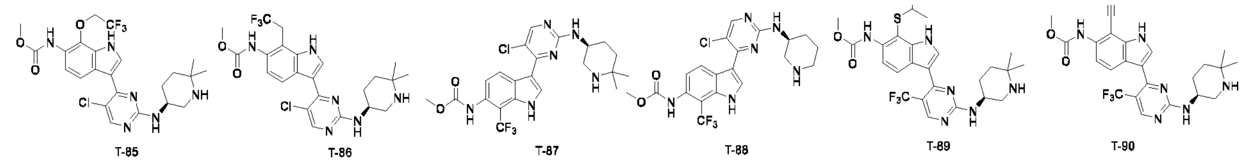
T-85 T-86 T-87 T-88 T-89 T-90

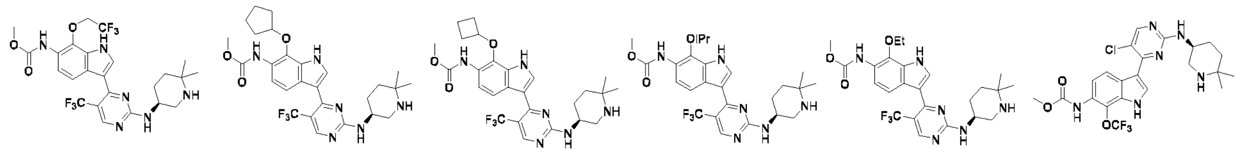

T-91 T-92 T-93 T-94 T-95 T-96

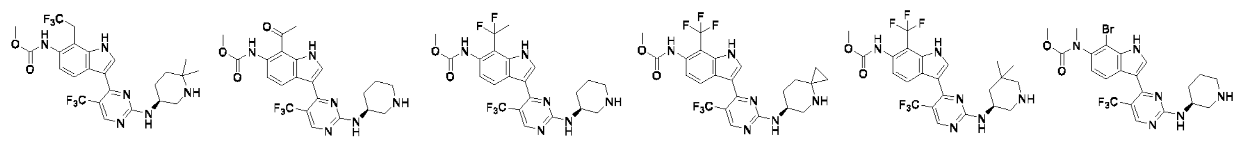

T-97 T-98 T-99 T-100 T-101 T-102

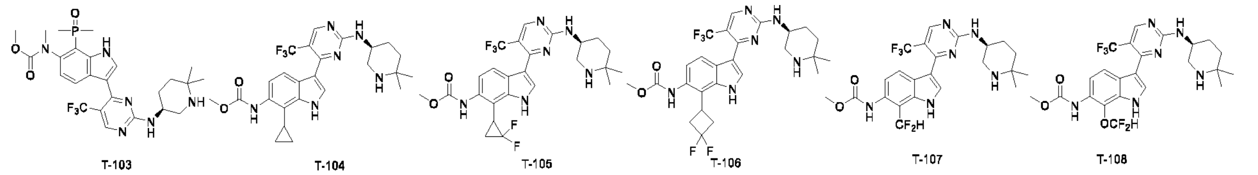

T-103 T-104 T-105 T-106 T-107 T-108

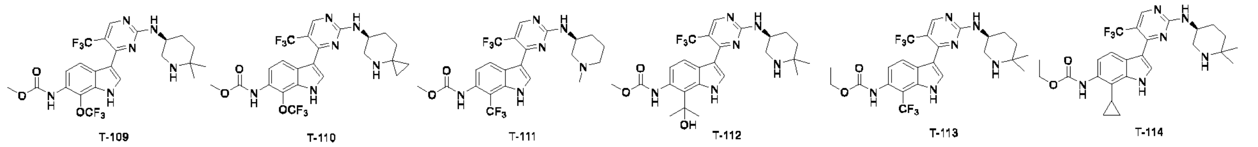

T-109 T-110 T-111 T-112 T-113 T-114

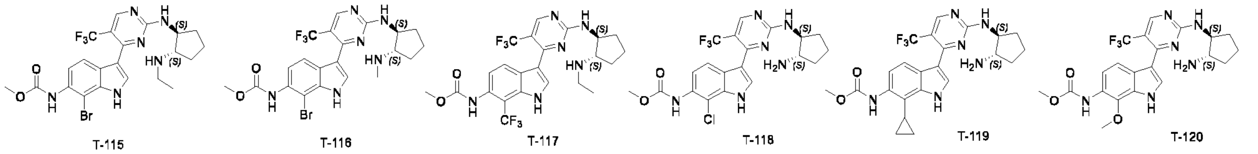

T-115 T-116 T-117 T-118 T-119 T-120

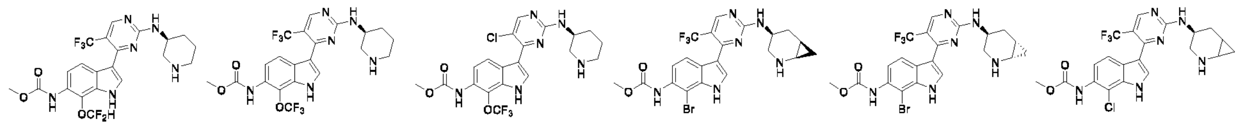

T-121 T-122 T-123 T-124 T-125 T-126

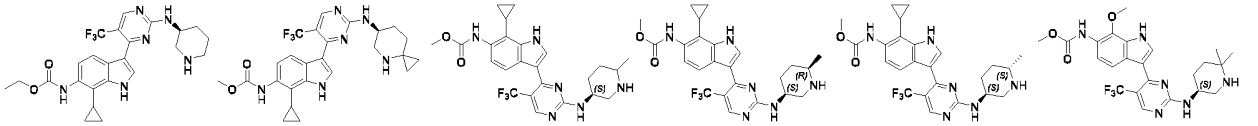

T-127 T-128 T-129 T-130 T-131 T-132

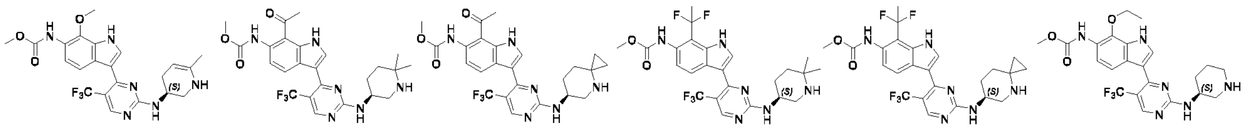

T-133 T-134 T-135 T-136 T-137 T-138

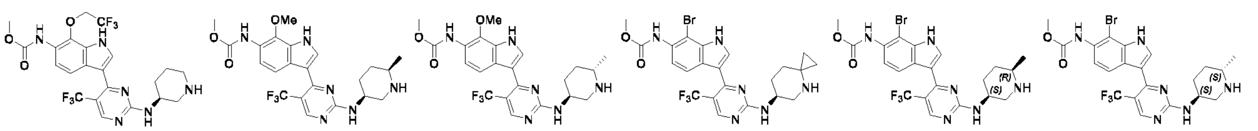

T-139 T-140 T-141 T-142 T-143 T-144

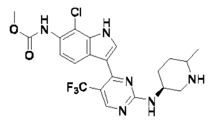
T-145

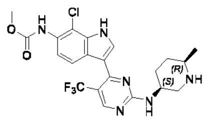
T-146

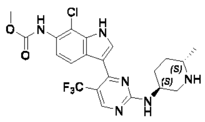
T-147

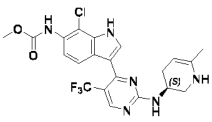
T-148

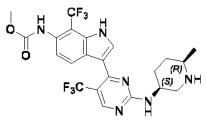
T-149

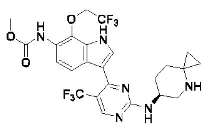
T-150

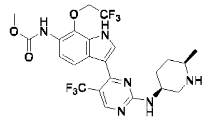
T-151

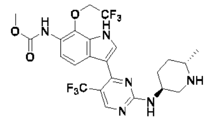
T-152

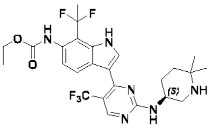
T-153

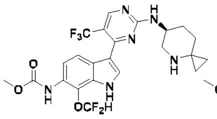
T-154

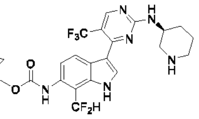
T-155

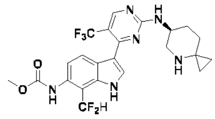
T-156

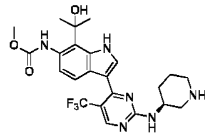
T-157

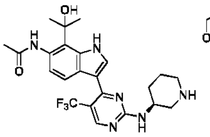
T-158

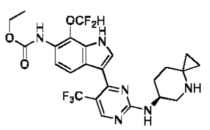
T-159

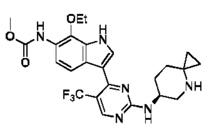
T-160

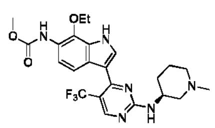
T-161

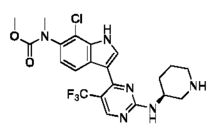
T-162

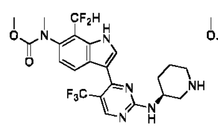
T-163

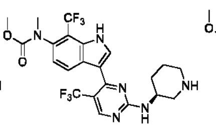
T-164

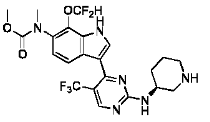
T-165

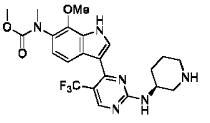
T-166

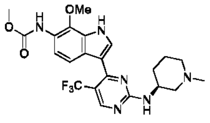
T-167

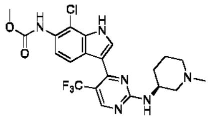
T-168

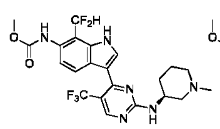
T-169

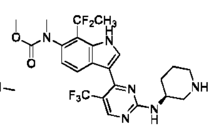
T-170

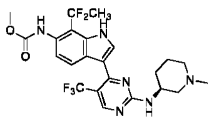
T-171

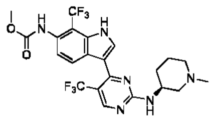
T-172

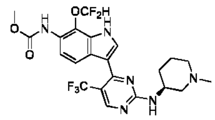
T-173

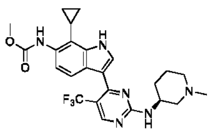
T-174

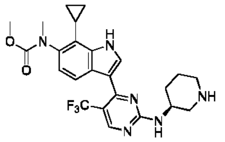
T-175

.

In another preferred embodiment, the compound is selected from the group consisting of:

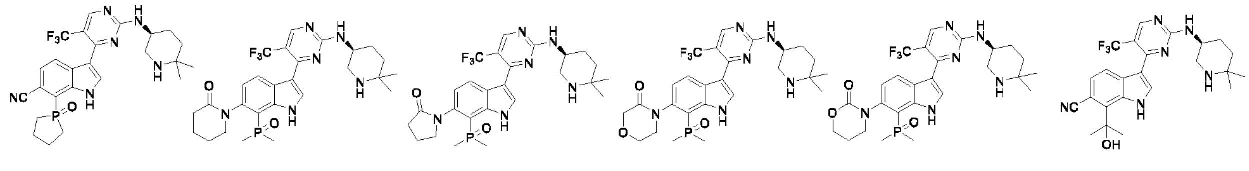
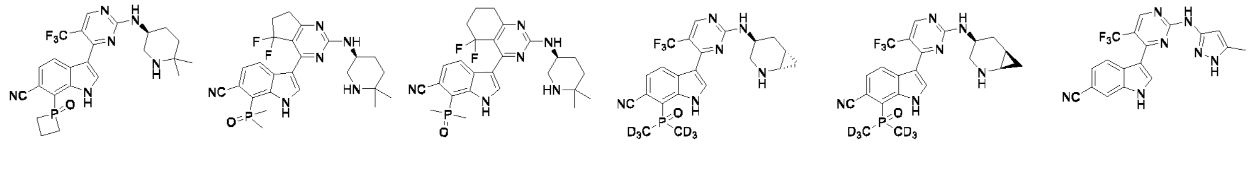

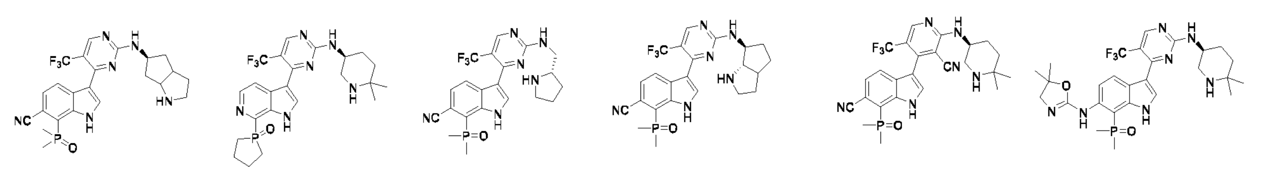

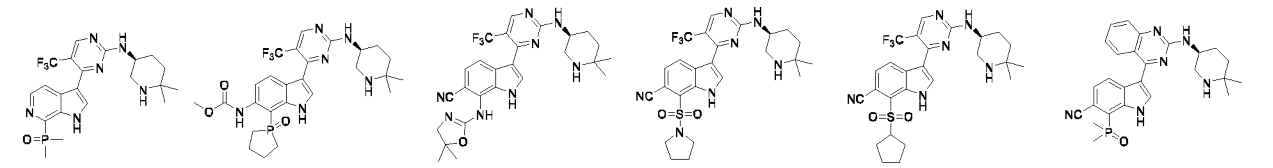

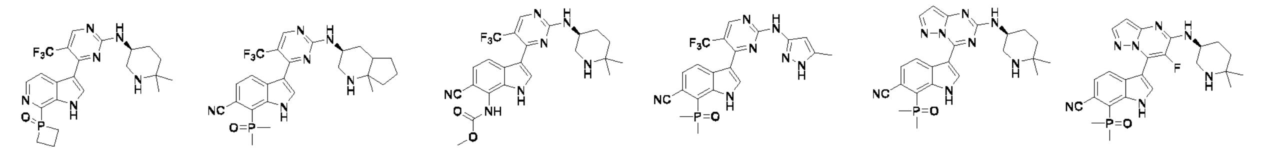

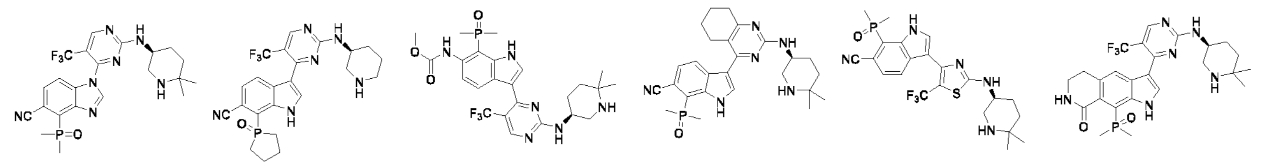

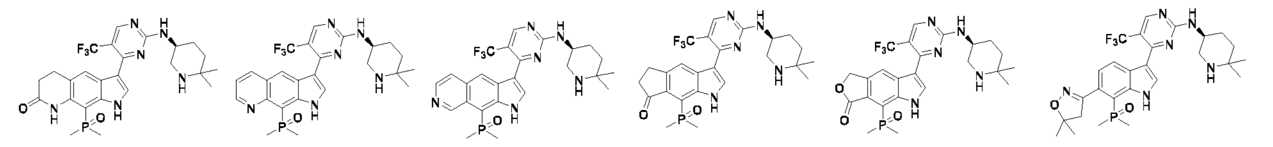

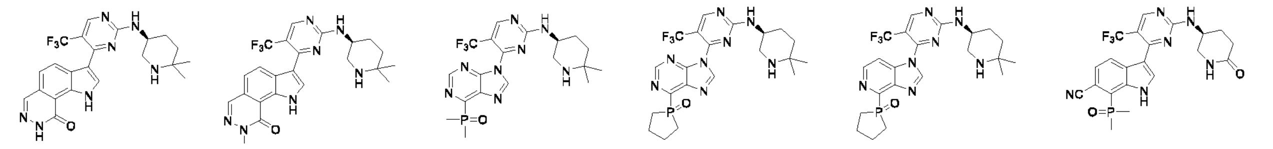

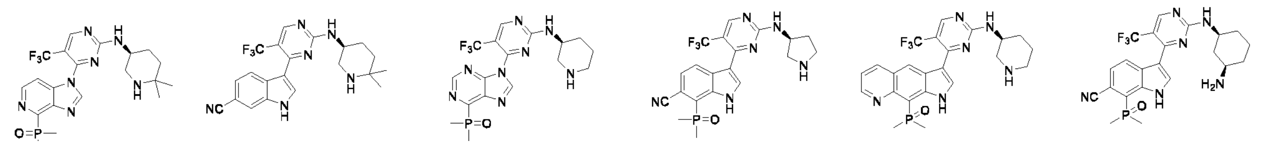

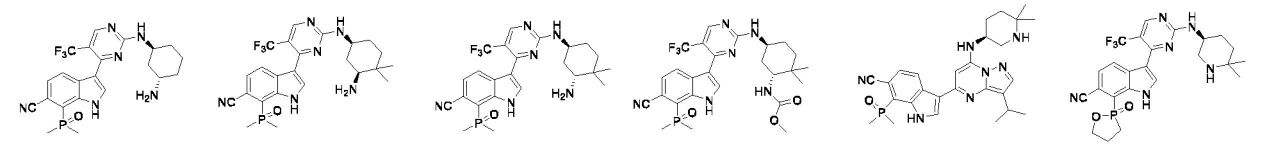

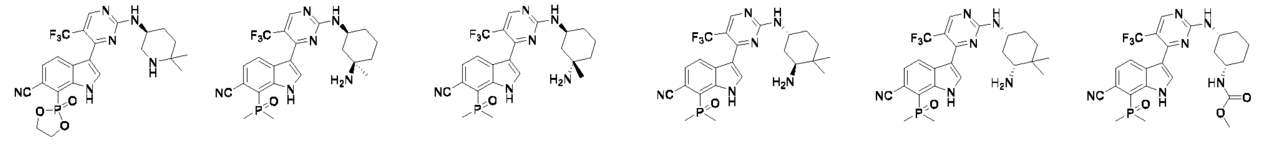

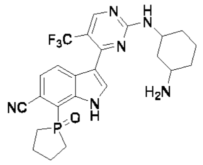 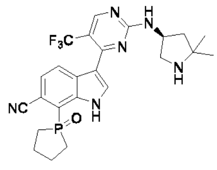 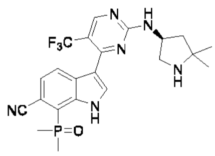 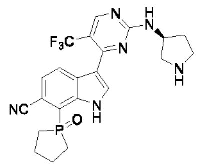 .

In another preferred embodiment, the pharmaceutically acceptable salt is an inorganic acid salt or an organic acid salt;

the inorganic acid salt is selected from the group consisting of hydrochloride, hydrobromide, hydroiodate, sulfate, bisulfate, nitrate, phosphate, and acid phosphate;
the organic acid salt is selected from the group consisting of formate, acetate, trifluoroacetate, propionate, pyruvate, hydroxyacetate, oxalate, malonate, fumarate, maleate, lactate, malate, citrate, tartrate, methanesulfonate, ethanesulfonate, benzene sulfonate, p-toluene sulfonate, salicylate, picrate, glutamate, ascorbate, camphorate, and camphor sulfonate.

In the second aspect of the present invention, it provides a pharmaceutical composition comprising a prophylactically and/or therapeutically effective amount of the compound according to the first aspect of the present invention and a pharmaceutically acceptable carrier.
In the third aspect of the present invention, it provides a use of the compound according to the first aspect of the present invention for the preparation of a medicament for use as a CDK7 kinase inhibitor.
In the fourth aspect of the present invention, it provides a use of the compound according to the first aspect of the present invention for the preparation of a medicament for modulating CDK7 kinase activity or for the prevention and/or treatment of CDK7-related diseases.
In another preferred embodiment, the CDK7-related disease is selected from the group consisting of inflammation, cancer, cardiovascular disease, infection, immune disease, and metabolic disease.
In another preferred embodiment, the cancer is selected from the group consisting of lung cancer, breast cancer, prostate cancer, colorectal cancer, liver cancer, pancreatic cancer, ovarian cancer, leukemia, neuroblastoma, gastric cancer, kidney cancer, esophageal cancer, and uterine cancer.
It should be understood that in the present invention, any of the technical features specifically described above and below (such as in the Examples) can be combined with each other, so as to constitute new or preferred technical solutions which will not redundantly be described one by one herein.

## DESCRIPTION OF FIGURES

Figure 1 shows the pharmacokinetic parameters of the compound of the present invention and the reference compound.

## DETAILED DESCRIPTION OF THE INVENTION

After extensive and in-depth research, the present inventors unexpectedly discovered a class of compounds with better CDK7 kinase inhibitory activity. In addition, the compound has excellent inhibitory activity on CDK7 kinase and has better pharmacodynamic/pharmacokinetic properties. On above basis, the present invention has been completed.

## TERMS

Unless otherwise specified, the following terms used in this application (including the specification and claims) have the definitions given below.
When a substituent is described by a conventional chemical formula written from left to right, the substituent also includes a chemically equivalent obtained substituent when the structural formula is written from right to left. For example, $-CH_2O-$ is equivalent to $-OCH_2-$.
"Alkyl (alone or as part of other groups)" refers to a monovalent linear or branched saturated hydrocarbon group containing 1 to 12 carbon atoms composed only of carbon and hydrogen atoms. The alkyl is preferably C1-C6 alkyl (i.e. containing 1, 2, 3, 4, 5 or 6 carbon atoms). Examples of alkyl include but are not limited to methyl, ethyl, propyl, isopropyl, isobutyl, sec-butyl, tert-butyl, amyl, n-hexyl, octyl and dodecyl, etc. In the present application, alkyl is also intended to comprise a substituted alkyl, i.e. one or more positions in the alkyl are substituted, in particular 1-4 substituents, which

may be substituted at any position. "Haloalkyl" refers to an alkyl as defined herein in which one or more hydrogen is replaced by the same or different halogens. Examples of haloalkyl include $-CH_2Cl$, $-CH_2CF_3$, $-CH_2CCl_3$, perfluoroalkyl (e.g.$-CF_3$), etc.

"Alkylene" refers to divalent group of alkyl, e.g. $-CH_2-$, $-CH_2CH_2-$ and $-CH_2CH_2CH_2-$.

"Alkoxy (alone or as part of other groups)" refers to alkyl to which an oxy is attached with a formula alkyl O-, wherein the alkyl has the definition described above, and preferably the alkoxy is C1-C6 alkoxy. Alkoxy includes but is not limited to methoxy, ethoxy, propoxy, tert-butoxy, etc. "Haloalkoxy" refers to the formula -OR, wherein, R is a haloalkyl as defined herein. Examples of haloalkoxy include but are not limited to trifluoromethoxy, difluoromethoxy, and 2, 2, 2-trifluoroethoxy, etc.

"Thioalkyl" refers to that the carbon in the alkyl is substituted by S, S (O) or S $(O)_2$. "Alkenyl (alone or as part of other groups)" refers to aliphatic group containing at least one double bond, typically having between 2 and 20 carbon atoms. In the present invention, "C2-C6 alkenyl" refers to alkenyl containing 2, 3, 4, 5 or 6 carbon atoms. The alkenyl includes but is not limited to for example, vinyl, propenyl, butenyl, 1 -methyl-2-buten-1-yl, etc. In the present invention, the alkenyl includes substituted alkenyl.

"Alkenylene" refers to alkenyl having two connection points. For example, "vinylidene" refers to the group -CH = CH-. The alkenylene may also be in an unsubstituted form or in a substituted form having one or more substituents.

"Alkynyl (alone or as part of other groups)" refers to a straight or branched hydrocarbon chain containing more than 2 carbon atoms and characterized by one or more triple bonds, typically having 2 to 20 carbon atoms. In the present invention, "C2-6 alkynyl" refers to alkynyl having 2, 3, 4, 5 or 6 carbon atoms. Alkynyl includes but is not limited to ethynyl, propargyl and 3-hexynyl. One of the triple-bond carbons can optionally be the connection point of alkynyl substituent. In the invention, the alkynyl also includes substituted alkynyl.

"Alkynylene" refers to alkynyl having two connecting points. For example, "ethynylene" refers to the group: -C = C-. The alkynylene may also be in an unsubstituted form or in a substituted form having one or more substituents.

"Aliphatic group" refers to straight-chain, branched or cyclic hydrocarbon group including saturated and unsaturated group, such as alkyl, alkenyl and alkynyl.

"Aromatic ring system" refers to monocyclic, bicyclic or polycyclic hydrocarbon ring system in which at least one ring is aromatic.

"Aryl (alone or as part of other groups)" refers to a monovalent group of an aromatic ring system. Representative aryl include all aromatic ring systems, such as phenyl, naphthyl and anthryl; and ring systems in which aromatic carbocyclic rings are fused with one or more non-aromatic carbocyclic rings, such as indanyl, phthalimide, naphthylimide or tetrahydronaphthyl, etc. In the present invention, the aryl is preferably C6-C12 aryl. In the present invention, aryl is also intended to comprise substituted aryl.

"Arylalkyl" or "aralkyl" refers to an alkyl moiety in which the alkyl hydrogen atom is substituted by an aryl. An aralkyl includes a group in which one or more hydrogen atoms are substituted by aryl, wherein aryl and alkyl are defined as above. Examples of "arylalkyl" or "aralkyl" include benzyl, 2-phenylethyl, 3-phenylpropyl, 9-fluorenyl, diphenylmethyl and triphenylmethyl, etc.

"Aryloxy" refers to -O-(aryl), wherein the aryl moiety is as defined herein.

"Heteroalkyl" refers to a substituted alkyl having one or more skeleton chain atoms selected from atoms other than carbon, such as oxygen, nitrogen, sulfur, phosphorus, or a combination thereof. Numeric ranges may be given for example C1-C6 heteroalkyl refers to the number of carbons in the chain which includes 1 to 6 carbon atoms. For example, $-CH_2OCH_2CH_3$ is called "C3" heteroalkyl. The connection with the rest of the molecule can be through heteroatoms or carbons in heteroalkyl chain. "Heteroalkylene" refers to an optionally substituted divalent alkyl having one or more skeleton chain atoms selected from atoms other than carbon, such as oxygen, nitrogen, sulfur, phosphorus, or a combination thereof.

"Carbocyclic system" refers to a monocyclic, dicyclic or polycyclic hydrocarbon ring system in which each ring is fully saturated or contains one or more unsaturated units, but none of the rings are aromatic.

"Carbocyclic group" refers to a monovalent group of a carbocyclic system. These include, for example, cycloalkyl (cyclopentyl, cyclobutyl, cyclopropyl, cyclohexyl, etc.) and cycloalkenyl (e.g. cyclopentenyl, cyclohexenyl, cyclopentadienyl, etc.).

"Cycloalkyl" refers to a monovalent saturated carbocyclic group consisting of monocyclic or dicyclic ring having 3-12, preferably 3-10, more preferably 3-8 ring atoms. The cycloalkyl may optionally be substituted with one or more substituents, wherein each substituent is independently a hydroxyl, alkyl, alkoxy, halogen, haloalkyl, amino, monoalkylamino or dialkylamino. Examples of cycloalkyl include but are not limited to cyclopropyl, cyclobutyl, cyclopentyl, cyclohexyl and cycloheptyl, etc.

"Cycloalkoxy" refers to the formula -OR, wherein R is a cycloalkyl as defined herein. Examples of cycloalkyloxy include cyclopropyloxy, cyclobutyloxy, cyclopentyloxy and cyclohexyloxy, etc. "Cycloalkyl alkyl" means -(cycloalkyl)-alkyl in which cycloalkyl and alkyl are as disclosed herein. "Cycloalkyl alkyl" is bonded to the parent molecular structure by a cycloalkyl.

"Heteroaromatic ring system" refers to a monocyclic (e.g. 5 or 6 membered), dicyclic (6-12 membered), or polycyclic system in which at least one ring is both aromatic and contains at least one heteroatom (e.g. N, O, or S). And none of the other rings are heterocyclyl (as defined below). In some cases, rings that are aromatic and contain heteroatoms contain 1, 2, 3 or 4 ring heteroatoms in the ring. At least one ring is heteroaromatic, and the remaining rings may be saturated, partially unsaturated or completely unsaturated.

"Heteroaryl" refers to a monocyclic (e.g. 5 or 6 membered), dicyclic (e.g. 8-10 membered) or tricyclic group with 5 to 12 ring atoms, and it contains at least one aromatic ring containing 1, 2 or 3 cyclic heteroatoms selected from N, O or S, and the remaining ring atoms are C. It should be clear that the connection point of the heteroaryl should be located on the aromatic ring. Examples of heteroaryl include but are not limited to imidazolyl, aoxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinazinyl, naphthyridinyl, pterridinyl, carbazolyl, azepinyl, diazepinyl and acridinyl, etc.. Heteroarylene refers to heteroaryl having two connecting points.

"Heterocyclic system" refers to monocyclic, bicyclic and polycyclic systems where at least one ring is saturated or partially unsaturated (but not aromatic) and the ring contains at least one heteroatom. The heterocyclic system can be attached to the side group of any heteroatom or carbon atom to produce a stable structure and any ring atom can be optionally substituted.

"Heterocyclyl" refers to a monovalent group of a heterocyclic system. It usually refers to stable monocyclic (such as 3-8 membered, i.e. 3 membered, 4 membered, 5 membered, 6 membered, 7 membered or 8 membered) or bicyclic (such as 5-12 membered, i.e. 5 membered, 6 membered, 7 membered, 8 membered, 9 membered, 10 membered, 11 membered or 12 membered) or polycyclic (such as 7-14 membered, i.e. 7 membered, 8 membered, 9 membered, 10 membered, 11 membered, 12 membered, 13 membered or 14 membered), including fused ring, spiro ring and/or bridge ring structure, which is saturated and partially unsaturated and contains carbon atoms and 1, 2, 3 or 4 heteroatoms independently selected from N, O or S. Representative heterocyclyl include the following ring systems, where (1) each ring is non-aromatic and at least one ring contains a heteroatom, for example, tetrahydrofuryl, tetrahydropyranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl and quinuclidinyl. (2) At least one ring is non-aromatic and contains heteroatoms and at least one other ring is an aromatic carbocyclic ring, for example, 1, 2, 3, 4-tetrahydroquinolinyl, 1, 2, 3, 4-tetrahydroisoquinolinyl. And (3) at least one ring is non-aromatic and contains a heteroatom and at least one other ring is aromatic and contains a heteroatom, for example, 3, 4-dihydro-1H-pyrano [4, 3-c] pyridine and 1, 2, 3, 4-tetrahydro-2, 6-naphthyridine. Heterocyclylene refers to heterocyclyl having two connecting points. In the present invention, the heterocyclylene is preferably bicyclic, wherein one ring is a heteroaryl and connected to the other parts of the general formula through the heteroaryl. In the present invention, the heterocyclylene is preferably a 5-6-membered monocyclic heterocyclylene or an 8-10-membered bicyclic heterocyclylene.

"Heterocyclyl alkyl" refers to an alkyl substituted with a heterocyclyl, wherein the heterocyclyl and alkyl are defined as above.

"Alkylamino" refers to a group having a structure of alkyl-NR-, where R is H, or an alkyl, cycloalkyl, aryl, heteroaryl, etc. as described above.

"Cycloalkylamino" refers to the formula -NRaRb, wherein Ra is H, an alkyl as defined herein or a cycloalkyl as defined herein, and Rb is a cycloalkyl as defined herein; or Ra and Rb together with their attached N atoms form a 3-10-membered N-containing monocyclic or bicyclic heterocyclyl, such as tetrahydropyrrolyl. As used herein, C3 -C8 cycloalkylamino refers to an amino containing 3-8 carbon atoms.

As used herein, an "ester" refers to -C (O)-O-R or R-C (O)-O-, wherein R independently represents hydrogen, alkyl, cycloalkyl, aryl, heteroaryl, heterocyclyl as defined above.

As used herein, the term "amido" refers to a group with a structure of -CONRR', wherein R and R' independently represent hydrogen, alkyl or substituted alkyl, cycloalkyl or substituted cycloalkyl, aryl or substituted aryl, heterocyclyl or substituted heterocyclyl with the definitions as described above. R and R' may be the same or different in the dialkylamino segments.

As used herein, the term "sulfonamido" refers to a group with a structure of $-SO_2NRR'$, wherein R and R' independently represent hydrogen, alkyl or substituted alkyl, cycloalkyl or substituted cycloalkyl, aryl or substituted aryl, heterocyclyl or substituted heterocyclyl with the definitions as described above. R and R' may be the same or different in the dialkylamino segments.

"Ketocarbonyl" refers to R-C (= O)-, wherein R is alkyl, cycloalkyl, etc. with the definitions as described above.

When the substituent is a non-terminal substituent, it is a subunit of the corresponding substituent, such as alkyl corresponding to alkylene, cycloalkyl corresponding to cycloalkylene, heterocyclyl corresponding to heterocyclylene, alkoxy corresponding to alkyleneoxy, etc..

In the present invention, each of the above-mentioned groups of alkyl, alkoxy, cycloalkyl, heteroalkyl, aryl,

heteroaryl, cycloheteroalkyl, alkenyl, alkynyl, heterocycle, heterocyclyl, etc. may be substituted or unsubstituted.

As used herein, the term "substituted" refers to the substitution of one or more hydrogen atoms on a specific group by specific substituent. The specific substituents are the substituents described correspondingly in the foregoing, or the substituents appearing in the respective examples. Unless otherwise specified, a substituted group may have a substituent selected from a specific group at any substitutable position of the group, and the substituent may be the same or different at each position. Those skilled in the art should understand that the combinations of substituents contemplated by the present invention are those that are stable or chemically achievable. Typical substituents include but are not limited to one or more of the following groups: such as hydrogen, deuterium, halogen (such as monohalogenated substituent or polyhalogenated substituents, and the latter such as trifluoromethyl or alkyl containing $Cl_3$), cyano, nitro, oxo (= O), trifluoromethyl, trifluoromethoxy, cycloalkyl, alkenyl, alkynyl, heterocycle, aromatic ring, $OR_a$, $SR_a$, $S(=O)R_e$, $S(=O)_2R_e$, $P(=O)_2R_e$, $S(=O)_2OR_e$, $P(=O)_2OR_e$, $NR_bR_c$, $NR_bS(=O)_2R_e$, $NR_bP(=O)_2R_e$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_e$, $NR_dC(=O)NR_bR_c$, $NR_dS(=O)_2NR_bR_c$, $NR_dP(=O)_2NR_bR_c$, $NR_bC(=O)R_a$ or $NR_bP(=O)_2R_e$, wherein $R_a$ can independently represent hydrogen, deuterium, alkyl, cycloalkyl, alkenyl, alkynyl, heterocycle or aromatic ring, $R_b$, $R_c$ and $R_d$ can independently represent hydrogen, deuterium, alkyl, cycloalkyl, heterocycle or aromatic ring, or $R_b$ and $R_c$ together with the N atom form a heterocycle, $R_e$ can independently represent hydrogen, alkyl, cycloalkyl, alkenyl, alkynyl, heterocycle or aromatic ring. The above typical substituents, such as alkyl, cycloalkyl, alkenyl, cycloalkenyl, alkynyl, heterocyclyl or aromatic ring, may be optionally substituted. The substituent is such as (but is not limited to): halogen, hydroxyl, cyano, carboxy (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehydyl, C2-C10 acyl, C2-C10 ester, amino, C1-C6 alkoxy, C1-C10 sulfonyl, and C1-C6 uramido, etc..

"Cyano" refers to -CN.

"Nitro" refers to $-NO_2$.

"Hydroxyl" refers to -OH.

"Amino" refers to $-NH_2$ or RNH-, wherein R is ketocarbonyl, sulfonyl, sulfonamido, $R_a$-C(=O)-, $R_aR_b$N-C (=O)- and so on, wherein $R_a$ and $R_b$ are alkyl, cycloalkyl, aryl or heteroaryl, etc.

"Halogen (halogenated)" refers to any halogen group, e.g. -F, -Cl, -Br or -I.

"Deuterated compound" refers to the compound obtained by replacing one hydrogen atom (H) or multiple hydrogen atoms (H) with deuterium atoms (D) in a compound.

In the present invention, the term "more" independently refers to 2, 3, 4, or 5.

The structural formula of the carbamate is -NH-C(=O)-O-R, wherein R is aryl and heteroaryl.

**Active ingredient**

As used herein, the terms "compounds of the invention" or "active ingredients of the invention" are used interchangeably and refer to compounds of formula I, or pharmaceutically acceptable salt, hydrate, solvate, isotope compound (e.g. deuterated compound) or prodrug thereof. The term also includes racemate and optical isomer.

The compound of formula I has the following structure:

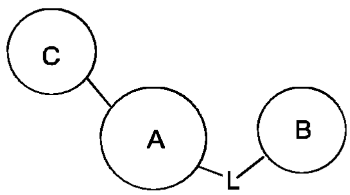

I

ring A, ring B, ring C and L are defined as above.

The salts that may be formed by the compound in the present invention are also within the scope of the present invention. Unless otherwise stated, the compound in the present invention is understood to include its salt. The term "salt" as used herein refers to a salt formed in the form of acid or base from inorganic or organic acid and base. Further, when the compound in the present invention contains a base fragment which includes, but is not limited to pyridine or imidazole, when it contains an acid segment which includes, but is not limited to carboxylic acid. The zwitter-ion that may form "inner salt" is included within the range of the term "salt". Pharmaceutically acceptable (i.e., non-toxic, physiologically acceptable)salt is preferred, although other salts are also useful and may be used, for example, in the separation or purification steps of the preparation process. The compound of the present invention may form a salt, for example, compound I is reacted with a certain amount (such as an equivalent amount) of an acid or base, and precipitated in a medium, or freeze-dried in aqueous solution.

The base fragment contained in the compounds of the present invention includes but is not limited to amines

or pyridine or imidazole rings, and may form salt with organic or inorganic acid. Typical acids that form salts include acetate (such as acetic acid or trihalogenated acetic acid, such as trifluoroacetic acid), adipate, alginate, ascorbate, aspartate, benzoate, benzene sulfonate, disulfate, borate, butyrate, citrate, camphorate, camphor sulfonate, cyclopentane propionate, diethylene glycolate, lauryl sulfate, ethanesulphonate, fumarate, gluceptate, glycerophosphate, hemisulphate, enanthate, caproate, hydrochloride, hydrobromide, hydriodate, isethionate(e.g., 2-hydroxyl-ethesulfonate), lactate, maleate, mesylate, naphthalenesulfonate (e.g., 2-naphthalenesulfonate), nicotinate, nitrate, oxalate, pectate, persulfate, phenylpropionate (e.g., 3-phenylpropionate), phosphate, picrate, pivalate, propionate, salicylate, succinate, sulfate (e.g., formed with sulfuric acid), sulfonate, tartrate, thiocyanate, toluenesulfonate(e.g., tosilate), dodecanoate, etc..

Some compounds of the invention may contain acidic fragments including, but not limited to carboxylic acid which may form salts with various organic or inorganic bases. Salt formed by typical base includes ammonium salt, alkali metal salt (such as sodium, lithium and potassium salts), alkaline earth metal salt (such as calcium and magnesium salts), and salt formed by organic bases (such as organic amines), such as benzathine, dicyclohexylamine, hydrabamine (salt formed with *N,N*- bis (dehydroabietyl) ethylenediamine), *N*-methyl-D-glucosamine, *N*-methyl-D-glucoamide, tert-butyllamine, and the salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl), dialkyl sulfate (such as dimethyl, diethyl, dibutyl, and dipentyl sulfates), long chain halides (such as chlorides, bromides and iodides of decyl, dodecyl, tetradecyl, and tetradecyl), aralkyl halides (such as bromides of benzyl and phenyl), etc..

The prodrug and solvate of the compound in the present invention are also included within the scope of the present invention.

The term "prodrug" herein refers to a compound, which produces a compound, salt, or solvate in the present invention resulting from the chemical transformation of a metabolic or chemical process when used in the treatment of an associated disease. The compounds of the invention include solvates such as hydrates.

Compound, salt or solvate in the present invention, may be present in tautomeric forms such as amide and imino ether. All of these tautomers are part of the present invention.

Stereisomers of all compounds (e.g., those asymmetric carbon atoms that may be present due to various substitutions), including their enantiomeric forms and non-enantiomed forms, all belong to the protection scope of the present invention. The independent stereoisomer in the present invention may not coexist with other isomers (e.g., as a pure or substantially pure optical isomer with special activity), or may be a mixture (e.g., racemate), or a mixture formed with all other stereoisomers or a part thereof. The chiral center of the present invention has two configurations of S or R, which is defined by International Union of Pure and Applied Chemistry (IUPAC) in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acids.

Weight content of compound in the present invention obtained by preparation, separation and purification in turn is equal to or greater than 90%, such as equal to or greater than 95%, equal to or greater than 99% ("very pure" compound), which is listed in the description of the text. In addition, the "very pure" compound of the present invention is also part of the present invention.

All configuration isomers of the compound of the present invention are within the scope, whether in mixture, pure or very pure form. The definition of the compound of the present invention comprises cis (*Z*) and trans (*E*) olefin isomers, and cis and trans isomers of carbocycle and heterocycle.

In the entire specification, the groups and substituents can be selected to provide stable fragments and compounds.

Specific functional groups and chemical term definitions are described in detail below. For the purposes of the present invention, the chemical elements are consistent with Periodic Table of the Elements, CAS version, Handbook of Chemistry and Physics, 75th Ed. The definition of a particular functional group is also described therein. In addition, the basic principles of Organic Chemistry as well as specific functional groups and reactivity are described in "Organic Chemistry", Thomas Sorrell, University Science Books, Sausalito: 1999, the entire content of which is incorporated herein by reference.

Some compounds of the present invention may exist in specific geometric or stereoisomer forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety of ratios of isomers. For example, mixtures with only two isomers may have the following combinations: 50:50, 60:40, 70:30, 80:20, 90:10, 95:5, 96:4, 97:3, 98:2, 99:1, or 100:0, all ratios of the isomers are within the scope of the present invention. Similar ratios readily understood by those of ordinary skill in the art and ratios for mixtures of more complex isomers are also within the scope of the

present invention.

The invention also includes isotope labeled compounds, which are equivalent to the original compounds disclosed herein. However, in practice, it usually occurs when one or more atoms are replaced by atoms with a different atomic weight or mass number. Examples of compound isotopes that may be listed in the present invention include hydrogen, carbon, nitrogen, oxygen, phosphorus, sulfur, fluorine and chlorine isotopes, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$ respectively. Compound, or enantiomer, diastereomer, isomer, or pharmaceutically acceptable salt or solvate, the above compound containing isotopes or other isotope atoms are all within the scope of the invention. Some isotope-labeled compounds in the present invention, such as the radioactive isotopes of $^{3}H$ and $^{14}C$, are also included and are useful in experiments on the tissue distribution of drugs and substrates. Tritium ($^{3}H$) and Carbon-14 ($^{14}C$), which are relatively easy to prepare and detect, are the preferred choice. In addition, heavier isotope substitutions such as deuterium, i.e. $^{2}H$, have advantages in certain therapies due to their good metabolic stability, such as increased half-life or reduced dosage in vivo, and thus may be preferred in certain situations. Isotope-labeled compounds can be prepared by conventional methods through replacing non-isotopic reagents with readily available isotope-labeled reagents using the disclosed scheme shown in the Example.

If the synthesis of a specific enantiomer of the compound of the invention is to be designed, it can be prepared by asymmetric synthesis, or derivatized with chiral auxiliary reagent, separating the resulting diastereomeric mixture and removing the chiral auxiliary reagent to obtain a pure enantiomer. In addition, if a molecule contains a basic functional group, such as an amino acid, or an acidic functional group, such as a carboxyl group, a diastereomer salt can be formed with a suitable optically active acids or bases, and it can be separated by conventional means, such as crystallization or chromatography, to obtain a pure enantiomer.

As described herein, the compound in the present invention may be substituted with any number of substituents or functional groups to extend its scope. In general, whether the term "substituted" appears before or after the term "optional", the general formula that includes substituents in the compound of the present invention means the substitution of a specified structural substituent for a hydrogen radical. When multiple locations in a particular structure are replaced by multiple specific substituents, each location of the substituents can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic organic compounds. In the present invention, such as heteroatomic nitrogen, its valence state may be supplemented by a hydrogen substituent or by any permitted organic compound described above. Furthermore, the invention does not unintentionally limit the substituted organic compounds in any way. The present invention considers that a combination of substituents and variable groups is good for the treatment of diseases in the form of stable compounds. The term "stable" herein refers to a stable compound which is sufficient for maintaining the integrity of the compound structure within a sufficiently long time, preferably being effective in a sufficiently long time, which is hereby used for the above purposes.

The metabolites of the compounds of the present application and their pharmaceutically acceptable salts, and prodrugs that can be converted into the compounds of the present application and their pharmaceutically acceptable salts thereof in vivo, are also included in the claims.

In another preferred embodiment, in the compound, any one of the groups is a corresponding group in the specific compound.

## PREPARATION METHOD

Methods for preparing compounds of formula I are described in the following schemes and examples. Raw materials and intermediates are purchased from commercial sources, prepared by known steps, or otherwise described. In some cases, the sequence of steps to perform the reaction scheme may be changed to facilitate the reaction or avoid unwanted side reaction products.

The preparation method of the compound of formula I of the present invention is more specifically described below, but these specific methods do not constitute any limitation of the invention. The compound of the invention may also optionally be conveniently prepared by combining the various synthetic methods described in this specification or known in the art, such a combination may be easily performed by a skilled person in the art to which the invention belongs.

Generally, in the preparation process, each reaction is usually carried out under the protection of inert gas and in an appropriate solvent at 0 to 150 °C, and the reaction time is usually 2-24 hours.

Preferably, the preparation method is as follows:

method one:

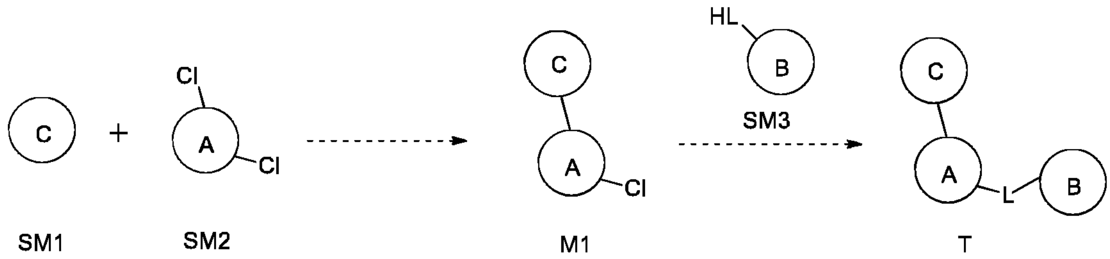


Step 1: in solvents (such as 1,2-dichloroethane, dioxane, tetrahydrofuran), under the condition of Lewis acid (such as aluminum trichloride, zinc chloride, boron trifluoride ether, etc.), SM1 reacts with SM2 at 70-120 °C to form M1;
Step 2: in inert solvents (such as DMF, dioxane, ethylene glycol dimethyl ether, N-methylpyrrolidone, etc.), under basic condition (such as diisopropylethylamine, potassium acetate, DBU, etc.) or in the presence of catalysts and ligands (such as $Pd(PPh_3)_4$, $Pd_2(dba)_3$\t-BuXphos, etc.), M1 reacts with SM3 to form T (i.e. the compound of formula I).
method two:

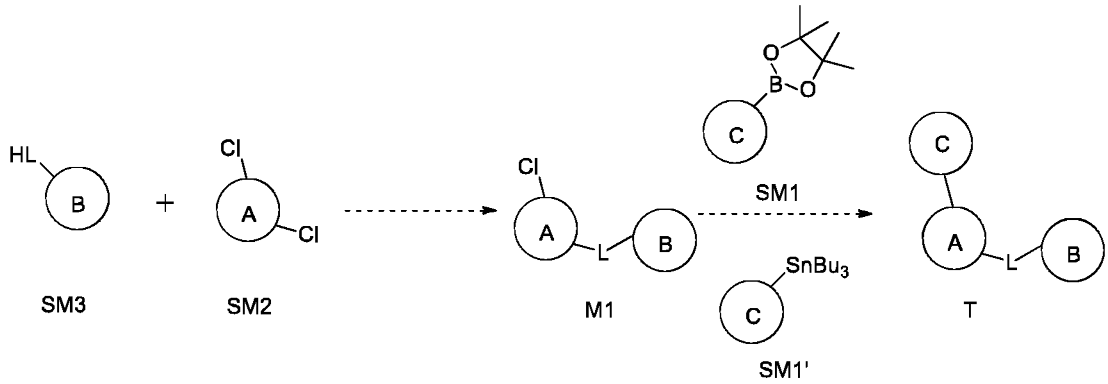


Step 1: in inert solvents (such as tetrahydrofuran, 1,2-dichloroethane, N,N-dimethylformamide, dioxane, ethylene glycol dimethyl ether, etc.), under the catalysis of Lewis acid (zinc chloride, ferric chloride, etc.), SM3 reacts with SM2 to obtain M1;
Step 2: in inert solvents (such as N,N-dimethylformamide, dioxane, dimethyl sulfoxide, etc.), under basic conditions (such as potassium carbonate, potassium phosphate, etc.), in the presence of catalysts and ligands (such as $Pd(PPh_3)_4$), M1 reacts with SM1 or SM1' to form T (i.e. the compound of formula I).

Among the above formulas, ring A, ring B, ring C and L are as described above.
Unless otherwise specified, the above starting materials can be purchased commercially or synthesized according to the reported literature.

**Pharmaceutical composition and method for administration**

The pharmaceutical compositions of the present invention are used to prevent and / or treat the following diseases: inflammation, cancer, cardiovascular disease, infection, immunological disease, metabolic disease.
The compounds of formula I can be used in combination with other drugs known to treat or improve similar conditions. When administered in combination, the original administration method and dosage for the drug can remain unchanged, while the compound of formula I may be administered simultaneously or subsequently. Pharmaceutical composition simultaneously containing one or more known drugs and the compound of formula I may be preferred when administered in combination with one or more other drugs. The drug combination also includes administering the compound of formula I and other one or more known drugs at overlapping time. When the compound of formula I is combined with other one or more drugs, the dose of the compound of formula I or known drug may be lower than that of their individual use.
The drug or active ingredients that can be used in pharmaceutical use with the compounds of formula I include but are not limited to PD-1 inhibitor (such as nivolumab, pembrolizumab, JS-001, SHR-120, BGB-A317, IBI-308, GLS-010, GB-226, STW204, HX008, HLX10, BAT 1306, AK105, LZM 009 or the biological analogue thereof, etc.), PD-L1 inhibitor (such as durvalumab, atezolizumab, CS1001, KN035, HLX20, SHR-1316, BGB-A333, JS003, CS1003, KL-A167, F 520 , GR1405, MSB2311 or the biological analogue thereof, etc.), CD20 antibody (such as rituximab, obinutuzumab, ofatumumab, tositumomab, ibritumomab, etc.), CD47 antibody (such as Hu5F9-G4, CC-90002, TTI-621, TTI-622, OSE-172, SRF-231, AlX-148, NI-1701, SHR-1603, IBI188, IMM01), ALK inhibitor (such as Ceritinib, Alectinib, Brigatinib, Lorlatinib, Ocatinib), PI3K inhibitor (such as Idelalisib, Dactolisib, Taselisib, Buparlisib, etc.), BTK inhibitor (such as Ibrutinib, Tirabrutinib, Acalabrutinib, etc.), EGFR inhibitor (such as Afatinib, Gefitinib, Erlotinib , Lapatinib, Dacomitinib, Icotinib, Canertinib, etc.), VEGFR inhibitor (such as Sorafenib, Pazopanib, Regorafenib, Cabozantinib, Sunitinib, Donafenib, etc.), HDAC inhibitor (such as Givinostat, Droxinostat, Entinostat, Dacinostat, Tacedinaline, etc.), CDK inhibitor (such as Palbociclib, Ribociclib, Abemaciclib, Lerociclib, etc.), MEK inhibitor (such as Selumetinib (AZD6244), Trametinib (GSK1120212), PD0325901, U0126, AS-703026, PD184352 (CI-1040), etc.), mTOR inhibitor (such as Vistusertib, etc.), SHP2 inhibitor (such as RMC-4630, JAB-3068, TNO 155, etc.), IGF-1R inhibitor (such as Ceritinib, Okatinib, Linsitinib, BMS-754807, GSK1838705A, etc.), ER antagonist or degradant (such as tamoxifen, fulvestrant, etc.), aromatase inhibitor (such as letrozole, etc.), BCL2 or BCL-XL inhibitor (such as ABT-199, ABT-263, etc.), Hedgehog inhibitor (such as vismodegib, cyclopamine, etc.), chemotherapy drug (such as cisplatin, etoposide, topotecan, etc.), PARP inhibitor (such as Olaparib, Veliparib, Rucaparib, etc.), ATR/ATM inhibitor (such as Ceralasertib, Berzosertib, etc.) or combinations thereof.

The dosage forms of the pharmaceutical composition of the prensent invention include (but are not limited to) : injection, tablet, capsule, aerosol, suppository, pellicle, dripping pill, liniment for external use, controlled release or sustained-release or nano formulation.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxylmethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, such as kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying

and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, propellants and inhalants. The active ingredient is mixed under sterile conditions with a physiologically acceptable carrier and any preservatives, buffers, or propellants which may be required if necessary.

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1 ~2000mg, preferably 50~1000mg. Of course, specific doses should also consider factors such as the administration route, the health of the patient, etc., which are within the skill of the skilled physician.

The present invention also provides a preparation method of pharmaceutical composition comprising the step of mixing a pharmaceutically acceptable carrier with the compound of formula I or crystalline form, pharmacically acceptable salt, hydrate or solvate thereof of the present invention, thus forming the pharmaceutical composition.

The invention also provides a treatment method comprising the step of administering the compound of formula I, or its crystalline form, pharmaceutically acceptable salt, hydrate or solvate thereof, or the pharmaceutical composition of the invention to a subject in need thereof to inhibit CDK7.

Compared with the prior art, the present invention has the following main advantages:

(1) The compound of the invention has excellent inhibitory ability on CDK7 kinase;
(2) The compound of the invention has lower toxic and side effects;
(3) The compound of the invention has better pharmacodynamic and pharmacokinetic properties.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for demonstration purposes.

**Example**

The technical solution of the present invention will be further described below, but the protection scope of the present invention is not limited thereto.

**Synthesis of Intermediate 1**

The structural formula of intermediate 1 is as follows:

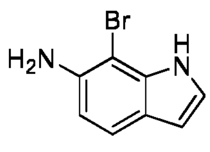

intermediate 1

The experimental process is as follows:

The synthetic route is as follows:

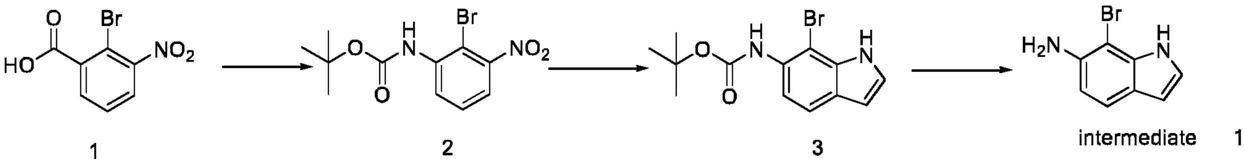

1 2 3 intermediate 1

Synthesis steps:

(1) Synthesis of Compound 2

175g(1.0eq) of compound 1 was dissolved in tert-butanol, stirred at room temperature, triethylamine was added to the reaction solution, the solution changed from clear yellow to clear reddish brown, DPPA(1.5eq) was slowly added under the protection of nitrogen, and the reaction system was slowly heated to 80 °C, and a large number of bubbles were formed at the same time. After the bubbles were reduced, the reaction was continued for 3 hours at 80°C, and then sodium carbonate aqueous solution was added to quench the reaction, extracted with ethyl acetate, organic phases were combined, dried, spin-dried, and purified by column to obtain 187.5g of compound 2.

(2) Synthesis of Compound 3

Under nitrogen protection, 1.0 mol/L vinyl magnesium bromide solution (5.0eq) was added to the reaction flask, and then a solution of compound 2(1.0eq) in tetrahydrofuran (THF) was slowly added dropwise in a dry ice bath. After the dropwise addition was completed, the temperature was naturally raised to room temperature to react overnight, ammonium chloride aqueous solution was added to quench the reaction, extracted with ethyl acetate, and the organic phases were combined, dried, spin-dried, and purified by column to obtain 132.4g of compound 3.

(3) Synthesis of Intermediate 1

Compound 3(132g) was added to 4.0 mol/L hydrochloric acid/1,4-dioxane solution at room temperature and reacted for two hours, the reaction system was filtered, and then the filter cake was washed with methyl tert-butyl ether to obtain 53.1g of intermediate 1 hydrochloride.

**Synthesis of Intermediate 2**

The structural formula of intermediate 2 is as follows:

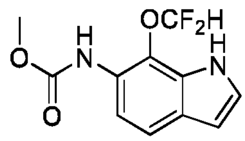

intermediate 2

The experimental process is as follows:
The synthetic route is as follows:

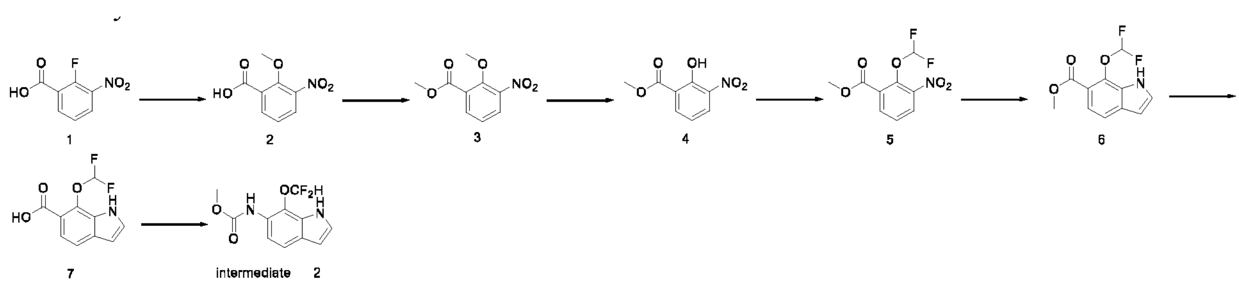

1 2 3 4 5 6

7 intermediate 2

Synthesis steps:

(1) Synthesis of Compound 2

At room temperature, compound 1(5.38g,1.0eq) was dissolved in methanol (60mL), and then sodium methoxide

(6.4g,2.0eq) was added. Then, the temperature was raised to reflux, and the reaction solution was reacted for 16 hours. After TLC monitored until the reaction was completed, the temperature was cooled down, methanol was spun off, then 100mL of ice water was added. The pH value of the reaction solution was adjusted to be weak acid (pH = 4-5) with 2mol/L hydrochloric acid, the product was precipitated, then filtered, the filter cake was washed with water, the filter cake was collected and dried to obtain 6.16g of compound 2.

(2) Synthesis of Compound 3

At room temperature, compound 2(6.36g,1.0eq) was dissolved in methanol (65mL), then the solution was cooled to 0°C and concentrated sulfuric acid (3.79g, 1.2eq) was added. Then, the temperature was raised slowly to reflux, the solution was reacted for 12 hours, and TLC monitored until the reaction was completed. The temperature was reduced to room temperature, then methanol was spun off, 50mL of ice water was added, the pH value was adjusted to be neutral with sodium bicarbonate aqueous solution, extracted with ethyl acetate, the organic phase was washed with saturated sodium chloride aqueous solution, dried, and spin-dried to obtain 5.8g of crude compound 3 which was used directly in the next step.

(3) Synthesis of Compound 4

At room temperature, compound 3(5.8g, 1.0eq) was dissolved in dichloromethane (100mL). Then, the temperature was lowered to -78°C under nitrogen protection, and boron tribromide (32.5mL, 5.0eq) was added dropwise. After the addtion was completed, the reaction was carried out at -78 °C for 1 hour. TLC monitored until the reaction was completed. At -78 °C, methanol (20mL) was added dropwise to quench the reaction, then the temperature was raised to 0 °C, 200mL of ice water was added, extracted with dichloromethane, and the organic phases were combined, dried, concentrated and purified by column to obtain 2.6g of compound 4.

(4) Synthesis of Compound 5

Compound 4(2.6g,1.0eq) was added to acetonitrile (20mL), then the temperature was cooled to -15°C, 10% potassium hydroxide aqueous solution (147.8g, 20.0eq) was added, diethyl bromodifluoromethylphosphate (10.6g,3.0eq) was slowly added dropwise, the reaction was continued at -10 °C for 2 hours after the addition, and then the temperature was slowly raised to room temperature to continue the reaction for 3 hours, TLC monitored until the reaction was completed. Ammonium chloride aqueous solution was added to quench the reaction, extracted with ethyl acetate, and the organic phases were combined, dried, spin-dried, and purified by column to obtain 0.92g of compound 5.

(5) Synthesis of Compound 6

Compound 5 (0.92g,1.0eq) was added to tetrahydrofuran (20mL), then the temperature was cooled to -78°C, then vinyl magnesium bromide (11.2mL, 1.0mol/L, 3.0eq) was added dropwise at this temperature, and then the reaction was continued at -78°C for 1 hour, and then the temperature was slowly increased to room temperature to react overnight. TLC monitored until the reaction was completed, then the temperature was reduced to -10 °C, and ammonium chloride aqueous solution was added dropwise to quench the reaction. The solution was extracted with ethyl acetate, organic phases were combined, dried, spin-dried, and purified by column to obtain 503mg of compound 6.

(6) Synthesis of Compound 7

Compound 6(500mg,1.0eq) was dissolved in methanol (5mL), then sodium hydroxide (250mg,3.0eq) was added, the temperature was raised to reflux to react overnight, TLC monitored until the reaction was completed, then the methanol was spin-dried, 20mL of ice water was added, the aqueous phase was extracted with methyl tert-butyl ether once, then the pH value of the aqueous phase was adjusted to be 4-5 with 2mol/L hydrochloric acid, solid was precipitated, the reaction solution was filtered by suction filtration, and the filter cake was washed with water, the filter cake was collected and dried to obtain 352mg of compound 7.

(7) Synthesis of Intermediate 2

Compound 7(350mg,1.0eq) was added to dioxane (5mL), then triethylamine (230mg,1.5eq) was added. After the temperature was raised to 90°C, diphenylphosphoryl azide (630mg,1.5eq) was slowly added dropwise, a large amount of gas was generated, the dropping speed was controlled to prevent spraying. After addition, the reaction was continued for 1 hour at this temperature, then methanol (1mL) was added dropwise, and then the reaction was continued for 3 hours. TLC monitored until the reaction was completed. The temperature was reduced to 0 °C, and then 2mol/L sodium hydroxide solution was added to quench the reaction, extracted with ethyl acetate, organic phases were combined, spin-dried, and purified by column to obtain 230mg of intermediate 2. LC-MS[M+1]:257.0.

**Synthesis of Intermediate 3**

The structural formula of intermediate 3 is as follows:

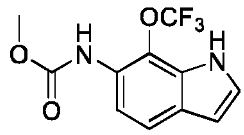

intermediate 3

The experimental process is as follows:
The synthetic route is as follows:

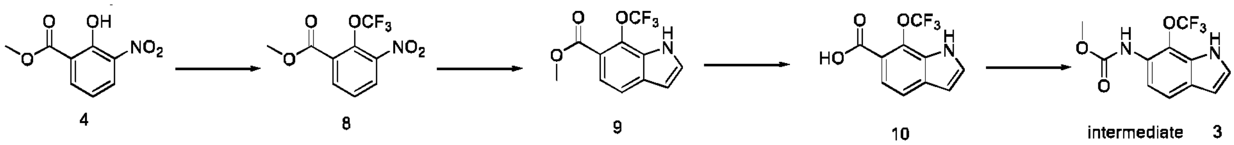

Synthesis steps:

(1) Synthesis of Compound 8
In the glove box, under nitrogen protection, silver trifluoromethanesulfonate (12.85g,5.0eq), selective fluoride reagent (Selectfluor)(7.1g,2.0eq), N-fluorobenzenesulfonimide (NFSI)(6.3g,2.0eq), cesium fluoride (9.1g,6.0eq) and compound 4(2.0g,1.0eq) were added to the reaction flask, then toluene (20mL) and trifluorotoluene (40mL) were added. Then, under nitrogen protection, trifluoromethyltrimethylsilane (11.36g, 8.0eq) and 2-fluoropyridine (4.85g, 5.0eq) were added. The reaction was carried out overnight at 35 °C, TLC monitored until the reaction was completed, filtered, the filter cake was washed with ethyl acetate, the organic phase was collected, spin-dried, and purified by column to obtain 0.15g of compound 8.
(2) Synthesis of Compound 9
Compound 8 (0.6g,1.0eq) was added to tetrahydrofuran (20mL), then the temperature was cooled to -78°C, then vinyl magnesium bromide (6.7mL, 1.0mol/L, 3.0eq) was added dropwise at this temperature, and then the reaction was continued at -78°C for 1 hour, and then the temperature was slowly increased to room temperature to react overnight. TLC monitored until the reaction was completed, then the temperature was reduced to -10 °C, and ammonium chloride aqueous solution was added dropwise to quench the reaction. The solution was extracted with ethyl acetate, and the organic phases were combined, dried. spin-dried, and purified by column to obtain 320mg of compound 9.
(3) Synthesis of Compound 10
Compound 9(300mg,1.0eq) was dissolved in methanol (5mL), then sodium hydroxide (140mg,3.0eq) was added, the temperature was raised to reflux to react overnight, TLC monitored until the reaction was completed, then the methanol was spin-dried, 20mL of ice water was added, the aqueous phase was extracted with methyl tert-butyl ether once, then the pH value of the aqueous phase was adjusted to be 4-5 with 2mol/L hydrochloric acid, solid was precipitated, the reaction solution was filtered by suction filtration, and the filter cake was washed with water, the filter cake was collected and dried to obtain 145mg of compound 10.
(4) Synthesis of Intermediate 3
Compound 10(100mg, 1.0eq) was added to dioxane (3mL), then triethylamine (62mg, 1.5eq) was added. After being heated to 90°C, diphenylphosphoryl azide (168mg, 1.5eq) was slowly added dropwise. After addition, the reaction was continued for 1 hour at this temperature, then methanol (1mL) was added dropwise, and then the reaction was continued for 3 hours. TLC monitored until the reaction was completed. The temperature was cooled to 0 °C, then ammonium chloride aqueous solution was added to quench the reaction, extracted with ethyl acetate, organic phases were combined, and spin-dried, and purified by column to obtain 54mg of intermediate 3. LC-MS[M+1]:275.0.

**Synthesis of Intermediate 4**

The structural formula of intermediate 4 is as follows:

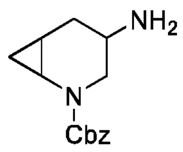

intermediate 4

The experimental process is as follows:

The synthetic route is as follows:

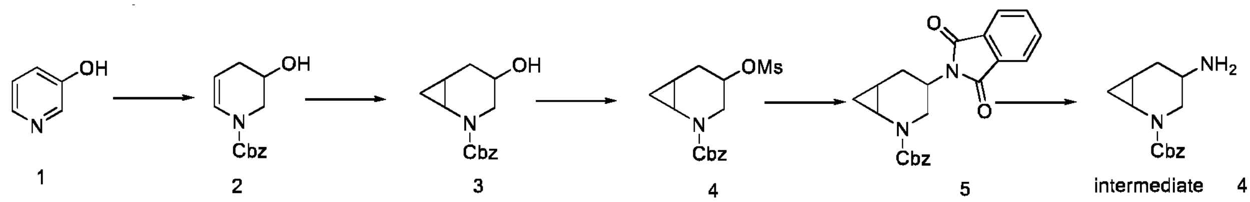

1 2 3 4 5 intermediate 4

Synthesis steps:

(1) Synthesis of Compound 2

12g(1eq) compound 1 was dissolved in 200ml of anhydrous methanol, 7.8g(0.8eq) $NaHCO_3$ was added, the temperature was cooled to -78 °C, 12.6g(3eq) $NaBH_4$ was added in batches, the temperature was controlled below -60 °C, 36.4ml Cbz-Cl(2.5eq) was dissolved in 50ml of anhydrous methanol, which was slowly added dropwise to the reaction system at -78 °C, and the temperature was kept for 1.5h, TLC monitored until the reaction was completed, 1mol/L NaOH(aq) was slowly added to the reaction solution to quench the reaction, extracted with EA, and 10g of compound 2 was obtained.

**(2) Synthesis of Compound 3**

Under nitrogen protection, 10g(1eq) compound 2 was dissolved in 150ml DCE, 100ml(1.5eq) 1M/L $ZnEt_2$ was slowly added dropwise, after the addtion, 26g(1.5eq) $CH_2I_2$ was added dropwise to react at RT for 6 hours. $NaHCO_3$(aq) was added to quench, extracted with DCM and dried, spin-dried to obtain 11g of compound 3 which was used directly in the next step.

**(3) Synthesis of Compound 4**

11g(1eq) compound 3 was dissolved in 110ml DCM, 14.3 g(2.5eq) DIEA was added, after the addition, 6.1g(1.2eq) MsCl was slowly added dropwise to react at rt. After the reaction was completed, water was added to quench, extracted with DCM, dried, spin-dried, and purified by column to obtain 6g of compound 4.

**(4) Synthesis of Compound 5**

6g(1eq) compound 4 was dissolved in 30ml DMF, 4g(1.2eq) of phthalimide potassium was added to react overnight at 100 °C. Water was added, extracted with EA, dried, spin-dried, and purified by column to obtain 5g of compound 5.

**(5) Synthesis of Intermediate 4**

5g(1eq) compound 5 was dissolved in 100ml of ethanol, 0.3g(2eq) of 80% hydrazine hydrate was added to react at 80 °C for 15min. The reaction solution was spin-dried, water was added, extracted with EA and dried, spin-dried to obtain 1.2g of intermediate 4. LC-MS[M+1]:247.1.

**Synthesis of Intermediate 5**

The structural formula of intermediate 5 is as follows:

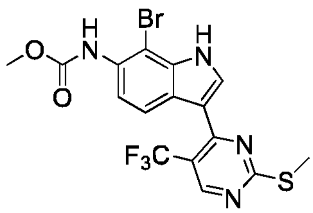

intermediate 5

The experimental process is as follows:

The synthetic route is as follows:

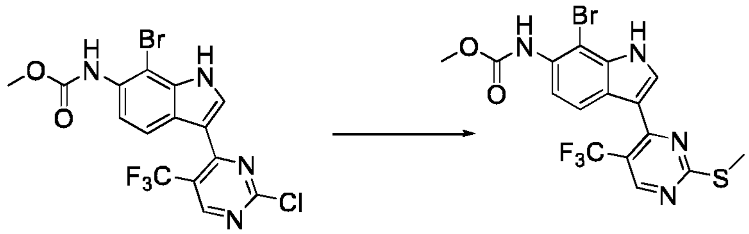

1 intermediate 5

Synthesis steps:

(1) Synthesis of Intermediate 5

Under nitrogen condition, 50g of compound 1, 11g of sodium thiomethoxide (1.3 eq.) and 800 mL 1,4-dioxane solvent were added to 1000 mL three-necked flask, the temperature was raised to reflux, and the temperature was kept to react. After TLC monitored until the raw materials were completely converted, the heating was stopped, the temperature was cooled to room temperature, the solution was extracted with EA and water, the organic phase was dried over anhydrous sodium sulfate, the solution was distilled and concentrated, recrystallized in EA, 39g of intermediate 5 was obtained with a yield of 71%.

**Synthesis of Intermediate 6 and Intermediate 7**

The structural formulas of intermediate 6 and intermediate 7 were as follows:

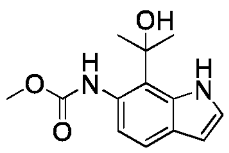

intermediate 6

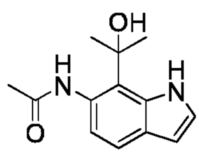

intermediate 7

The experimental process was as follows:
The synthetic route is as follows:

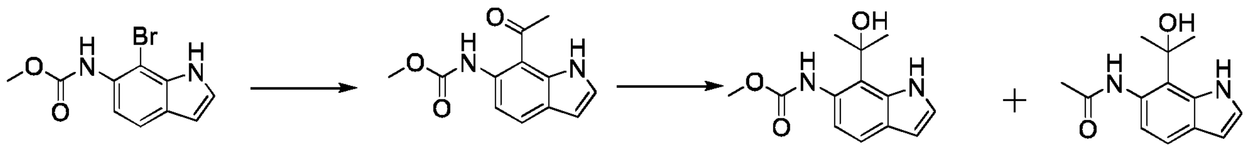

1 2 intermediate 6 intermediate 7

Synthesis steps:

(1) Synthesis of Compound 2

In a 250 ml three-necked flask, 27g(1 eq) compound 1 was dissolved in 250 ml n-butanol and stirred to dissolve, 27.6g(2eq) potassium carbonate, 20g(2.0eq) vinyl butyl ether, 2.3g(0.04eq) Xantphos and 0.44g(0.02eq) palladium acetate were added in turn, replaced with nitrogen for 3 times to react overnight at 95 °C. The reaction was monitored by TLC to be completed. Water was added, extracted with ethyl acetate twice, the ethyl acetate phase was transferred to a 250ml one-necked flask, 5ml (1.5eq) concentrated hydrochloric acid was added, stirred at room temperature for 3 hours. The reaction was monitored by TLC to be completed. 13.4g compound 2 was obtained by direct purification through a column.

### (2) Synthesis of Intermediate 6 and Intermediate 7

50mg(1 eq) compound 1 was added into a 50 ml one-necked flask, 1ml tetrahydrofuran was added, stirred to dissolve, 1.4ml (3eq) DAST was added dropwise at -20 °C, reacted at room temperature for 5h. The reaction was monitored by TLC to be completed. Water was added in the ice bath, extracted with ethyl acetate twice, and purified by column to obtain 10mg of intermediate 6 and 25mg of intermediate 7. Among them, the NMR of intermediate 7 was as follows: $^1$H NMR (400 MHz, DMSO-$d_6$) δ 10.68 (s, 1H), 9.39 (s, 1H), 7.33 (d, J = 8.2 Hz, 1H), 7.25 (t, J = 2.8 Hz, 1H), 6.76 (d, J = 8.3 Hz, 1H), 6.35 (dd, J = 3.2, 2.0 Hz, 1H), 5.72 (s, 1H), 2.00 (s, 3H), 1.62 (s, 6H).

## Example T-01

The compound synthesized by the invention:

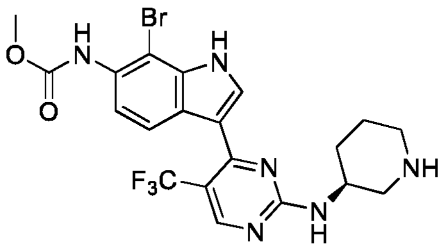

T-01

The experimental process was as follows:
The synthetic route was as follows:

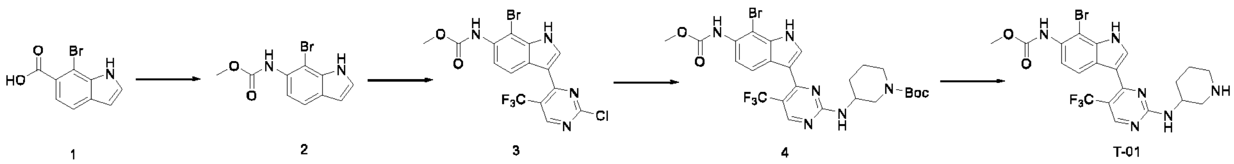

Synthesis step:

(1) Synthesis of Compound 2
11.5g(1.0eq) compound 1, 18.4g(1.4eq) DPPA, 8.7g(1.8eq) triethylamine and 100ml dioxane were added to a 250 ml three-necked flask, reacted at 80 °C for 1h under nitrogen protection, 3.5ml(2.0eq) methanol was added, the reaction was continued for 3h. The reaction was monitored by TLC to be completed. Water was added, extracted with EA, purified by column to obtain 9.2g of compound 2 as a white solid.
(2) Synthesis of Compound 3
8g(1.0eq) compound 1,12.7g(2.0eq) 2,4-dichloro-5-trifluoromethylpyrimidine, 6.8g(1.7eq) aluminum trichloride and 400ml DCE were added to a 500ml three-necked flask to react overnight at 80 °C under nitrogen protection. The reaction was monitored by TLC to be completed. Water was added to quench the reaction, EA was added to water phase to extract after liquid separation, organic layers were combined, spin-dried, then slurried by adding EA to obtain 5g of compound 3 as a purple solid.
(3) Synthesis of Compound 4
In a 100ml round bottom flask, 4.4g(1.0eq) compound 3, 4.1g(2.1eq) (S)-1-Boc-3-aminopiperidine and 50ml DMF were added to react overnight at room temperature. The reaction was monitored by TLC to be completed. Water was added, extracted with EA, purified by column to obtain 4.8g of compound 2 as a white solid.
(4) Synthesis of Compound T-01
2.0g compound 4, 10ml EA and 10ml 4M/L dioxane hydrochloride were added into a 25ml round bottom flask to react at room temperature for 4h. The reaction was monitored by TLC to be completed. Filter cake was obtained by suction filtration, and 1.1g of compound T-01 hydrochloride salt was obtained after washing with methyl tert-butyl ether. $^1$H NMR (400 MHz, DMSO-$d_6$) δ 12.07 (s, 1H), 9.20 (d, J = 14.0 Hz, 1H), 8.69 (d, J = 10.0 Hz, 1H), 8.30 (m, 1H), 8.08 (d, J = 7.6 Hz, 1H), 7.86 (s, 1H), 7.30 (t, J = 8.4 Hz, 1H), 4.21 (s, 1H), 3.72 (s, 3H), 3.13 (s, 2H), 2.89 - 2.69 (m, 2H), 2.07 (m, 1H), 1.89 (t, J = 10.0 Hz, 1H), 1.62 (m, 2H).

The hydrochloride salt of compound T-01 (50mg) was added with $NaHCO_3$ aqueous solution to be alkaline (pH = 8.0), extracted with EA and dried, spin-dried to obtain compound T-01. HPLC purity: 98.9%. LC-MS[M +1]:513.0, 515.0.

The following compounds were synthesized with reference to the method of Example T-01:

| Example | Compound structure | Characterization data |
|---|---|---|
| T-02 | 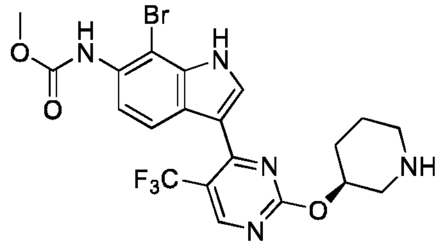 T-02 | LC-MS[M+1]:514.0,516.0 |
| T-36 | 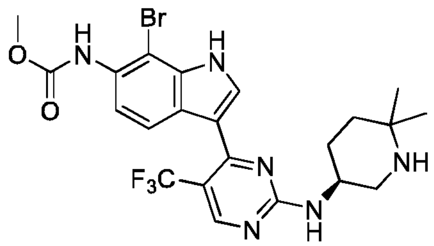 T-36 | LC-MS[M+1]:541.1,543.1<br><br>$^1$H NMR (400 MHz, DMSO-d6) δ 12.12 (s, 1H), 9.21 (d, J = 16.4 Hz, 1H), 8.70 (d, J = 9.6 Hz, 1H), 8.31 (m, 1H), 8.12 (d, J = 8.0 Hz, 1H), 7.86 (d, J = 10.0 Hz, 1H), 7.31 (m, 1H), 4.38 (s, 1H), 3.71 (s, 3H), 3.33 - 3.22 (m, 1H), 3.22 - 3.03 (m, 1H), 2.10 - 1.92 (m, 1H), 1.79 (m, 3H), 1.41 (t, J = 6.4 Hz, 6H) |
| T-48 | 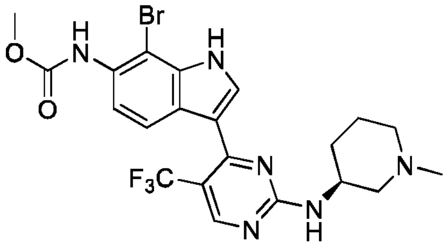 T-48 | LC-MS[M+1]:527.0,529.0<br><br>$^1$H NMR (400 MHz, DMSO-$d_6$) δ 11.97 (d, *J* = 17.6 Hz, 1H), 9.11 (d, *J* = 9.6 Hz, 1H), 8.60 (m, 1H), 8.23 (m, 1H), 7.91 - 7.70 (m, 2H), 7.19 (m, 1H), 4.02 (s, 1H), 3.65 (s, 3H), 3.01 - 2.81 (m, 1H), 2.64 (s, 1H), 2.18 (d, *J* = 2.8 Hz, 3H), 1.87 (s, 2H), 1.70 (d, *J* = 12.3 Hz, 1H), 1.63 - 1.41 (m, 1H), 1.30 (d, *J* = 11.3 Hz, 2H) |
| T-73 | 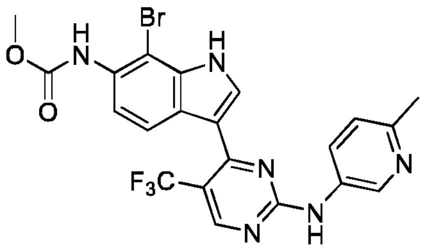 T-73 | LC-MS[M+1]:521.0,523.0<br><br>$^1$H NMR (400 MHz, DMSO-$d_6$) δ 12.07 (s, 1H), 10.24 (s, 1H), 9.15 (s, 1H), 8.89 - 8.63 (m, 2H), 8.09 (d, *J* = 8.8 Hz, 2H), 7.82 (s, 1H), 7.19 (d, *J* = 8.0 Hz, 2H), 3.66 (s, 3H), 2.43 (s, 3H) |

(continued)

| Example | Compound structure | Characterization data |
|---|---|---|
| T-74 | 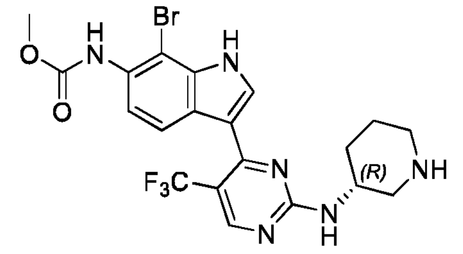 T-74 | LC-MS[M+1]:513.0,515.0 |
| T-96 | 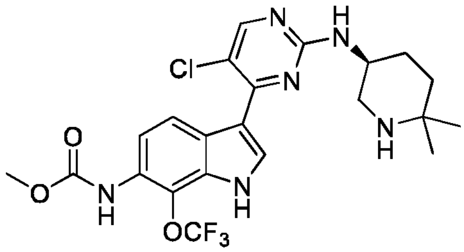 T-96 | LC-MS[M+1]:513.1,515.1 |
| T-108 | 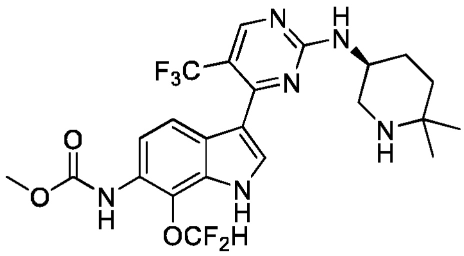 T-108 | LC-MS[M+1]:529.1 |
| T-109 | 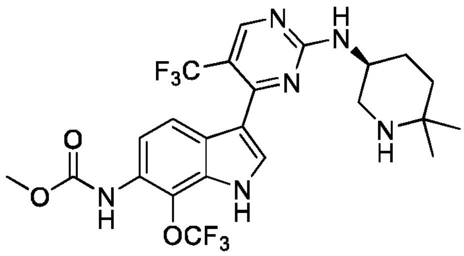 T-109 | LC-MS[M+1]:547.1 |
| T-110 | 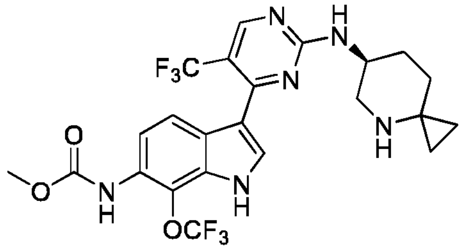 T-110 | LC-MS[M+1]:545.1 |

(continued)

| Example | Compound structure | Characterization data |
|---|---|---|
| T-121 | 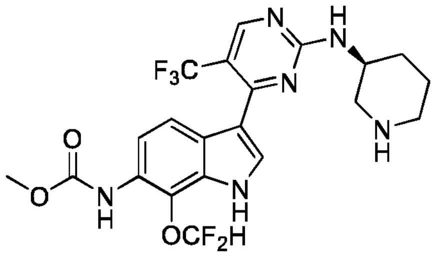<br>T-121 | LC-MS[M+1]:501.1 |
| T-122 | 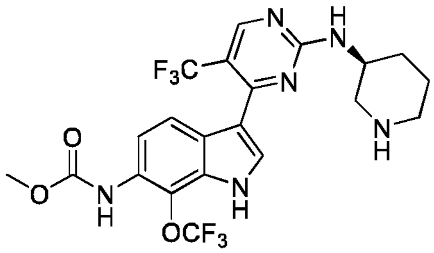<br>T-122 | LC-MS[M+1]:519.1 |
| T-123 | 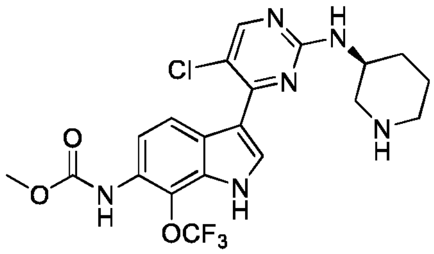<br>T-123 | LC-MS[M+1]:485.1,487.1 |
| T-142 | 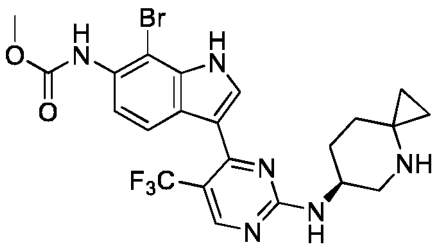<br>T-142 | LC-MS[M+1]:539.1,541.1 |
| T-143 | 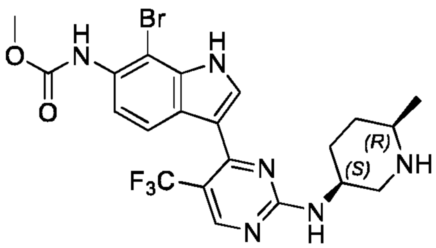<br>T-143 | LC-MS[M+1]:527.1,529.1 |

(continued)

| Example | Compound structure | Characterization data |
|---|---|---|
| T-144 | 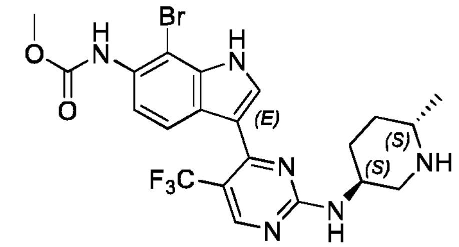 T-144 | LC-MS[M+1]:527.1,529.1 |
| T-154 | 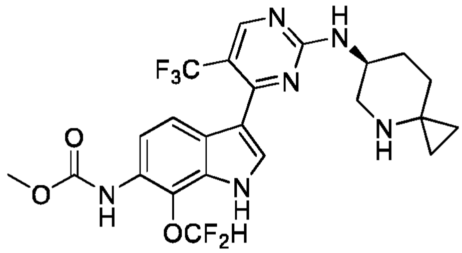 T-154 | LC-MS[M+1]:527.2 |
| T-155 | 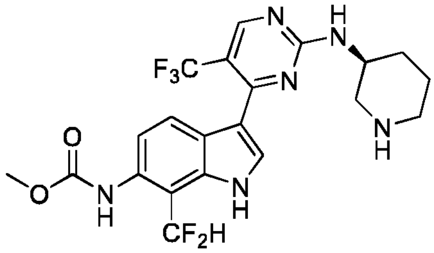 T-155 | LC-MS[M+1]:485.1 |
| T-156 | 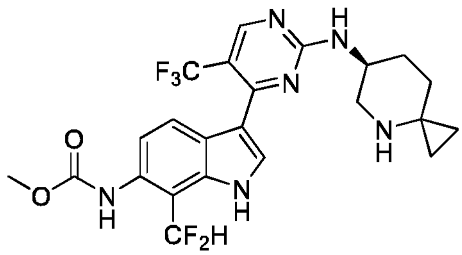 T-156 | LC-MS[M+1]:511.2 |
| T-157 | 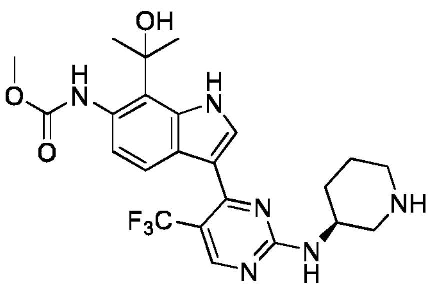 T-157 | LC-MS[M+1]:493.2 |

(continued)

| Example | Compound structure | Characterization data |
|---|---|---|
| T-158 | 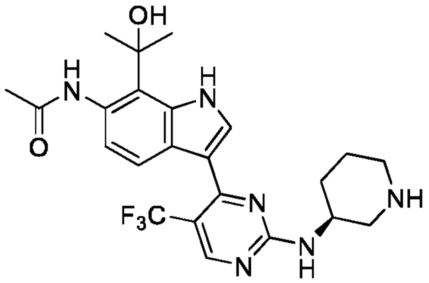 T-158 | LC-MS[M+1]:477.2 |
| T-169 | 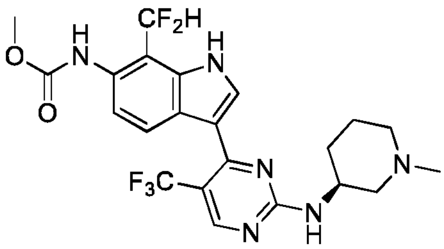 T-169 | LC-MS[M+1]:499.1 |
| T-173 | 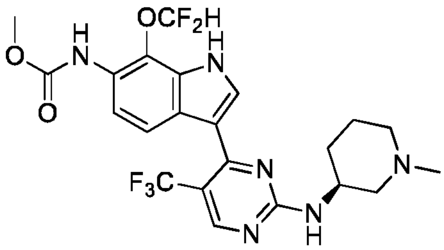 T-173 | LC-MS[M+1]:515.1 |

**Example T-07**

The compound synthesized by the invention:

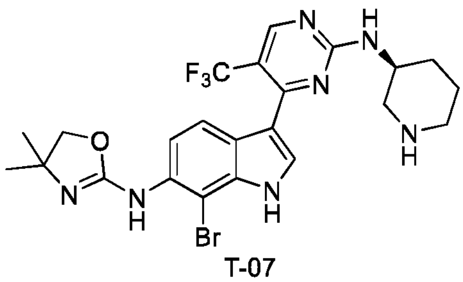

T-07

The experimental process was as follows:
The synthetic route is as follows:

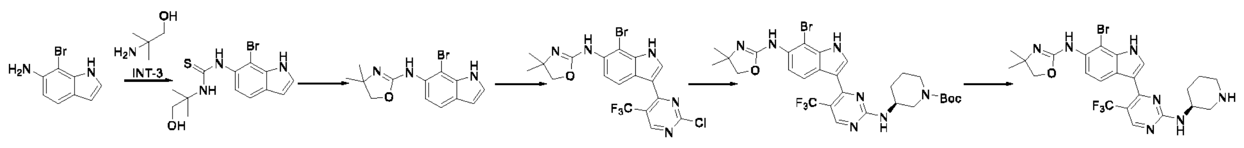


1 2 3 4 5 T-07

Synthesis step:

(1) Synthesis of Compound 2

0.28g(1.1eq) thiocarbonyldiimidazole was dissolved in 5ml of dry THF at -10 °C, 0.3g(1eq) compound 1 (dissolved in 5ml THF) was added, and then the temperature was kept to react for 5min. Then 10.17g(1.3eq) of INT-3 (dissolved into 1.5ml THF) was dropped into the reaction system, and then the temperature was gradually raised to room temperature to react. After the reaction was completed, water was added to quench, extracted with EA and dried, spin-dried, and purified by column to obtain 0.36g of compound 2.

(2) Synthesis of Compound 3

0.36g(1eq) compound 2 was dissolved in 15ml THF, 0.25g(6eq)sodium hydroxide (NaOH) was added, then 0.18g(0.9eq) p-toluenesulfonyl chloride was added to react at room temperature for 3h. After the reaction was completed, water was added to quench, extracted with EA, EA phase was dried, spin-dried, and purified by column to obtain 0.15g of compound 3.

(3) Synthesis of Compound 4

0.32g(3eq) 2,4-dichloro-5-trifluoromethylpyrimidinewas dissolved in 10ml of 1,2-dichloroethane (DCE), 0.1g(1.5eq) of aluminum trichloride ($AlCl_3$) was added, and the reaction was carried out at 80 °C for 30min under nitrogen protection. Then compound 3 was added to react overnight at 80 °C. After the reaction was completed, water was added to quench, DCE phase was separated, water phase was extracted with EA, DCE phase and EA phase were combined, dried, spin-dried, slurried in EA, and 0.1g of compound 4 was obtained.

(4) Synthesis of Compound 5

0.1g(1eq) compound 4 was dissolved in 1mL N,N-dimethylformamide (DMF), then 0.1g(2eq) (S)-1-Boc-aminopiperidine was added to react overnight at room temperature under nitrogen protection. Water was added to quench, extracted with EA and dried, spin-dried, and purified by column to obtain 0.1g of compound 5. LC-MS[M+1] :652.1,654.1.

(5) Synthesis of T-07

0.1g(1eq) compound 5 was taken and added into 1ml dioxane hydrochloride (4.0 mol/L), and the solution was reacted at room temperature until the reaction was completed. The reaction solution was added with $NaHCO_3$ aqueous solution to be alkaline, extracted with EA and dried, spin-dried, separated by preparation plate to obtain 0.02g of compound T-07. HPLC:89.8%. $^1$H NMR (400 MHz, DMSO-$d_6$) $\delta$ 11.59 (s, 1H), 8.55 (s, 1H), 8.12 (s, 1H), 7.65 (s, 1H), 6.81 (s, 1H), 4.02 (m, 2H), 3.90 (s, 1H), 3.06 (d, *J*= 12.0 Hz, 1H), 2.80 (d, *J*= 12.0 Hz, 1H), 2.49 - 2.36 (m, 2H), 2.07 - 1.80 (m, 2H), 1.65 (m, 1H), 1.57 - 1.34 (m, 4H), 1.25 (s, 6H).

## Examples T-08 and T-11

The compound synthesized by the invention:

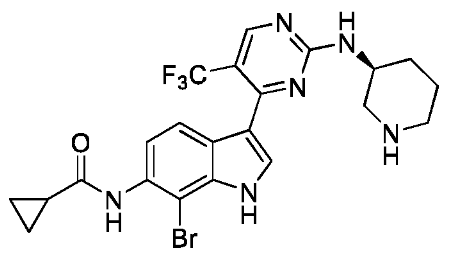

T-08

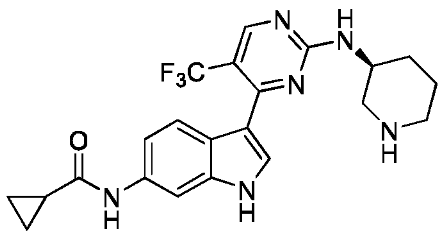

T-11

The experimental process was as follows:
The synthetic route is as follows:

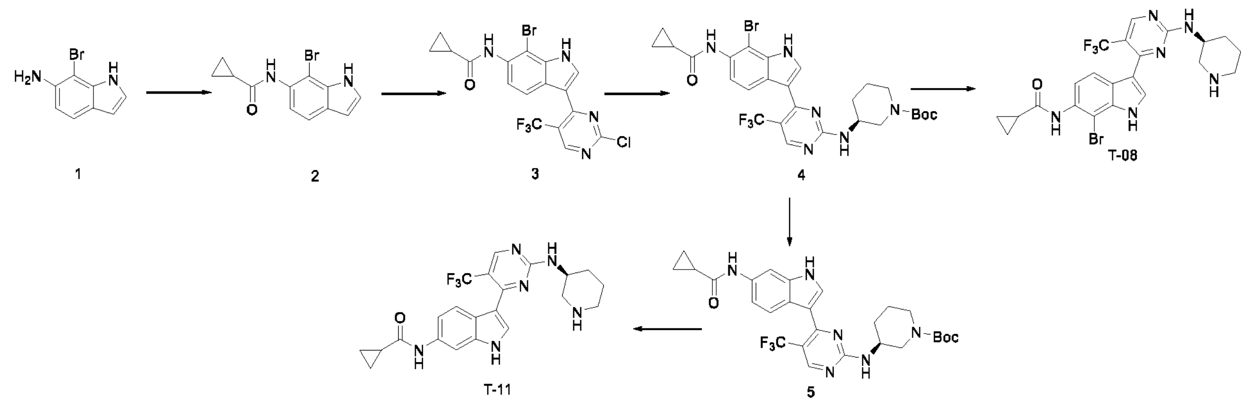

1 2 3 4 T-08 5 T-11

Synthesis step:

(1) Synthesis of Compound 2
1.0g(1.0eq) of cyclopropanecarboxylic acid was taken and added into a 50mL round bottom flask, 10mL N,N-dimethylformamide (DMF) was added, and stirred at room temperature. Subsequently, 2.7g(1.5eq) 2-(7-azabenzotriazole)-N,N,N',N'-tetramethylurea hexafluorophosphate (HATU) and 1.2g(2.0eq) diisopropyl ethylamine (DIPEA) were added. After reacting at room temperature for 5min, compound 1(1.0eq) was added, and the reaction was continued at room temperature. After the reaction was completed, 100mL of water was slowly added, solid was precipitated, and 1.1g of compound 2 was obtained by suction filtration, yield 85%.
(2) Synthesis of Compound 3
In a 50 mL round bottom flask, 1.4g(3eq) 2,4-dichloro-5-trifluoromethylpyrimidine, 30 mL dichloroethane (DCE) and 0.5g(1.7eq) $AlCl_3$ were added, the temperature was raised to 80 °C to react for 30min under nitrogen protection, 0.6g(1eq) compound 2 was added, and the reaction was continued at 80 °C under nitrogen protection. After the reaction was completed, water was added, extracted with EA, the organic layer was evaporated, and EA was added to slurry to obtain 200mg of compound 3.
(3) Synthesis of Compound 4
0.2g(1eq) compound 3 was dissolved in 6mL DMF, 0.18g of (S)-1-Boc-3-aminoperidine(2eq) was added to react overnight under stirring at room temperature. After the reaction was completed, water was added, extracted with EA and dried, spin-dried to obtain 0.24g of compound 4.
(4) Synthesis of Compound 5
110mg(1eq) compound 4 was dissolved in 4mL methanol (MeOH), 30mg Pd/C(2eq) and 2 drops of $Et_3N$ were added to react overnight under stirring at rt under $H_2$ protection. After the reaction was completed, the solution was filtered and the filtrate was spin-dried to obtain 92mg of compound 5.
(5) Synthesis of compound T-08:
100mg(1eq) compound 4 was taken and added into 3mL 4M/L dioxane hydrochlorideto react at room temperature. After the reaction was completed, $NaHCO_3$ aqueous solution was added to adjust the solution to be alkaline, extracted with EA and dried, spin-dried to obtain 47mg of compound T-08. HPLC:96.4%. $^1$H NMR (400 MHz, DMSO-d6) δ 11.94 (s, 1H), 9.85 (d, J = 12.0 Hz, 1H), 8.58 (d, J = 20.0 Hz, 1H), 8.23 (m, 1H), 7.80 (m, 2H), 7.26 (t, J = 7.6 Hz, 1H), 3.90 (s, 1H), 3.07 (d, J = 11.6 Hz, 1H), 2.87 - 2.72 (m, 1H), 2.44 (t, J = 11.2 Hz, 2H), 1.97 (m, 3H), 1.65 (d, J = 11.6 Hz, 1H), 1.42 (d, J = 19.6 Hz, 3H), 0.86 (m, 3H).
(6) Synthesis of T-11:
80mg (1eq) compound 5 was taken and added into 3mL 2M/L dioxane hydrochloride to react at room temperature. After the reaction was completed, $NaHCO_3$ aqueous solution was added to adjust the solution to be alkaline, extracted with EA and dried, spin-dried to obtain 35mg of compound T-11. HPLC:96.7%. $^1$H NMR (400 MHz, DMSO-d6) δ 11.84 (s, 1H), 10.31 (s, 1H), 8.65 (d, J = 5.2 Hz, 1H), 8.31 (m, 2H), 8.12 - 7.96 (m, 2H), 7.86 (s, 1H), 7.34 (m, 1H), 4.30 (s, 1H), 3.22 (s, 2H), 2.89 (t, J = 10.8 Hz, 1H), 2.00 - 1.84 (m, 2H), 1.84 - 1.48 (m, 3H), 1.30 (s, 3H), 0.97 - 0.81 (m, 3H).

The following compounds were synthesized with reference to the methods in Examples T-08 and T-11:

| Example | Compound structure | Characterization data |
| --- | --- | --- |
| T-09 | 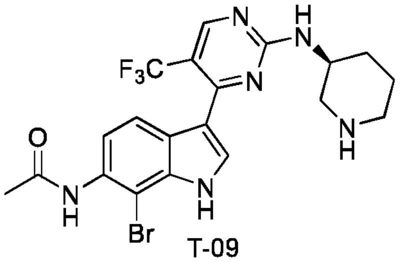 T-09 | $^1$H NMR (400 MHz, DMSO-d$_6$) δ 12.07 (s, 1H), 9.72 (d, *J* = 11.2 Hz, 1H), 8.70 (d, *J* = 7.2 Hz, 1H), 8.47-8.07 (m, 2H), 7.86 (s, 1H), 7.34 (m, 1H), 4.31 (s, 1H), 3.22 (d, *J* = 8.4 Hz, 2H), 2.92 (m, 2H), 2.15 (s, 3H), 2.10 - 1.88 (m, 2H), 1.72 (dt, *J* = 24.5, 11.2 Hz, 2H). |
| T-10 | 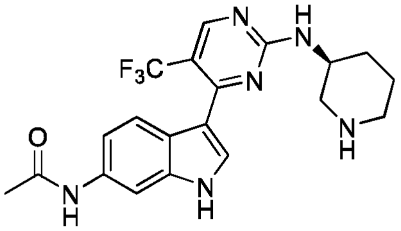 T-10 | $^1$H NMR (400 MHz, DMSO-*d*$_6$) δ 11.79 (s, 1H), 9.96 (s, 1H), 8.52 (d, *J* = 19.6 Hz, 1H), 8.42 - 8.14 (m, 1H), 8.07 (s, 1H), 7.84 - 7.62 (m, 2H), 7.16 (s, 1H), 3.90 (d, *J* = 35.2 Hz, 1H), 3.06 (s, 2H), 2.04 (d, *J* = 22.8 Hz, 5H), 1.62 (s, 2H), 1.44 (s, 2H). |

**Example T-12**

The compound synthesized by the invention:

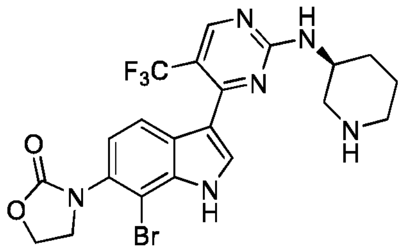

T-12

The experimental process was as follows:
The synthetic route is as follows:

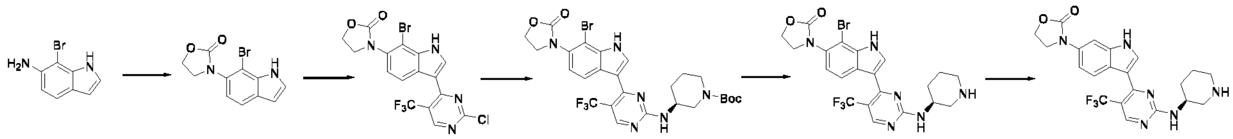

1 2 3 4 T-12 T-49

Synthesis step:

(1) Synthesis of Compound 2
2g(1eq) compound 1 was dissolved in 40mL THF at 0 °C, 2.4g(2.5eq) of triethylamine (TEA) was added, and then chloroethyl chloroformate was added dropwise into the reaction system. The reaction was monitored by TLC until the reaction of compound 1 was completed, 1.2g(5eq) sodium hydride (NaH) was added in batches at 0 °C and then reacted at room temperature. After the reaction was completed, water was added to quench, extracted with EA and dried, spin-dried, and purified by column to obtain 1.2g of compound 2. LC-MS[M+1]: 280.9, 282.9.
(2) Synthesis of Compound 3
2.78g (3eq) 2,4-dichloro-5-trifluoromethylpyrimidine was dissolved in 60 mL 1,2-dichloroethane (DCE), 0.85g(1.5eq) aluminum trichloride ($AlCl_3$) was added, and the reaction was carried out at 80 °C for 30min under nitrogen protection.

Then, compound 2 was added to react overnight at 80 °C, after the reaction was completed, water was added, DCE phase was separated, the aqueous phase was extracted with EA, DCE phase and EA phase were combined, dried, spin-dried, slurried in EA, and 1.3g of compound 3 was obtained. LC-MS[M+1]: 460.9,462.9.

(3) Synthesis of Compound 4

0.3g(1eq) compound 3 was dissolved in 3mL N,N-dimethylformamide (DMF), then 0.24g(2eq) (S)-1-Boc-3-aminopiperidine was added to react overnight at room temperature under nitrogen protection. Water was added to quench, extracted with EA and dried, spin-dried, and purified by column to obtain 0.3g of compound 4. LC-MS[M+1]: 625.1,627.1.

(4) Synthesis of Compound T-12 Hydrochloride

0.1g (1eq) compound 4 was taken and added into 2mL ethyl acetate (EA) and 1mL dioxane hydrochloride (4 mol/L), and the solution was reacted at room temperature to the reaction was completed. 0.06g of compound T-12 hydrochloride was obtained by filtration. HPLC purity: 96.4%.

(5) Synthesis of Compound T-12

0.1g(1eq) compound 4 was taken and added into 2mL dioxane hydrochloride (4 mol/L), to react at room temperature until the reaction was completed. $NaHCO_3$ aqueous solution was added until the solution was alkaline (pH = 8.0), extracted with EA and dried, spin-dried, separated by preparation plate to obtain 0.03g of compound T-12. HPLC purity: 93.4%. $^1H$ NMR (400 MHz, DMSO-$d_6$) δ 12.28 (s, 1H), 8.73 (s, 1H), 8.16 (d, $J$ = 8 Hz, 1H), 7.93 (d, $J$ = 6.0 Hz, 1H), 7.36 (t, $J$ = 8.8 Hz, 1H), 4.60 (t, $J$ = 8 Hz, 2H), 4.34 (s, 1H), 4.14 (t,$J$ = 8 Hz, 2H), 3.24 (s, 1H), 2.89 (s, 1H), 2.17 - 1.50 (m, 8H).

(6) Synthesis of Compound T-49

0.15g(1eq) compound 4 was dissolved in 10mL of methanol ($CH_3OH$), 0.01g of palladium carbon (Pd/C) and 3 drops of TEA were added to react overnight under $H_2$, and the solution was reacted at room temperature until the reaction was completed. The solution was filtered, dried and spin-dried to obtain 0.1g of compound T-49.

The following compounds were synthesized with reference to the method in Example T-12:

| Example | Compound structure | Characterization data |
|---|---|---|
| T-03 | 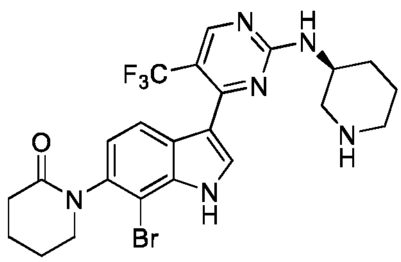 T-03 | 1H NMR (400 MHz, DMSO-d6) δ 11.88 (s, 1H), 8.60 (d, J = 20.4 Hz, 1H), 8.25 (m, 1H), 7.92 - 7.68 (m, 2H), 7.21 - 6.97 (m, 1H), 3.90 (s, 1H), 3.66-3.45 (m, 3H), 3.07 (d, J = 11.2Hz, 1H), 2.81 (d, J = 10.8 Hz, 1H), 2.49 - 2.36 (m, 3H), 1.97 (m, 5H), 1.74 - 1.37 (m, 4H). LC-MS[M +1]:537.1, 539.1 |
| T-04 | 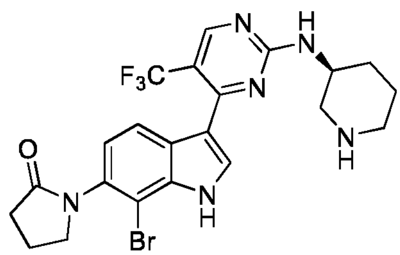 T-04 | $^1H$ NMR (400 MHz, DMSO-$d_6$) δ 12.11 (s, 1H), 8.60 (d, $J$ = 19.6 Hz, 1H), 8.27 (m, 1H), 7.82 (s, 2H), 7.14 (m,1H), 3.91 (s, 1H), 3.74 (s, 2H), 3.05 (m, 2H), 2.82 (s, 2H), 2.46 (t, $J$ = 8.0 Hz, 2H), 2.18 (m, $J$ = 8 Hz, 2H), 1.98 (t, $J$ = 8 Hz, 2H), 1.67 (s, 1H), 1.45 (s, 2H). LC-MS[M +1]:523.1, 525.1 |
| T-05 | 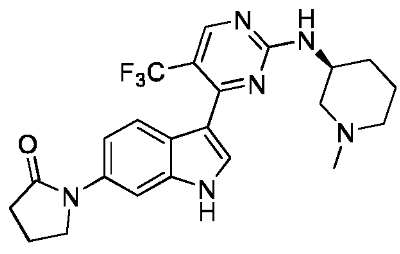 T-05 | LC-MS[M+1]:459.2 |

(continued)

| Example | Compound structure | Characterization data |
|---|---|---|
| T-06 | 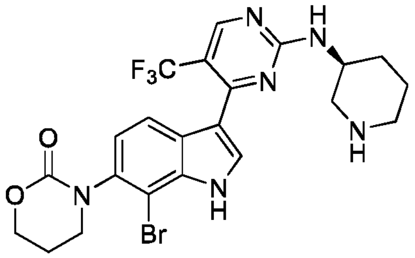 T-06 | $^1$H NMR (400 MHz, DMSO-$d_6$) δ 12.12 (s, 1H), 8.60 (d, *J* = 19.6 Hz, 1H), 8.27 (m, 1H), 7.90 - 7.77 (m, 2H), 7.24 (m, 1H), 4.49 - 4.30 (m, 2H), 3.92 (s, 1H), 3.73 - 3.47 (m, 3H), 3.13 - 3.00 (m, 1H), 2.83 (s, 1H), 2.47 (m, 2H), 2.17 (s, 2H), 1.99 (m, 1H), 1.66-1.47 (m, 3H). LC-MS[M +1]:539.1, 541.1 |
| T-13 | 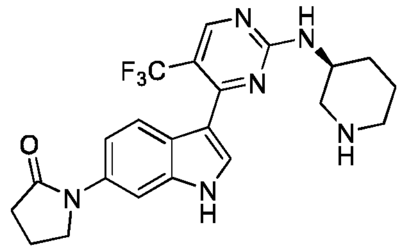 T-13 | $^1$H NMR (400 MHz, Methanol-$d_4$) δ 8.59 (s, 1H), 8.37 (s, 1H), 8.24 (s, 1H), 7.78 (s, 1H), 7.61 (s, 1H), 4.03 (t, *J* = 7.2 Hz, 2H), 3.79 - 3.54 (m, 4H), 2.65 (t, *J* = 8.0 Hz, 2H), 2.39 - 1.78 (m, 7H). LC-MS[M+1]:445.1 |
| T-18 | 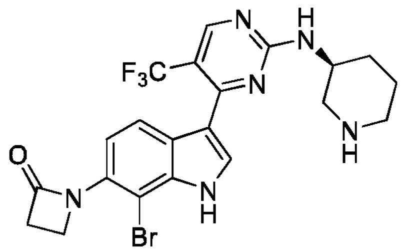 T-18 | $^1$H NMR (400 MHz, Methanol-$d_4$) δ 8.60 (s, 1H), 8.26 (m, 2H), 7.92 (s, 1H), 7.42 (d, *J* = 8.4 Hz, 1H), 6.58 (m, 1H), 6.41 (m, 1H), 5.84 (d, *J* = 10Hz, 1H), 4.60 (s, 1H), 4.34 (s, 1H), 3.56 (m, 1H), 3.02 (m, 2H), 2.34 - 1.69 (m, 4H). LC-MS[M +1]:509.1, 511.1 |
| T-46 | 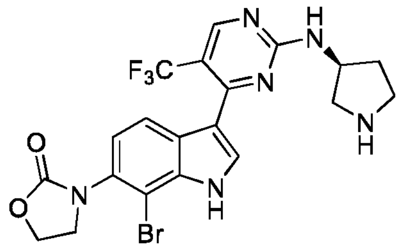 T-46 | $^1$H NMR (400 MHz, Methanol-$d_4$) δ 8.63 (s, 1H), 8.32 (s, 1H), 7.97 (s, 1H), 7.28 (d, *J* = 8.4 Hz, 1H), 4.67 - 4.53 (m, 2H), 4.16 - 4.02 (m, 2H), 3.65-3.36 (m, 5H), 2.44 (m, 1H), 2.24 m, 1H). LC-MS[M +1]:511.0, 513.0 |
| T-47 | 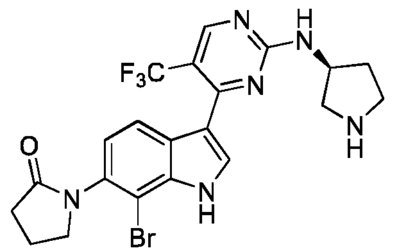 T-47 | $^1$H NMR (400 MHz, Methanol-$d_4$) δ 8.60 (s, 1H), 8.38 (s, 1H), 8.16 (s, 1H), 7.28 (s, 1H), 4.69 (s, 1H), 3.88 (t, *J* = 7.2 Hz, 2H), 3.72 - 3.52 (m, 2H), 3.48 (m, 2H), 2.63 (t, *J* = 8.0 Hz, 2H), 2.51 (s, 1H), 2.32 (t, *J* = 8.0 Hz, 3H). |

## Example T-14

The compound synthesized by the invention:

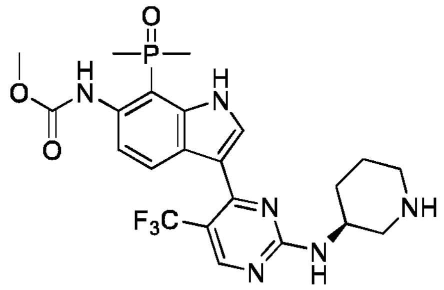

T-14

The experimental process was as follows:
The synthetic route is as follows:

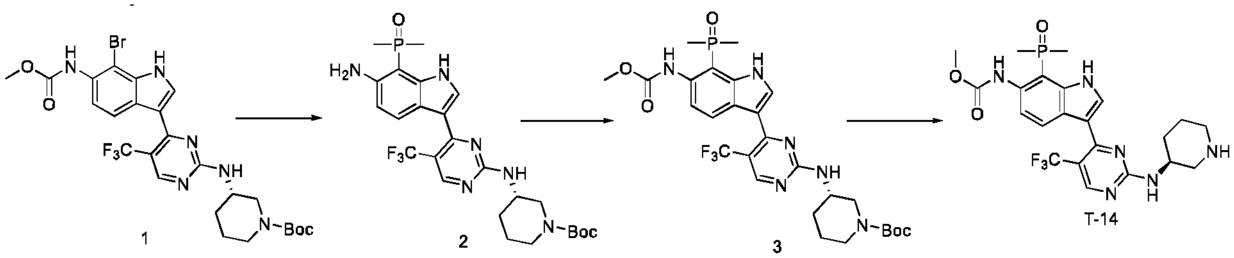

1 2 3 T-14

Synthesis step:

(1) Synthesis of Compound 2
230mg(1eq) compound 1 was dissolved in 6mL of dry DMF, 16mg(0.2eq) palladium acetate, 84mg(0.4eq) Xantphos and 226mg(8eq) dimethylphosphine oxide were added to react at 140 °C under nitrogen protection. After the reaction was completed, water was added, extracted with EA and dried, spin-dried to obtain 40mg of compound 2 (crude product).
(2) Synthesis of Compound 3
50mg(1eq) compound 4 was dissolved in 3ml methyl chloroformate, 100mg $Na_2CO_3$ was added to react at room temperature. After the reaction was completed, water was added, extracted with EA and dried, spin-dried to obtain 40mg of compound 3 (crude product).
(3) Synthesis of Compound T-14
40mg (1eq) compound 3 was taken and added into 2mL 4M/L dioxane hydrochlorideto react at room temperature. After the reaction was completed, $NaHCO_3$ aqueous solution was added to adjust the solution to be alkaline, extracted with EA and dried, spin-dried, and 10mg of compound TY-2652 was obtained from a preparative plate. HPLC:88%. $^1H$ NMR (400 MHz, DMSO-$d_6$) δ 11.75 (s, 1H), 8.69 (s, 1H), 8.50 - 8.04 (m, 2H), 7.95 (d, *J* = 14.7 Hz, 1H), 7.65 (s, 1H), 4.31 (s, 1H), 3.71 (s, 3H), 3.22 (s, 1H), 3.01 - 2.82 (m, 2H), 1.95 (d, *J* = 13.6 Hz, 6H), 1.83 - 1.47 (m, 4H), 1.40 (d, *J* = 4.0 Hz, 2H).

The following compounds were synthesized with reference to the method in Example T-14:

| Example | Compound structure | Characterization data |
|---|---|---|
| T-103 | 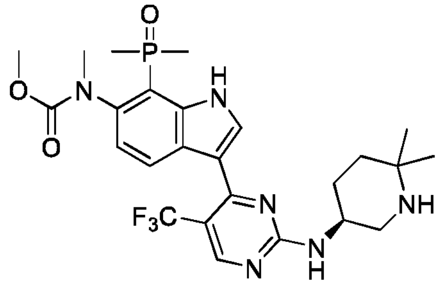 T-103 | LC-MS[M+1]:553.2 |

**Example T-15**

The compound synthesized by the invention:

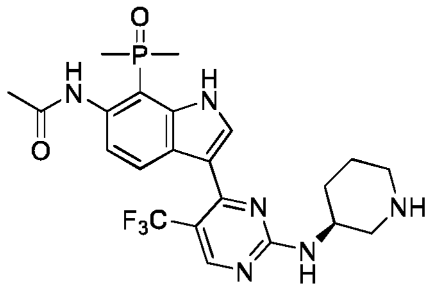

T-15

The experimental process was as follows:
The synthetic route is as follows:

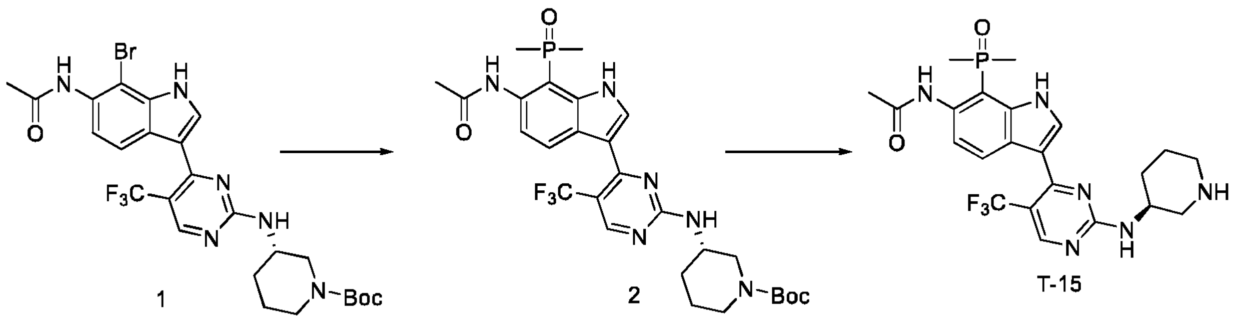

Synthesis step:

(1) Synthesis of Compound 2
250mg(1eq) compound 1 was dissolved in 6ml of dry DMF, 19mg(0.2eq) palladium acetate, 97mg(0.4eq)Xantphos and 262mg(8eq) dimethylphosphine oxide were added to react at 140 °C for 1.5h under nitrogen protection. Water was added, extracted with EA and dried, spin-dried to obtain 150mg of crude compound 2.
(2) Synthesis of compound T-15:
150mg (1eq) compound 2 was taken and added into 4mL 4M/L dioxane hydrochloride to react at rt. After the reaction was completed, $NaHCO_3$ aqueous solution was added to adjust the solution to be alkaline, extracted with EA and dried, spin-dried to obtain 10mg of compound T-15. HPLC:96.7%. $^1$H NMR (400 MHz, DMSO-$d_6$) $\delta$ 11.73 (s, 1H), 10.36 (d, $J$ = 30.0 Hz, 1H), 8.57 (d, $J$= 14.8 Hz, 1H), 8.16 (d, $J$= 193.2 Hz, 2H), 7.80 (s, 1H), 7.39 (s, 1H), 7.27 - 7.03 (m, 1H), 3.94 (s, 1H), 3.11 (s, 2H), 2.87 (d, $J$= 16.9 Hz, 2H), 2.07 (s, 3H), 1.90 (m, 6H), 1.68 (s, 2H), 1.47 (s, 2H).

**Example T-16**

The compound synthesized by the invention:

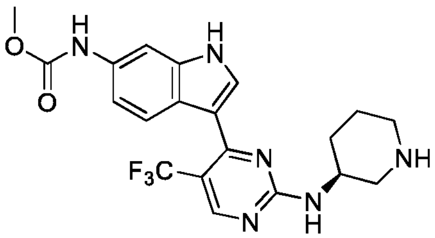

T-16

The experimental process was as follows:
The synthetic route is as follows:

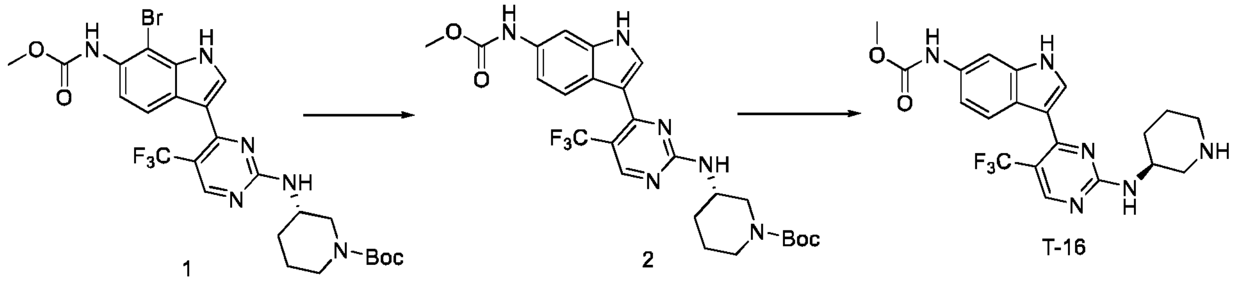

Synthesis step:

(1) Synthesis of Compound 2
220mg(1eq) compound 1 was dissolved in 6mL MeOH, 50mg Pd/C and 3 drops of $Et_3N$ were added to react overnight under stirring at room temperature under $H_2$ protection. After the reaction was completed, the solution was filtered and the filtrate was spin-dried to obtain 200mg of compound 2.
(2) Synthesis of Compound T-16
200mg(1eq) compound 2 was taken and added into 3mL 4M/L dioxane hydrochloride to react at room temperature. After the reaction was completed, $NaHCO_3$ aqueous solution was added to adjust the solution to be alkaline, extracted with EA and dried, spin-dried to obtain 10mg of compound T-16. HPLC:92.6%. $^1$H NMR (400 MHz, DMSO-$d_6$) δ 11.78 (s, 1H), 9.63 (s, 1H), 8.51 (d, $J$ = 18.4 Hz, 1H), 8.26 (m, 1H), 7.77 (d, $J$ = 12.4 Hz, 2H), 7.68 (d, $J$ = 8.0 Hz, 1H), 7.16 (m, 1H), 3.90 (d, $J$= 31.2 Hz, 1H), 3.68 (s, 3H), 3.16 - 2.97 (m, 1H), 2.88 - 2.70 (m, 1H), 2.43 (m, 2H), 2.12 - 1.86 (m, 1H), 1.65 (s, 1H), 1.45 (m, 2H).

**Example T-17**

The compound synthesized by the invention:

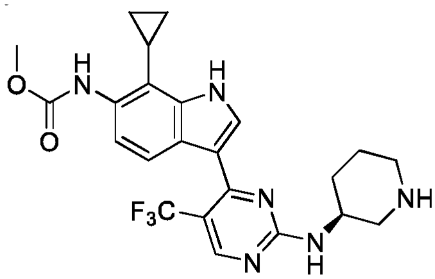

T-17

The experimental process was as follows:

The synthetic route is as follows:

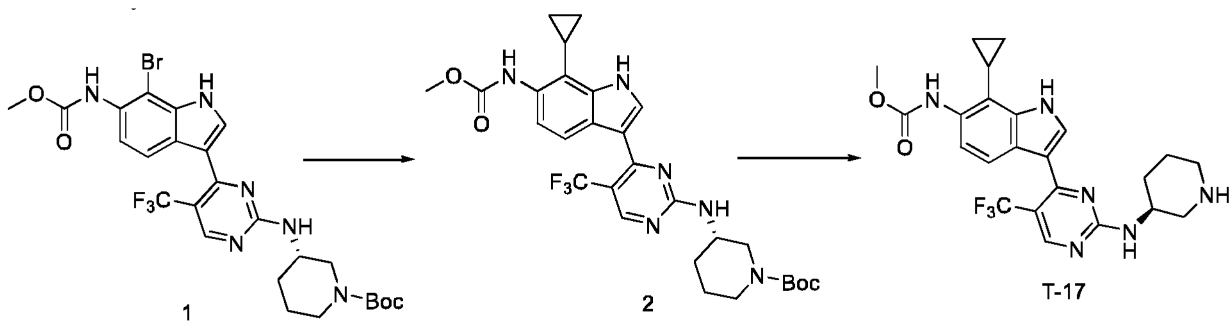

1 2 T-17

Synthesis step:

(1) Synthesis of Compound 2

0.5g(1.0eq) compound 1 was dissolved in 10mL of dioxane and 1mL of water, and 0.34g(3eq) of K2CO3, 0.35g(5eq) of cyclopropylboronic acid and 0.12g(0.2eq) of Pd(dppf)Cl2 were added to react at 100 °C under nitrogen protection. After the reaction was completed, water was added, extracted with EA and dried, spin-dried, and purified by column to obtain 0.40g of compound 3.

(2) Synthesis of Compound T-17

200mg (1eq) compound 3 was taken and added into 4mL 4M/L dioxane hydrochloride solution and 3mL EA at room temperature to react at room temperature. After the reaction was completed, filtered, the filter cake was washed with methyl tert-butyl ether to obtain 175mg of compound T-17 hydrochloride. HPLC:93.9%. $^1$H NMR (400 MHz, DMSO-$d_6$) δ 11.47 (d, *J* = 13.6 Hz, 1H), 8.82 (d, *J* = 13.6 Hz, 1H), 8.53 (d, *J* = 18.4 Hz, 1H), 8.16 (m, 1H), 7.82 - 7.58 (m, 2H), 7.11 (s, 1H), 3.90 (s, 1H), 3.66 (s, 3H), 3.05 (m, 1H), 2.80 (d, *J* = 12.0 Hz, 2H), 2.05 - 1.82 (m, 2H), 1.64 (d, *J* = 10.4 Hz, 1H), 1.42 (d, *J* = 18.8 Hz, 2H), 1.23 (s, 1H), 1.08 - 0.92 (m, 2H), 0.53 (d, *J* = 5.6 Hz, 2H).

The following compounds were synthesized with reference to the method in Example T-17:

| Example | Compound structure | Characterization data |
|---|---|---|
| T-68 | 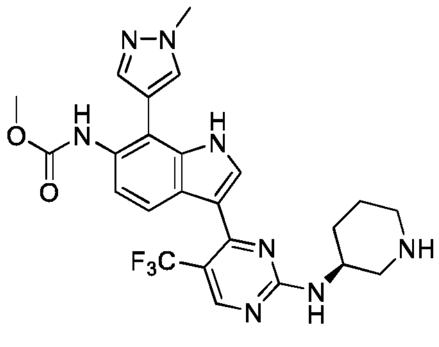 T-68 | LC-MS[M+1]:515.2<br><br>$^1$H NMR (400 MHz, DMSO-$d_6$) δ 11.49 (d, *J* = 13.1 Hz, 1H), 9.26 (s, 1H), 9.09 (s, 1H), 8.75 - 8.25 (m, 2H), 7.99 (s, 1H), 7.74 (d, *J* = 6.0 Hz, 1H), 7.66 (s, 1H), 4.30 (s, 1H), 4.03 (d, *J* = 7.1 Hz, 3H), 3.82 (s, 3H), 3.57 (d, *J* = 3.1 Hz, 3H), 3.38 (s, 1H), 3.03 - 2.77 (m, 2H), 1.99 (s, 1H), 1.96 - 1.85 (m, 1H), 1.83-1.50 (m, 2H), 1.32 - 1.13 (m, 1H). |
| T-83 | 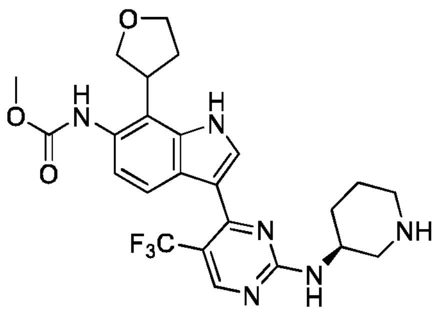 T-83 | LC-MS[M+1]:505.2 |

(continued)

| Example | Compound structure | Characterization data |
|---|---|---|
| T-84 | 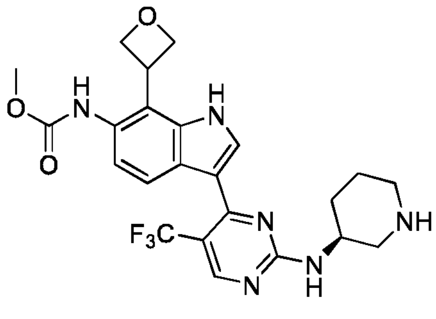 T-84 | LC-MS[M+1]:491.1 |
| T-86 | 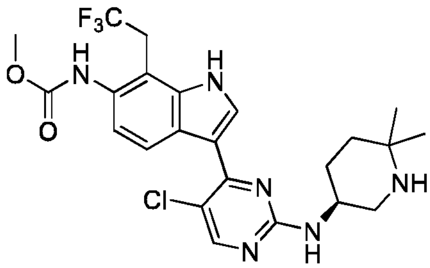 T-86 | LC-MS[M+1]:511.1,513.1 |
| T-90 | 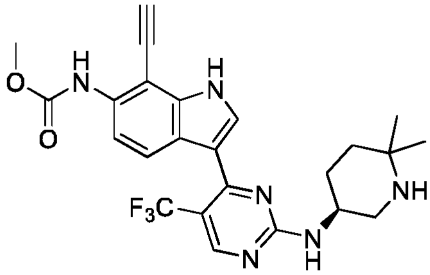 T-90 | LC-MS[M+1]:487.1 |
| T-97 | 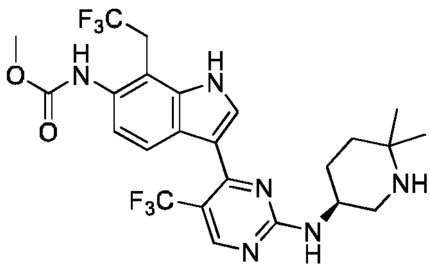 T-97 | LC-MS[M+1]:545.2 |

(continued)

| Example | Compound structure | Characterization data |
|---|---|---|
| T-99 | 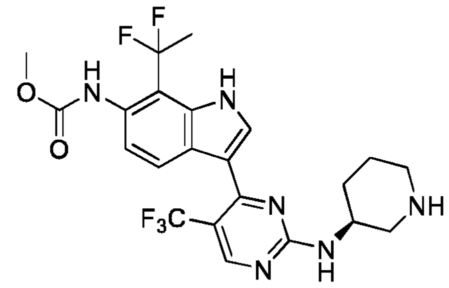 T-99 | LC-MS[M+1]:499.1 |
| T-104 | 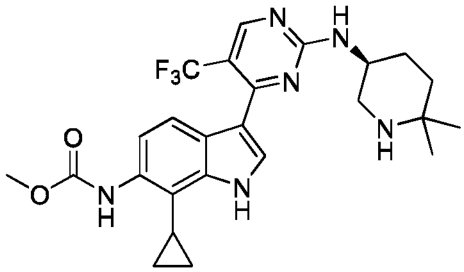 T-104 | LC-MS[M+1]:503.2 |
| T-105 | 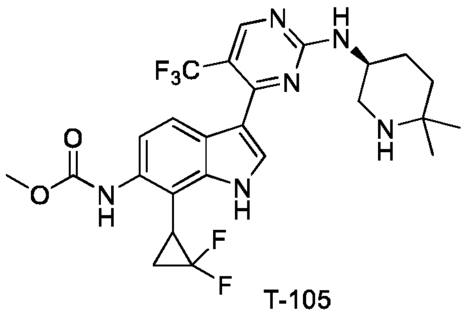 T-105 | LC-MS[M+1]:539.1 |
| T-106 | 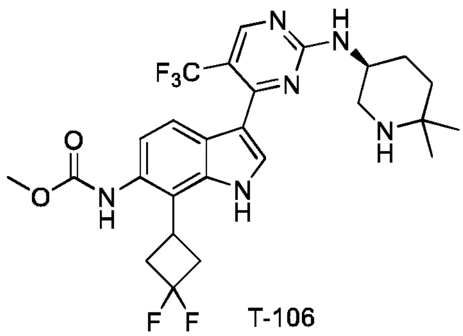 T-106 | LC-MS[M+1]:553.2 |

(continued)

| Example | Compound structure | Characterization data |
|---|---|---|
| T-114 | 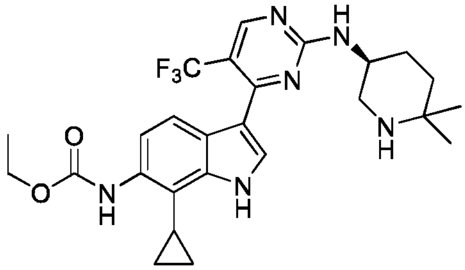 T-114 | LC-MS[M+1]:517.2 |
| T-127 | 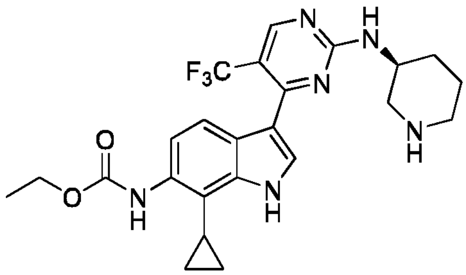 T-127 | LC-MS[M+1]:489.2 |
| T-128 | 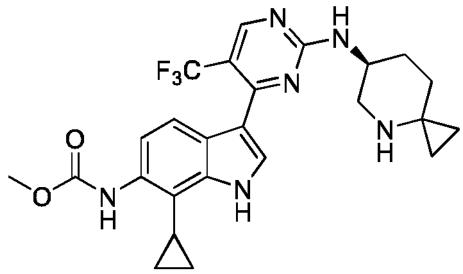 T-128 | LC-MS[M+1]:501.2 |
| T-129 | 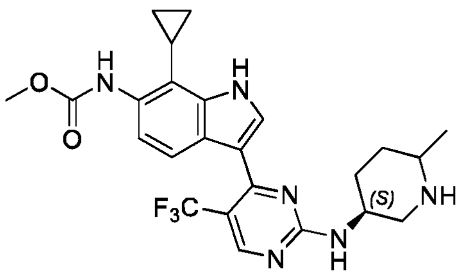 T-129 | LC-MS[M+1]:489.2 |

(continued)

| Example | Compound structure | Characterization data |
|---|---|---|
| T-130 | 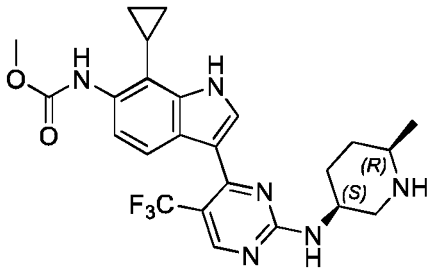 T-130 | LC-MS[M+1]:489.2 |
| T-131 | 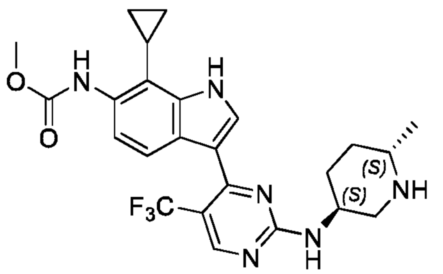 T-131 | LC-MS[M+1]:489.2 |
| T-171 | 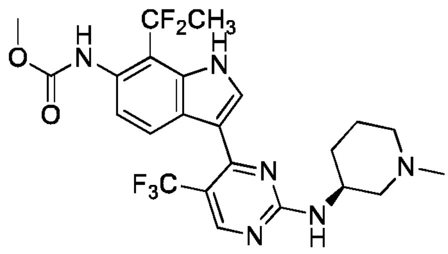 T-171 | LC-MS[M+1]:513.2 |
| T-174 | 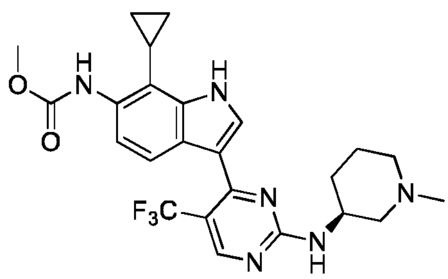 T-174 | LC-MS[M+1]:489.2 |

**Example T-20**

The compound synthesized by the invention:

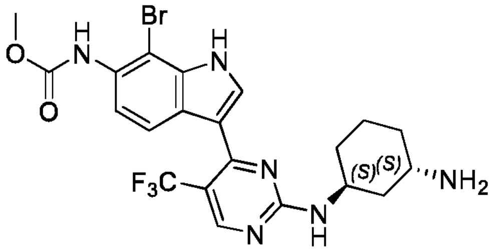

T-20

The experimental process was as follows:
The synthetic route is as follows:

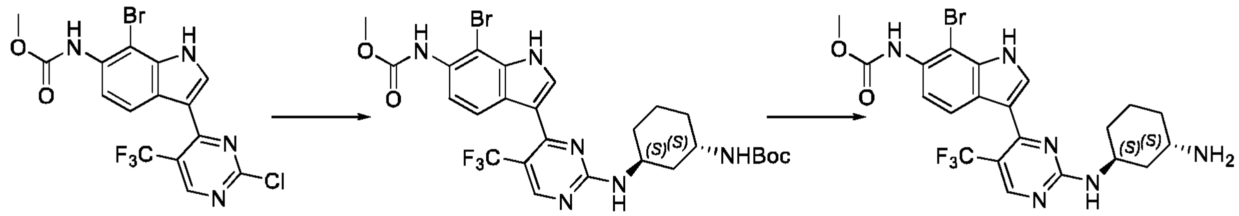

1 2 T-20

Synthesis step:

(1) Synthesis of Compound 2
0.25g(1eq) compound 1 was dissolved in 3mL (N,N-dimethylformamide) DMF, and 0.14g(1.1eq) tert-butyl (1S,3S)-3-aminocyclohexylcarbamate (CAS:1788036-28-1) was added to react overnight at room temperature. Water was added, extracted with EA and dried, spin-dried, and purified by column to obtain 0.2g of compound 2. LC-MS[M +1]:627.1, 629.1.
(2) Synthesis of T-20 Hydrochloride and T-20
0.2g(1eq) compound 2 was dissolved into 5mL of ethyl acetate (EA), 2mL of dioxane hydrochloride (4.0mol/L) was added to react until the reaction was completed at room temperature. The solution was filtered to obtain 0.13g of compound T-20 hydrochloride. HPLC:98.7%. $^1$H NMR (400 MHz, Methanol-$d_4$) δ 8.63 (s, 1H), 8.37 (s, 1H), 8.16 (s, 1H), 7.61 (s, 1H), 4.69 (s, 1H), 3.78 (s, 3H), 3.61 - 3.44 (m, 1H), 2.52 - 1.39 (m, 9H).

The compound T-20 hydrochloride (50mg) was added with $NaHCO_3$ aqueous solution to be alkaline (pH = 8.0), extracted with EA and dried, spin-dried to obtain compound T-20. HPLC purity: 98.4%. LC-MS[M +1]:527.1, 529.1.
The following compounds were synthesized with reference to the method in Example T-20:

| Example | Compound structure | Characterization data |
|---|---|---|
| T-21 | 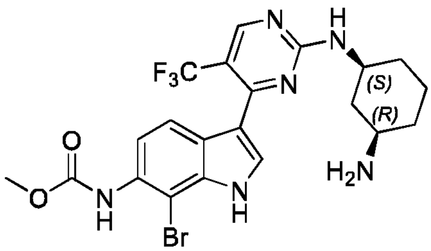 T-21 | $^1$H NMR (400 MHz, Methanol-$d_4$) δ 8.58 (s, 1H), 8.37 (s, 1H), 8.20 (s, 1H), 7.59 (s, 1H), 4.30 (s, 1H), 3.79 (s, 3H), 2.42 (s, 1H), 2.17 (m, 4H), 1.72 - 1.24 (m, 5H). |

(continued)

| Example | Compound structure | Characterization data |
|---|---|---|
| T-22 | 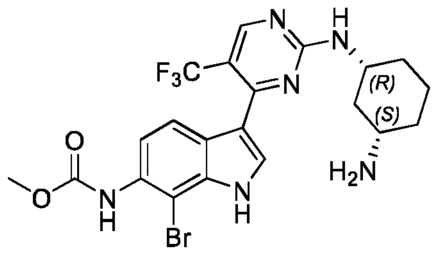<br>T-22 | LC-MS[M +1]:527.1,529.1 |
| T-23 | 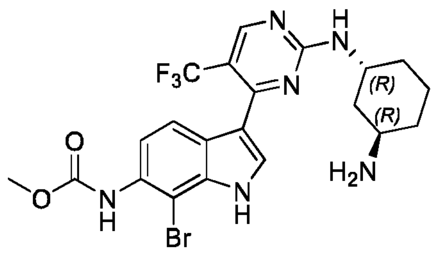<br>T-23 | LC-MS[M +1]:527.1,529.1 |
| T-24 | 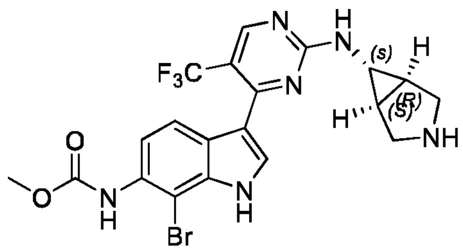<br>T-24 | $^1$H NMR (400 MHz, DMSO-$d_6$) δ 12.11 (s, 1H), 9.35 m, 2H), 9.10 (s, 1H), 8.61 (s, 2H), 8.42 (s, 1H), 8.24 (s, 1H), 7.78 (s, 1H), 7.28 (d, *J* = 8.4 Hz, 1H), 3.66 (s, 3H), 3.57 (s, 2H), 2.96 (s, 1H), 2.01 (s, 2H). |
| T-25 | 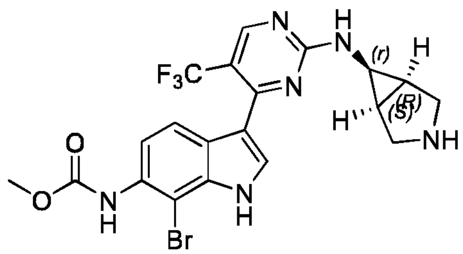<br>T-25 | LC-MS[M +1]:511.0, 513.0 |
| T-26 | 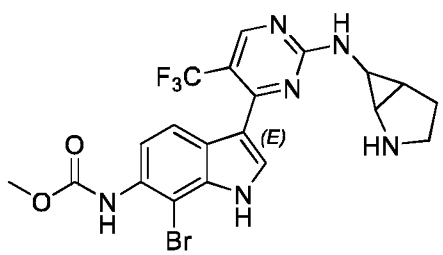<br>T-26 | LC-MS[M +1]:511.0, 513.0 |

(continued)

| Example | Compound structure | Characterization data |
|---|---|---|
| T-29 | 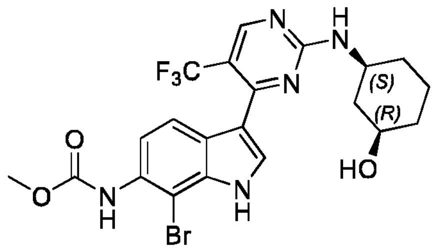 T-29 | $^1$H NMR (400 MHz, Methanol-$d_4$) δ 8.48 (s, 1H), 8.28 (s, 1H), 7.83 (s, 1H), 7.39 (s, 1H), 4.08 (s, 1H), 3.76 (s, 3H), 3.68 (s, 1H), 2.32 (s, 1H), 2.10 - 1.77 (m, 3H), 1.47 - 1.21 (m, 4H). |
| T-30 | 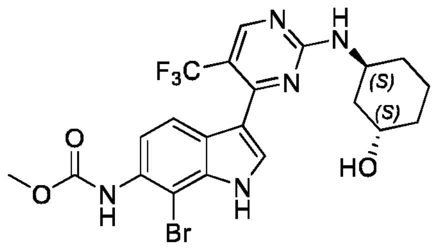 T-30 | LC-MS[M +1]:528.1, 530.1 |
| T-39 | 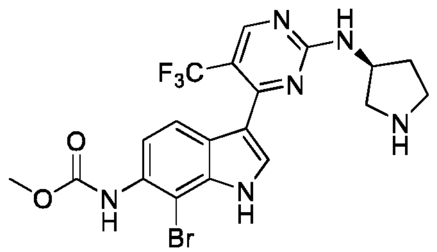 T-39 | LC-MS[M +1]:499.0, 501.0 |
| T-53 | 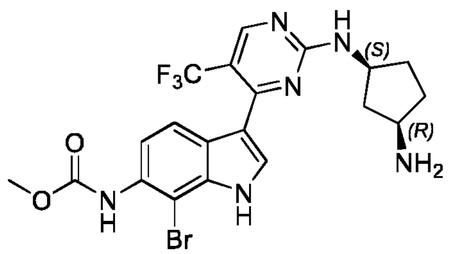 T-53 | $^1$H NMR (400 MHz, Methanol-$d_4$) δ 8.59 (s, 1H), 8.39 (s, 1H), 8.19 (s, 1H), 7.60 (s, 1H), 3.79 (s, 3H), 3.74 (m, 1H), 2.85 - 2.70 (m, 1H), 2.36 - 1.73 (m, 6H). |
| T-54 | 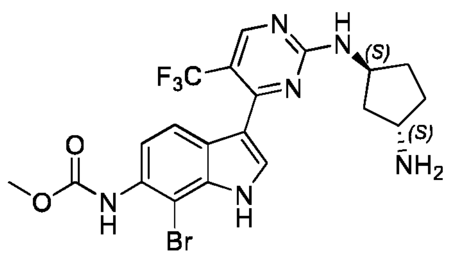 T-54 | $^1$H NMR (400 MHz, Methanol-$d_4$) δ 8.57 (s, 1H), 8.39 (s, 1H), 8.14 (s, 1H), 7.59 (s, 1H), 3.85 (m, 1H), 3.74 (s, 3H), 2.49 -2.29 (m, 4H), 1.97 - 1.63 (m, 3H). |

(continued)

| Example | Compound structure | Characterization data |
|---|---|---|
| T-55 | 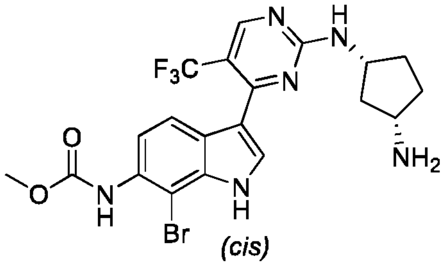 (cis) T-55 | $^1$H NMR (400 MHz, Methanol-$d_4$) δ 8.59 (s, 1H), 8.39 (s, 1H), 8.18 (s, 1H), 7.60 (s, 1H), 3.79 (s, 3H), 3.73 (m, 1H), 2.78 (m, 1H), 2.35 - 1.76 (m, 6H). |
| T-56 | 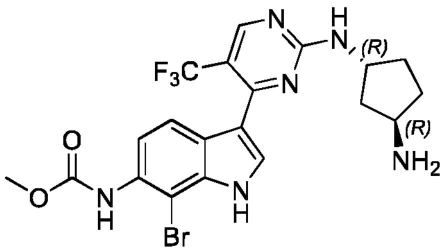 T-56 | $^1$H NMR (400 MHz, Methanol-$d_4$) δ 8.57 (s, 1H), 8.39 (s, 1H), 8.10 (s, 1H), 7.59 (s, 1H), 3.91 (m, 1H), 3.78 (s, 3H), 2.52 - 2.14 (m, 4H), 2.01 - 1.63 (m, 3H). |
| T-61 | 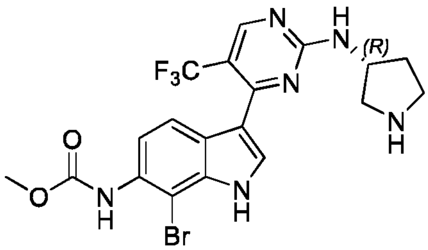 T-61 | LC-MS[M +1]:499.0, 501.0 |

**Example T-27**

The compound synthesized by the invention:

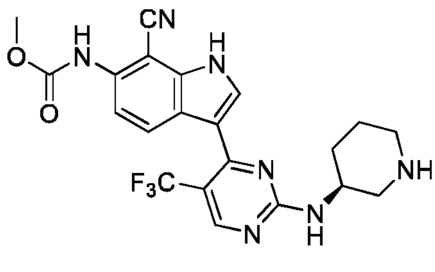

T-27

The experimental process was as follows:
The synthetic route is as follows:

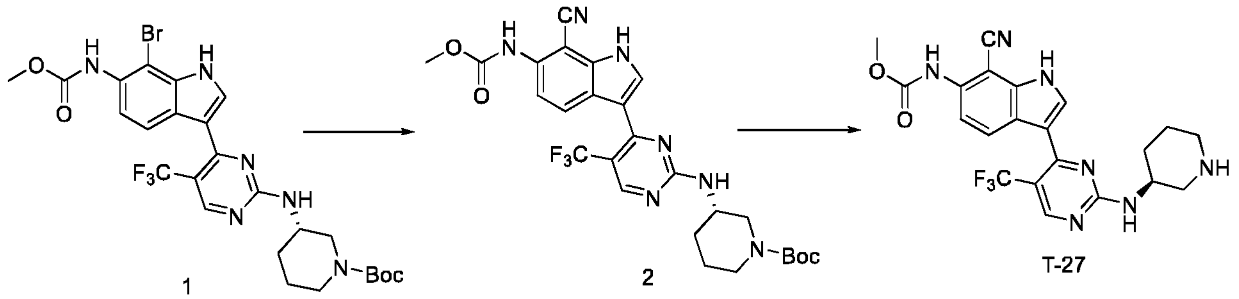

Synthesis step:

(1) Synthesis of Compound 2
0.5g(1.0eq) compound 1 was dissolved in 6mL DMF, 0.22g(3eq) CuCN was added to react overnight at 100 °C. After the reaction was completed, ammonia and water were added, extracted with EA and dried, spin-dried to obtain 0.13g of crude compound 2. LC-MS[M +1]:560.2.
(2) Synthesis of Compound T-27
130mg(1eq) compound 2 was taken and added into 4 mL 4M/L dioxane hydrochloride and 4mL EA to react at room temperature. After the reaction was completed, $NaHCO_3$ aqueous solution was added to adjust the solution to be alkaline, extracted with EA and dried, spin-dried, separated by preparation plate to obtain 40mg of compound T-27. HPLC:95.1%. LC-MS[M +1]:460.1. $^1H$ NMR (400 MHz, DMSO-$d_6$) δ 9.81 (s, 1H), 8.67 - 8.52 (m, 1H), 8.16 (m, 2H), 7.81 (s, 1H), 7.25 (m, 1H), 3.90 (d, *J* = 10.1 Hz, 1H), 3.71 (s, 3H), 3.07 (d, *J* = 12.0 Hz, 1H), 2.81 (s, 1H), 2.44 (s, 2H), 1.97 (d, *J* = 13.4 Hz, 1H), 1.66 (s, 1H), 1.46 (d, *J* = 10.1 Hz, 2H).

**Example T-28**

The compound synthesized by the invention:

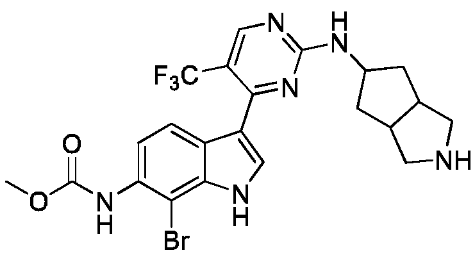

T-28

The experimental process was as follows:
The synthetic route is as follows:

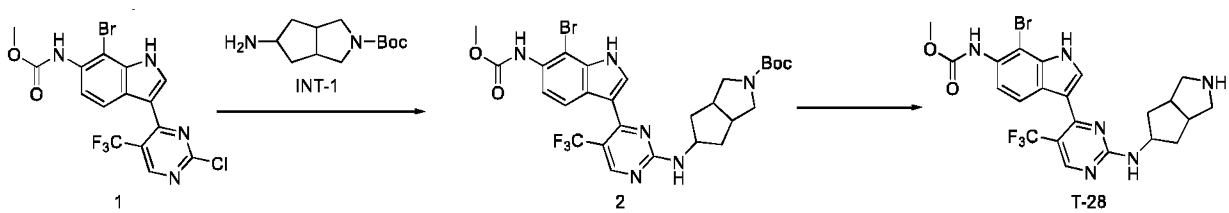


Synthesis step:

(1) Synthesis of Compound 2
200 mg compound **1,** INT-1 (2.0 e.q.) and 15 mL DMF as solvent were added into a 100 mL three-necked flask to react at room temperature under nitrogen. After the reaction was completed, distilled to remove DMF solvent, separated and purified by silica gel column chromaography to obtain 220 mg of compound **2** with a yield of 77.5%, LC-MS [M +1]:639.1, 641.1.

(2) Synthesis of T-28 Hydrochloride and T-28

220 mg of compound **2** was added into a 50 mL three-necked flask, dissolved to be clear with 10 mL of ethyl acetate, then 3 mL 1 M of hydrochloric acid dioxane solution was added to react under stirring for 1 hour at room temperature under nitrogen. After the reaction was completed, suction filtered, solid was washed with an appropriate amount of ethyl acetate and ether in turn to obtain100 mg of T-28 hydrochloride as a yellow solid with a yield of 50.5%, LC-MS[M +1]:539.1, 541.1, HPLC purity:99.0%

The compound T-28 hydrochloride (50mg) was added with $NaHCO_3$ aqueous solution to be alkaline (pH = 8.0), extracted with EA and dried, spin-dried to obtain compound T-28. HPLC purity: 98.8%. LC-MS[M +1]:539.1, 541.1.

The following compounds were synthesized with reference to the method in Example T-28:

| Example | Compound structure | Characterization data |
|---|---|---|
| T-19 | 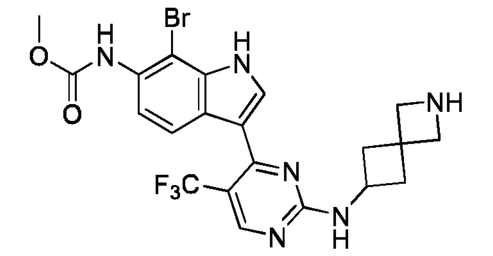 T-19 | LC-MS[M +1]:525.1,527.1 |
| T-31 | 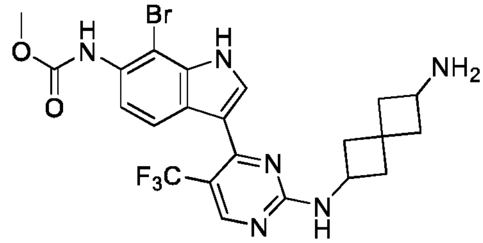 T-31 | LC-MS[M +1]:539.1,541.1 |

## Example T-32 and T-33

The compound synthesized by the invention:

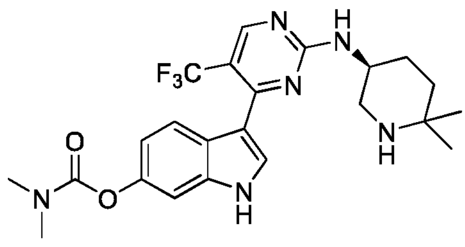

T-32

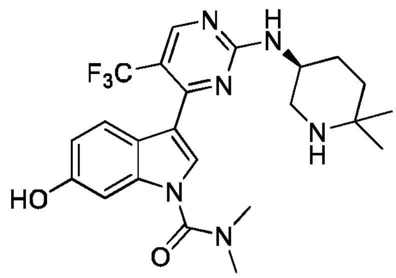

T-33

The experimental process was as follows:
The synthetic route is as follows:

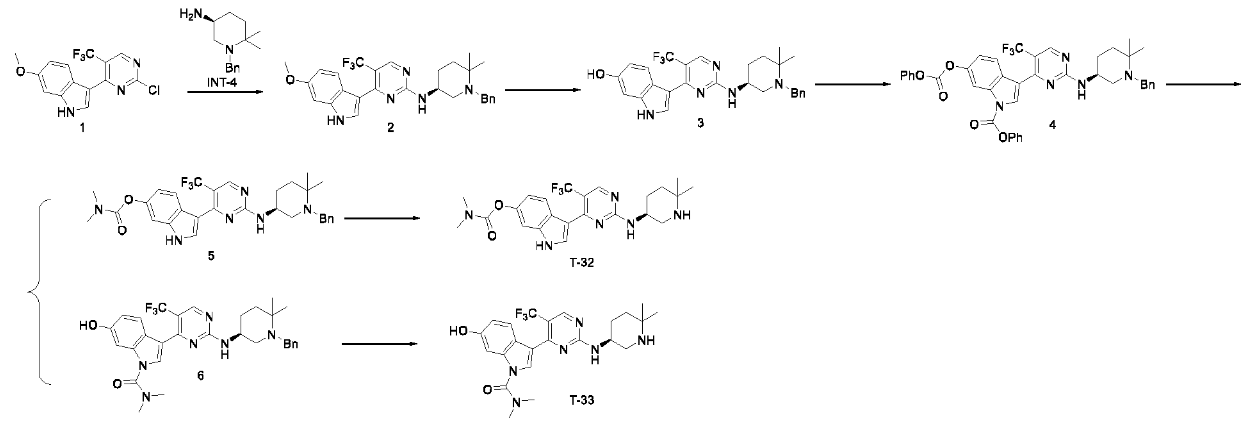

1 2 3 4 5 T-32 6 T-33

Synthesis step:

(1) Synthesis of Compound 2
0.2g (300mg, 1eq) compound 1 was dissolved in 5ml DMF, then INT-4(146mg,1.1eq) and DIPEA(118.3mg,1.5eq) were added to react overnight at rt under nitrogen protection. After the reaction was completed, water was added to the system, extracted with EA and dried, spin-dried, and purified by column to obtain compound 2 (470mg).
(2) Synthesis of Compound 3
Compound 2 (200mg,1.0eq) was dissolved in DCM(5ml), cooled to -45 °C, then a solution of boron tribromide (151.4mg,1.5eq) in DCM(1.0ml) was added dropwise under nitrogen protection, after the dropping, the reaction solution was heated to -30 °C to react for 10min, then the temperature was naturally heated to room temperature to react for 2h. After the reaction was completed, sodium bicarbonate aqueous solution was added to quench, extracted with DCM, compound 3(130mg) was obtained by drying and liquid separation.
(3) Synthesis of Compound 4
Compound 3 (130mg,1.0eq) was added to DCM(5ml), being insoluble, then phenyl chloroformate (42mg,1.0eq) was added, dissolved, then TEA(27mg,1.0eq) was added to react at room temperature for 2h. After the reaction was completed, water was added to the system, then DCM was added for extraction, the liquid was separated, dried, filtered and compound 4 (112mg) was obtained from a preparation plate.
(4) Synthesis of Compound 5 and Compound 6:
Compound 4(112mg,1.0eq) was dissolved in THF(5ml), then dimethylamine aqueous solution (6ml) was added to react overnight, water was added to the system after the reaction was completed, then EA was added for extraction, the liquid was separated, dried, filtered and compound 5(23mg) and compound 6(31mg) were obtained from a preparative plate.
(5) Synthesis of Compound T-32 and T-33
Compound 5(23mg) and compound 6(31mg) were dissolved in methanol respectively, then Pd/C was added respectively, $H_2$ was introduced and reacted overnight. After the reaction was completed, the reaction solution was filtered, and spin-dried to obtain the final product compound T-32(12mg), HPLC purity: 95.6%, LC-MS[M +1]:477.2. Compound T-33(8mg), HPLC purity 94.2%. LC-MS[M +1]:477.2.

### Example T-34

The compound synthesized by the invention:

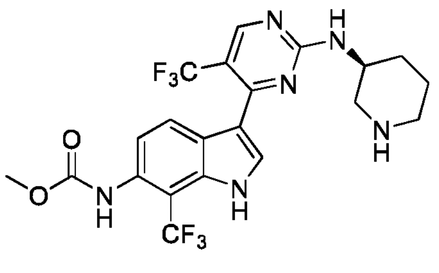

T-34

The experimental process was as follows:
The synthetic route is as follows:

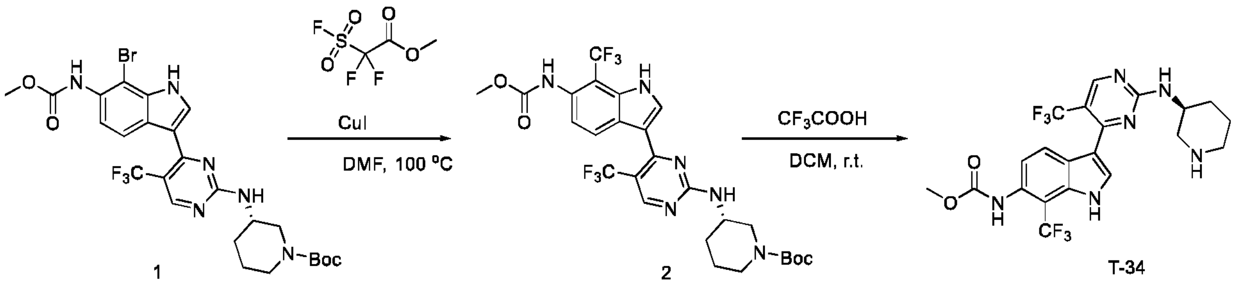

1 2 T-34

Synthesis step:

(1) Synthesis of Compound 2
Under anhydrous and oxygen-free conditions, 300 mg of compound 1, methyl fluorosulfonyl difluoroacetate (5.0 e.q.) and CuI (5.0 e.q.) were added into 10 mL of reaction branch pipe, the temperature was raised to 100 °C and reacted at this temperature. After the reaction was completed, cooled to room temperature, several milliliters of 1M dilute hydrochloric acid was added, extracted with water and EA, the liquid was separated, and the organic phase was dried over anhydrous sodium sulfate, concentrated, separated and purified by silica gel column chromatography to obtain 110 mg of compound **2**with a yield of 37.3%, LC-MS [M +1]:603.2.
(2) Synthesis of Compound T-34
110 mg of compound **2** was added to a 50 mL three-necked flask, 6 mL of dichloromethane was added, after being dissolved to be clear, and then 2 mL of trifluoroacetic acid was added to react under stirring at room temperature for half an hour. After the reaction was completed, concentrated to remove excess solvent, then the pH value was adjusted to be alkaline, extracted with EA, spin-dried, separated and purified by reverse column under medium pressure to obtain 60 mg of compound T-34 with a yield of 65.2%, HPLC purity: 95.0%, and LC-MS[M +1]:503.1.

The following compounds were synthesized with reference to the method in Example T-34:

| Example | Compound structure | Characterization data |
|---|---|---|
| T-35 | 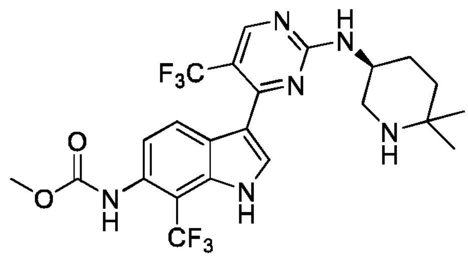 T-35 | LC-MS[M+1]:531.2 |
| T-87 | 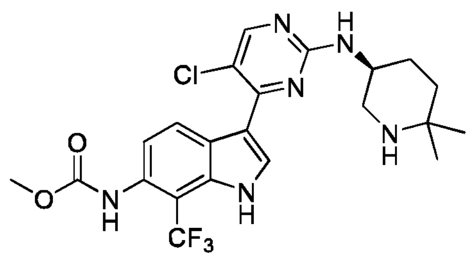 T-87 | LC-MS[M+1]:497.1 |

(continued)

| Example | Compound structure | Characterization data |
|---|---|---|
| T-88 | 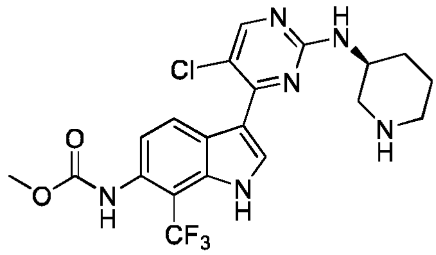<br>T-88 | LC-MS[M+1]:469.1 |
| T-100 | 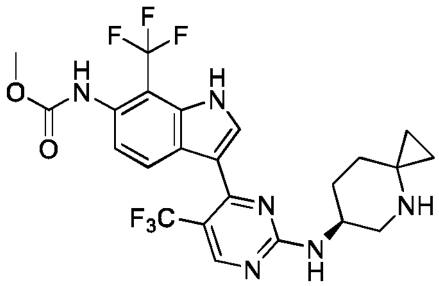<br>T-100 | LC-MS[M+1]:529.1 |
| T-101 | 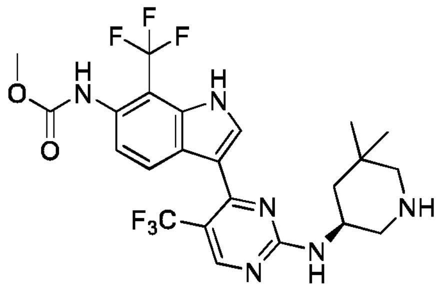<br>T-101 | LC-MS[M+1]:531.2 |
| T-107 | 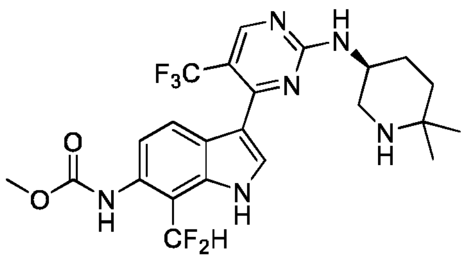<br>T-107 | LC-MS[M+1]:513.1 |
| T-111 | 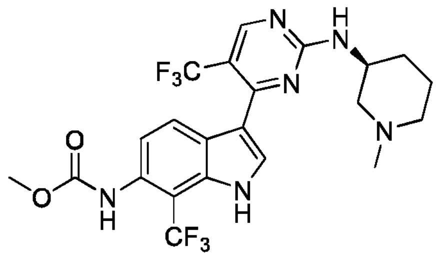<br>T-111 | LC-MS[M+1]:517.1 |

(continued)

| Example | Compound structure | Characterization data |
|---|---|---|
| T-113 | 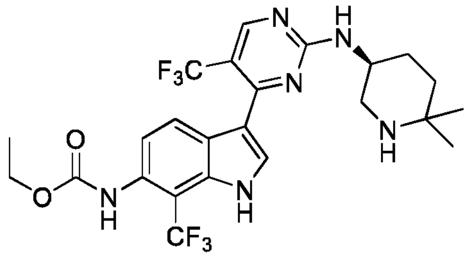 T-113 | LC-MS[M+1]:545.2 |
| T-149 | 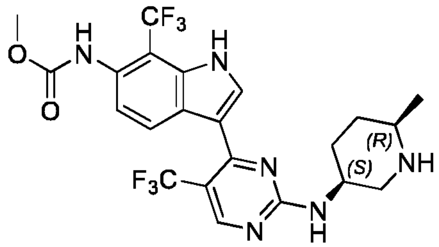 T-149 | LC-MS[M+1]:517.2 |
| T-169 | 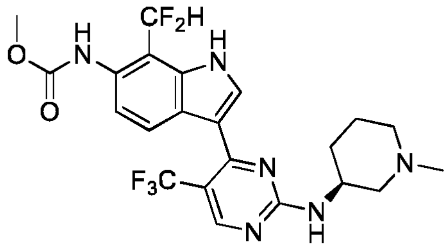 T-169 | LC-MS[M+1]:499.1 |
| T-172 | 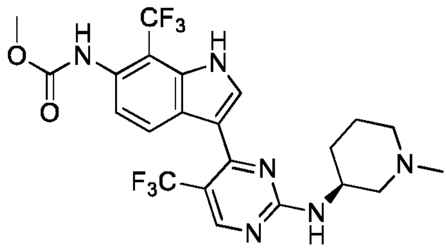 T-172 | LC-MS[M+1]:517.2 |

**Example T-37**

The compound synthesized by the invention:

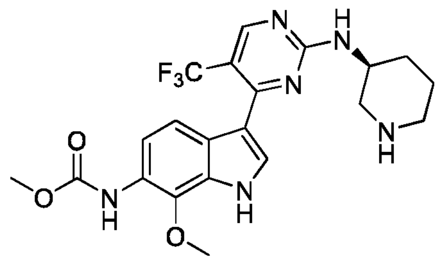

T-37

The experimental process was as follows:
The synthetic route is as follows:

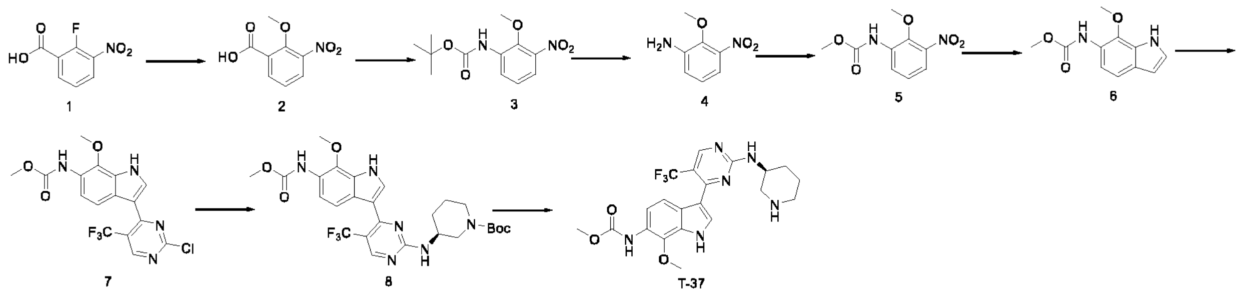

Synthesis step:

(1) Synthesis of Compound 2
At 0 °C, compound 1(10.0g,1.0eq) was dissolved in methanol (100ml), then sodium methoxide (6.9g, 2.0eq) was added, after the addtion, the temperature was raised to 65 °C to react for 3 hours. TLC monitored until the reaction was completed. After the temperature was cooled down, the solvent was spin-dried, a small amount of methanol was added to be slurried, filtered, and the filter cake was collected to obtain compound 2(10.06g).
(2) Synthesis of Compound 3
Compound 2(3.0g,1.0eq) was dissolved in tert-butanol (15ml), then diphenylphosphoryl azide (DPPA)(5.86g,1.4eq) and triethylamine (2.77g,1.8eq) were added, and the temperature was raised to 80 °C under nitrogen protection to react for 2 hours. TLC monitored until the reaction was completed, water was added to the system after cooling, EA was added for extraction, EA phase was collected, dried over anhydrous sodium sulfate, filtered, spin-dried, and purified by column to obtain compound 3(2.2g).
(3) Synthesis of Compound 4
HCl/dioxane (10ml) was added to compound 3(2.2g), stirred at room temperature for 1.5h, TLC monitored until the reaction of the raw materials was completed, the solvent was spin-dried, petroleum ether was added to be slurried, filtered to obtain compound 4(1.2g).
(4) Synthesis of Compound 5
Methyl chloroformate (10ml) was added to compound 4(1.2g, 1.0eq), cooled to 0 °C, sodium carbonate (3.0g,4.0eq) was added in batches, after the addtion, the temperature was naturally raised to room temperature to react overnight. TLC monitored until the reaction was completed, water was added into the system, then EA was added for extraction, EA phase was collected, dried over anhydrous sodium sulfate, filtered and spin-dried, and purified by column to obtain compound 5(1.3g).
(5) Synthesis of Compound 6
Compound 5(1.0g,1.0eq) was dissolved in dry THF(10ml), the nitrogen was charged to replace by evacuation, then cooled to -78 °C, 1mol/L vinyl magnesium bromide (26.5ml,6.0eq) was added dropwise. After the dropping, the temperature was naturally raised to room temperature, and the reaction solution was stirred overnight. TLC monitored until the reaction of the raw materials was completed, cooled, ammonium chloride aqueous solution (20ml) was added to the system, stirred for 0.5h, then EA was added for extraction, liquid was separated, EA phase was collected, dried over anhydrous sodium sulfate, filtered, spin-dried, and purified by column to obtain compound 6(0.3g).
(6) Synthesis of Compound 7
0.58g(3.0eq) 2,4-dichloro-5-trifluoromethylpyrimidine was dissolved in 6mL dichloroethane (DCE), 0.18g(1.5eq)

aluminum trichloride ($AlCl_3$ was added to react at 80 °C for 30min under nitrogen protection. Then, compound 6(0.2g, 1.0eq) was added to react overnight at 80 °C. After the reaction was completed, water was added, DCE phase was separated, the aqueous phase was extracted with EA, DCE phase and EA phase were combined, dried, spin-dried, slurried in EA, filtered to obtain compound 7(60mg).

(7) Synthesis of Compound 8

60mg(1.0eq) compound 7 was dissolved in 1mL DMF, then (S)-1-Boc-3-aminopiperidine(60mg,2eq) was added to react overnight at room temperature under N2 protection. After the reaction was completed, water was added to the system, extracted with EA and dried, spin-dried, and purified by column to obtain compound 8(70mg).

(8) Synthesis of compound T-37 hydrochloride:

HCl/dioxane (5ml) was added to compound 8(70mg), stirred at room temperature for 1.5h, TLC monitored until the reaction of the raw materials was completed, the solvent was spin-dried, petroleum ether was added to be slurried, filtered to obtain T-37(43mg, purity: 97.2%). $^1$H NMR (400 MHz, DMSO-$d_6$) δ 12.12 - 11.93 (m, 1H), 9.17 - 8.78 (m, 3H), 8.03 (d, *J* = 10.1 Hz, 1H), 7.74 (s, 1H), 7.33 (d, *J* = 12.8 Hz, 1H), 4.27 (s, 1H), 3.87 (s, 3H), 3.65 (s, 3H), 3.19 (s, 1H), 2.95 - 2.77 (m, 2H), 1.95 (d, *J* = 32.2 Hz, 2H), 1.72 (d, *J* = 12.5 Hz, 2H), 1.31 - 1.13 (m, 2H).

The following compounds were synthesized with reference to the method in Example T-37:

| Example | Compound structure | Characterization data |
|---|---|---|
| T-38 | 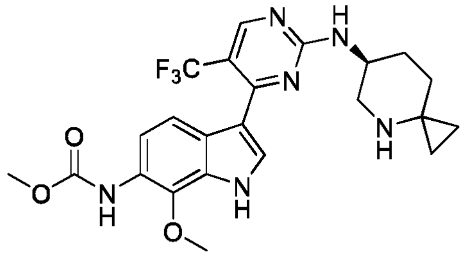 **T-38** | LC-MS[M+1]:491.2 |
| T-85 | 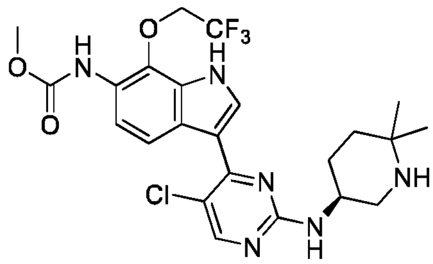 **T-85** | LC-MS[M+1]:527.1,529.1 |
| T-91 | 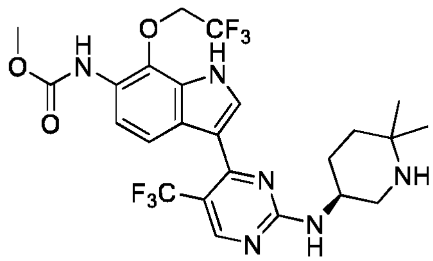 **T-91** | LC-MS[M+1]:561.1 |

(continued)

| Example | Compound structure | Characterization data |
|---|---|---|
| T-92 | 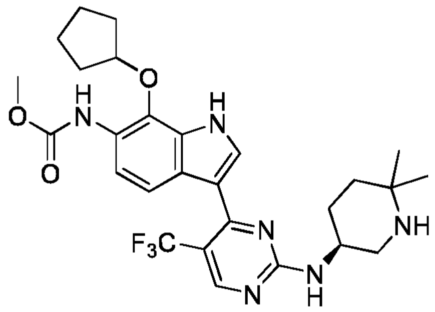 T-92 | LC-MS[M+1]:547.2 |
| T-93 | 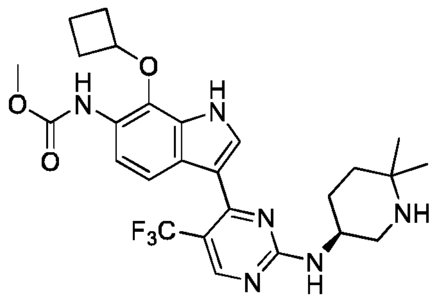 T-93 | LC-MS[M+1]:533.2 |
| T-94 | 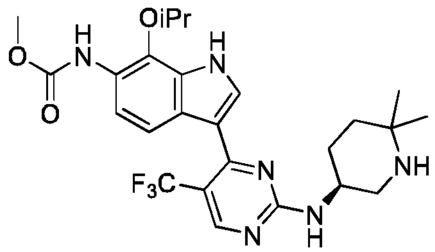 T-94 | LC-MS[M+1]:521.2 |
| T-95 | 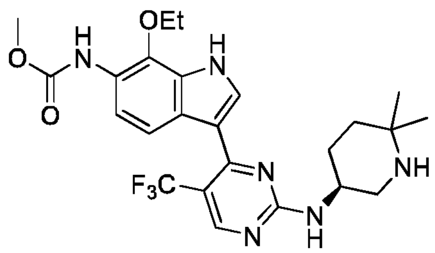 T-95 | LC-MS[M+1]:507.2 |

(continued)

| Example | Compound structure | Characterization data |
|---|---|---|
| T-132 | 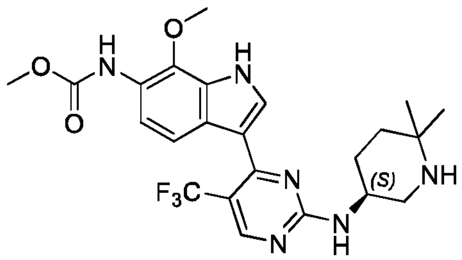 T-132 | LC-MS[M+1]:493.2 |
| T-133 | 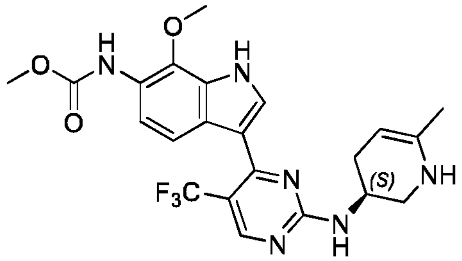 T-133 | LC-MS[M+1]:477.1 |
| T-138 | 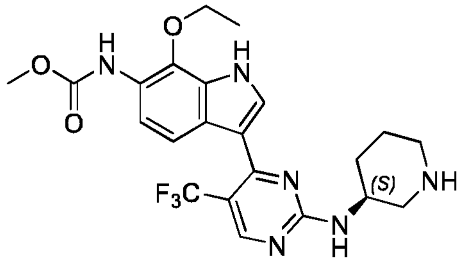 T-138 | LC-MS[M+1]:479.1 |
| T-139 | 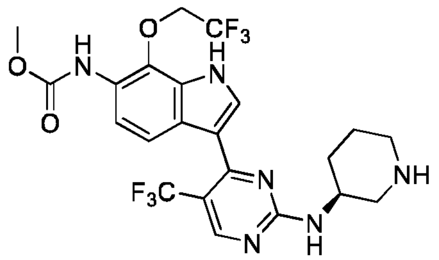 T-139 | LC-MS[M+1]:533.1 |
| T-140 | 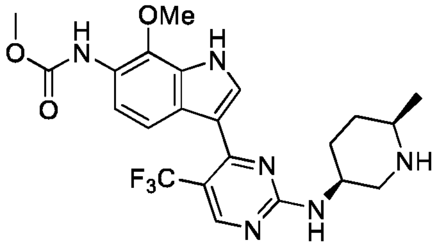 T-140 | LC-MS[M+1]:479.2 |

(continued)

| Example | Compound structure | Characterization data |
|---|---|---|
| T-141 | 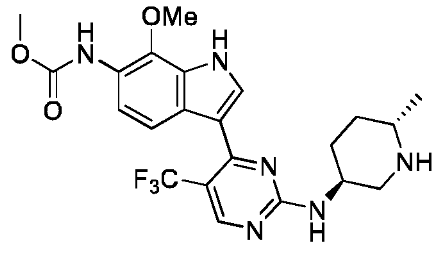 T-141 | LC-MS[M+1]:479.2 |
| T-150 | 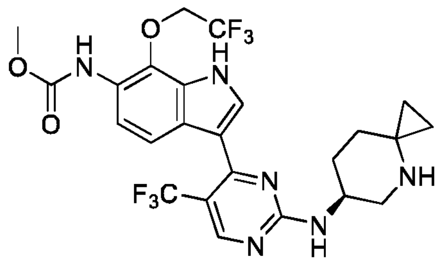 T-150 | LC-MS[M+1]:559.1 |
| T-151 | 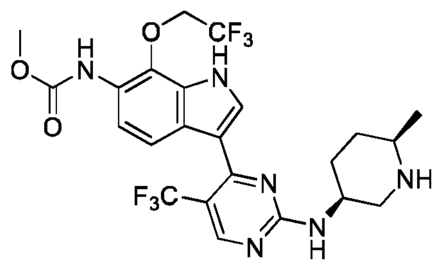 T-151 | LC-MS[M+1]:547.1 |
| T-152 | 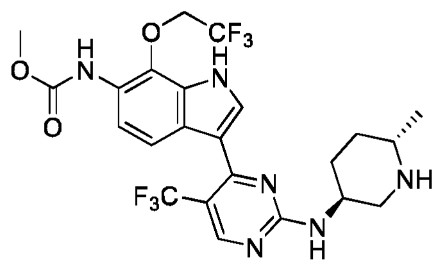 T-152 | LC-MS[M+1]:547.1 |
| T-160 | 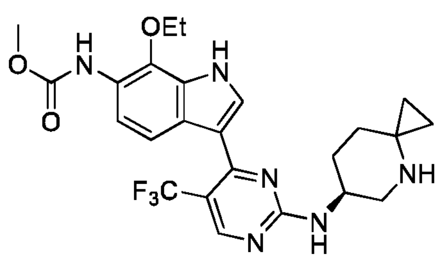 T-160 | LC-MS[M+1]:505.2 |

(continued)

| Example | Compound structure | Characterization data |
|---|---|---|
| T-161 | 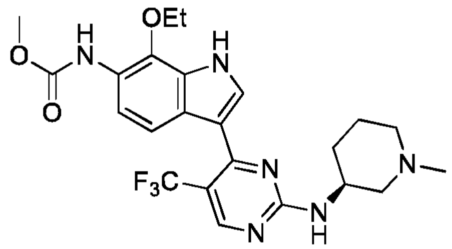 T-161 | LC-MS[M+1]:493.2 |
| T-167 | 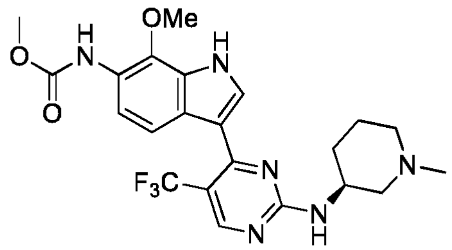 T-167 | LC-MS[M+1]:479.2 |

**Example T-40**

The compound synthesized by the invention:

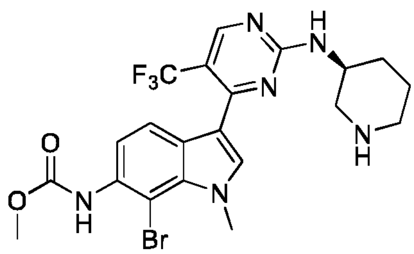

T-40

The experimental process was as follows:
The synthetic route is as follows:

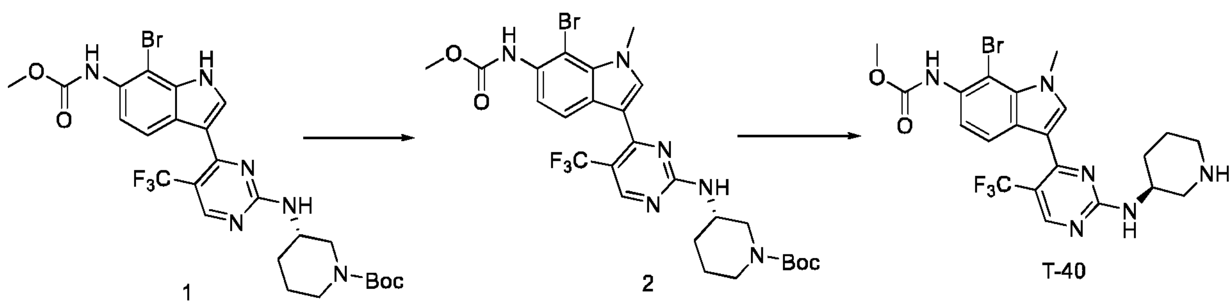

1 2 T-40

Synthesis step:

(1) Synthesis of Compound 2

350mg(1eq) compound 1 was dissolved in 4mL DMF, 200mg(2.5eq) of $K_2CO_3$ and 410mg(5eq) of methyl iodide ($CH_3I$) were added to react at room temperature. After the reaction was completed, 40mL of water was added, extracted with EA and dried, spin-dried to obtain 250mg of crude compound 2.

(2) Synthesis of Compound T-40

250mg (1eq) compound 5 (crude) obtained in above step was taken and added into 4mL 4M/L dioxane hydrochloride to react at room temperature. After the reaction was completed, $NaHCO_3$ aqueous solution was added to adjust the solution to be alkaline, extracted with EA and dried, spin-dried, separated by preparation chromatography to obtain 40mg of compound T-40. HPLC:96.2%. LC-MS[M + 1]:527.0, 529.0. $^1$H NMR (400 MHz, DMSO-$d_6$) δ 9.10 (d, $J$ = 12.8 Hz, 1H), 8.59 (d, $J$ = 19.2 Hz, 1H), 8.18 (m, 1H), 7.89 - 7.72 (m, 2H), 7.18 (d, $J$ = 8.4 Hz, 1H), 4.22 (d, $J$ = 1.6 Hz, 3H), 3.86 (m, 1H), 3.65 (s, 3H), 3.03 (m, 1H), 2.88 - 2.68 (m, 1H), 2.45 - 2.33 (m, 2H), 1.93 (s, 1H), 1.70 - 1.57 (m, 1H), 1.44 (m, 2H).

**Example T-41**

The compound synthesized by the invention:

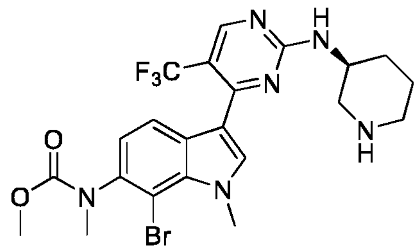

T-41

The experimental process was as follows:
The synthetic route is as follows:

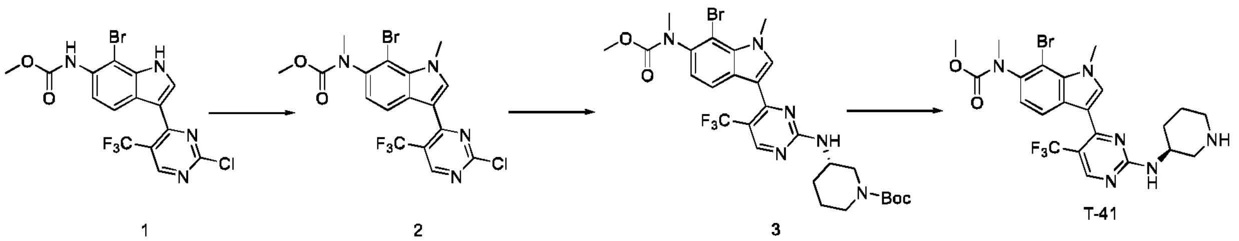

Synthesis step:

(1) Synthesis of Compound 2

500mg(1eq) compound 1 was dissolved in 6mL THF, 267mg(6eq) of NaH was added in salt ice bath, stirred for 5min, then 1.39g(5eq)$CH_3I$ was added to react at room temperature. After the reaction was completed, 40mL of water was added, extracted with EA and dried, spin-dried to obtain 500mg of crude compound 2. LC-MS[M +1] :477.0, 479.0.

(2) Synthesis of Compound 3

0.5g(1.0eq) compound 2 was dissolved in 6mL DMF, 0.46g(2.2eq) of (S)-1-Boc-3-aminopiperidine was added, stirred at room temperature to react overnight. After the reaction was completed, water was added, extracted with EA and dried, spin-dried, and purified by column to obtain 0.21g of compound 3. LC-MS[M +1]:641.1, 643. 1.

(3) Synthesis of Compound T-41

150mg (1eq) compound 5 was taken and added into 3mL 4M/L dioxane hydrochlorideto react at room temperature. After the reaction was completed, $NaHCO_3$ aqueous solution was added to adjust the solution to be alkaline, extracted with EA and dried, spin-dried, 25mg of compound T-41 was obtained by preparation separation. HPLC:94.1%. LC-MS[M +1]:541.1, 543.1. $^1$H NMR (400 MHz, DMSO-$d_6$) δ 8.65 - 8.52 (m, 1H), 8.19 (m, 1H), 7.85 (t, $J$ = 4.8 Hz, 2H), 7.18 (q, $J$ = 8.0 Hz, 1H), 4.23 (s, 3H), 3.89 (s, 1H), 3.70 (s, 1H), 3.52 (d, $J$ = 4.2 Hz, 3H), 3.16 (d, $J$ = 11.5 Hz, 3H), 3.04 (d, $J$ = 11.6 Hz, 1H), 2.97 - 2.72 (m, 2H), 2.44 (s, 1H), 1.95 (m, 1H), 1.84 - 1.61 (m, 1H), 1.61 - 1.42 (m, 2H).

**Example T-42**

The compound synthesized by the invention:

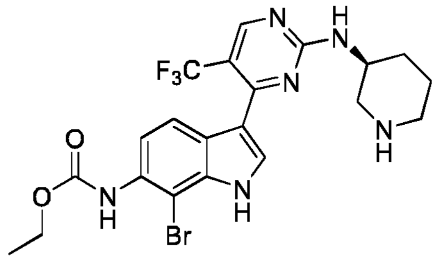

T-42

The experimental process was as follows:
The synthetic route is as follows:

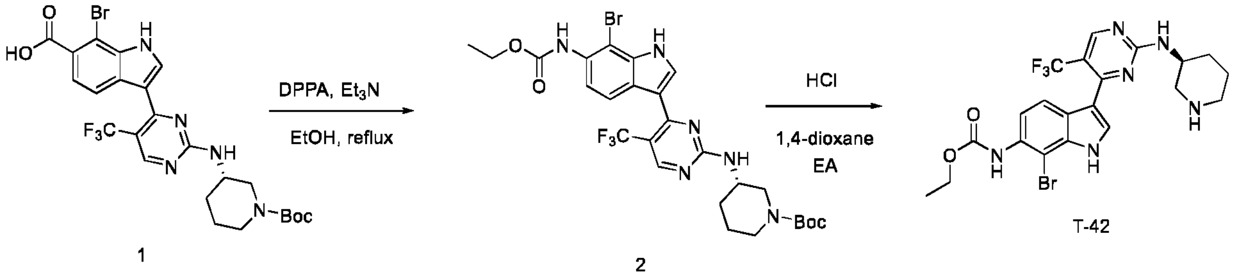


Synthesis step:

(1) Synthesis of Compound 2
Under nitrogen condition, 250 mg of compound **1**and 5 mL of absolute ethanol were sequentially added to 10 mL reaction branch pipe. After being dissolved to be clear, DPPA(10.0 e.q.) and $Et_3N$(5.0 e.q.) were added respectively. The temperature was raised until reflux, and the reaction was carried out for two hours. After the reaction was completed, cooled to room temperature, quenched with water, extracted and separated by EA and water, and the organic phase was dried over anhydrous sodium sulfate, concentrated, separated and purified by silica gel column chromatography to obtain 116 mg of compound **2 with** a yield of 43.2%, HPLC purity : 95.1%.
(2) Synthesis of Compound T-42 Hydrochloride and T-42
156 mg of compound **2** was added into a 50 mLthree-necked flask, dissolved to be clear with 5 mL of ethyl acetate, then 2 mL 1 M of hydrochloric acid dioxane solution was added, reacted under stirring for 1 hour at room temperature under nitrogen. After the reaction was completed, suction filtered, solid was washed with an appropriate amount of ethyl acetate and ether in turn to obtain116 mg of T-42 hydrochloride as a yellow solid with a yield of 82.8%, LC-MS[M +1]:527.1, 529.1, HPLC purity: 95.3%.

The compound T-42 hydrochloride (40mg) was added with $NaHCO_3$ aqueous solution to be alkaline (pH = 8.0), extracted with EA and dried, spin-dried to obtain compound T-42. HPLC purity: 95.3%. LC-MS[M +1]:527.1, 529.1.
The following compounds were synthesized with reference to the method in Example T-42:

| Example | Compound structure | Characterization data |
|---|---|---|
| T-43 | 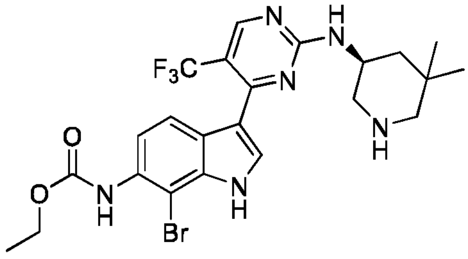 T-43 | LC-MS[M+1]:555.1 |
| T-44 | 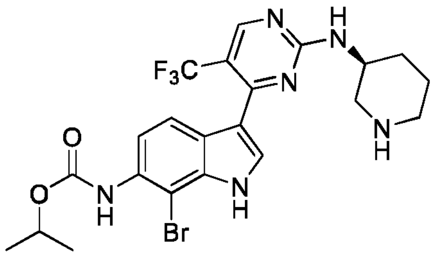 T-44 | LC-MS[M+1]:541.1 |
| T-45 | 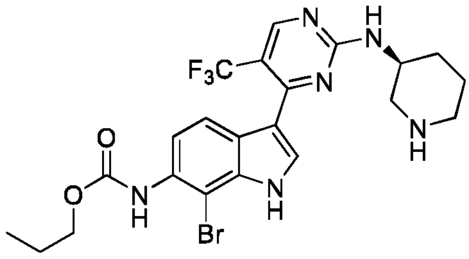 T-45 | LC-MS[M+1]:541.1 |

**Example T-52**

The compound synthesized by the invention:

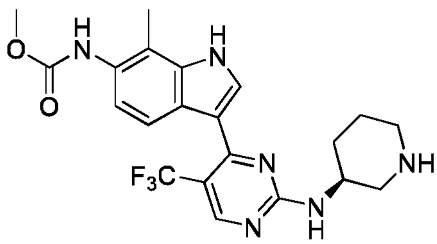

T-52

The experimental process was as follows:
The synthetic route is as follows:

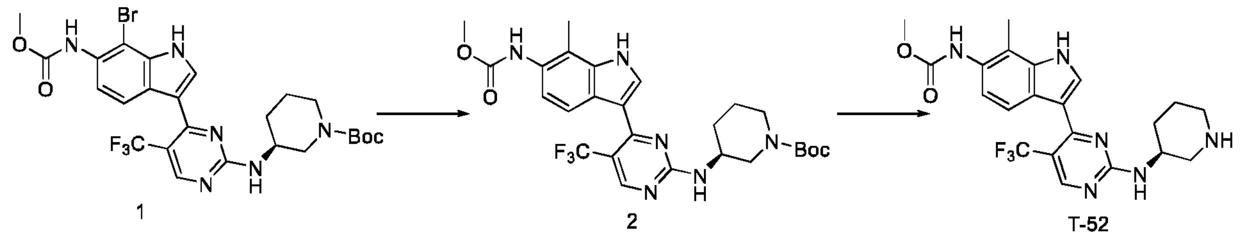

Synthesis step:

(1) Synthesis of Compound 2

274mg(1.0eq) compound 1 was added into a mixed solvent of dioxane (4ml) and water (0.4ml), then methylboric acid 150mg(5.0eq), 207mg of Pd(dppf)$Cl_2$(0.2eq) and 207mg of potassium carbonate (3.0eq) were added to react at 100 °C under $N_2$ protection until the reaction was completed. Water was added, extracted with EA and dried, spin-dried, and purified by column to obtain compound 2(121mg).

(2) Synthesis of compound T-52:

100mg of compounds 3, 1ml of TFA and 4ml of DCM were added to the reaction system. At room temperature, TLC monitored until the reaction was completed after 5 hours. A total of 58mg of T-52 was obtained by purification from the prepared liquid phase with a purity of 98.7%. LC-MS[M+1]: 449.1, $^1$H NMR (400 MHz, DMSO-$d_6$) $\delta$ 11.76 (d, $J$ = 16.4 Hz, 1H), 8.95 (s, 1H), 8.54 (d, $J$ = 19.8 Hz, 1H), 8.10 (dd, $J$ = 51.7, 8.6 Hz, 1H), 7.81 - 7.60 (m, 2H), 7.06 (d, $J$ = 8.6 Hz, 1H), 4.03 (q, $J$ = 7.1 Hz, 1H), 3.90 (d, $J$ = 21.7 Hz, 1H), 3.64 (s, 3H), 3.06 (dd, $J$ = 12.0, 4.0 Hz, 1H), 2.81 (d, $J$ = 12.2 Hz, 1H), 2.48 - 2.38 (m, 2H), 2.35 (d, $J$ = 2.2 Hz, 3H), 1.99 (s, 2H), 1.69 - 1.56 (m, 1H), 1.52 - 1.39 (m, 2H), 1.23 (s, 1H), 1.17 (t, $J$ = 7.1 Hz, 1H).

**Example T-57**

The compound synthesized by the invention:

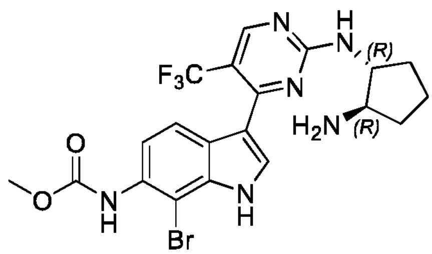

**T-57**

The experimental process was as follows:
The synthetic route is as follows:

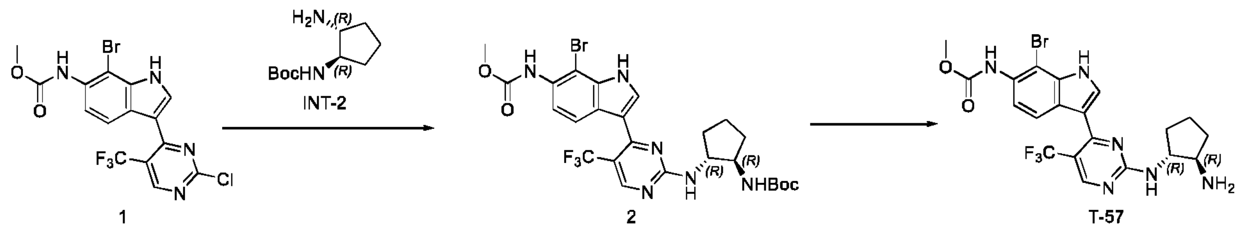


Synthesis step:

(1) Synthesis of Compound 2

379 mg compound 1, INT-2(2.0 eq.) and 15 mL DMF as solvent were added to a 100 mL three-necked flask to react at room temperature under nitrogen. After the reaction was completed, DMF solvent was removed by spin evaporation, separated and purified by silica gel column chromatography to obtain 386 mg of compound **2** with a yield of

74.7%, LC-MS [M +1]:613.1, 615.1.

(2) Synthesis of T-57 Hydrochloride and T-57

253 mg compound **2** was added into a 50 mLthree-necked flask, dissolved to be clear with 10 mL ethyl acetate, then 3 mL 4M hydrochloric acid dioxane solution was added to react under stirring for 1 hour at room temperature under nitrogen. After the reaction was completed, suction filtered, solid was washed with an appropriate amount of ethyl acetate and ether in turn to obtain 190 mg of T-57 hydrochloride as a yellow solid with a yield of 83.7%, LC-MS[M +1]:513.1, 515.1, HPLC purity: 95.7%.

The compound T-57 hydrochloride (60mg) was added with $NaHCO_3$ aqueous solution to be alkaline (pH = 8.0), extracted with EA and dried, spin-dried to obtain compound T-57. HPLC purity: 95.7%. LC-MS[M +1]:513.1, 515.1.

The following compounds were synthesized with reference to the method in Example T-57:

| Example | Compound structure | Characterization data |
|---|---|---|
| T-58 | 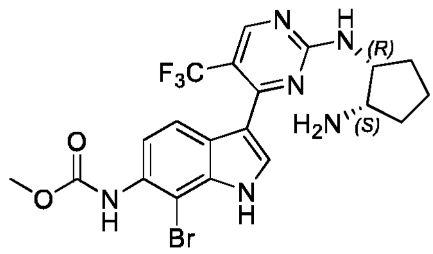 T-58 | LC-MS[M +1]:513.1,515.1 |
| T-59 | 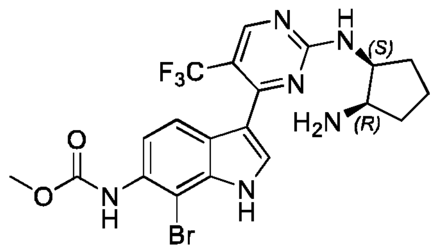 T-59 | LC-MS[M +1]:513.1,515.1 |
| T-67 | 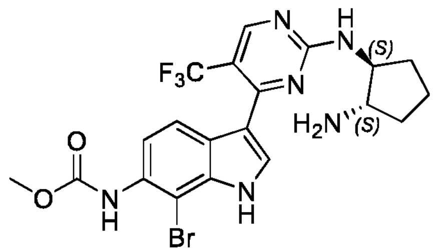 T-67 | LC-MS[M+1]:513.1,515.1 |
| T-115 | 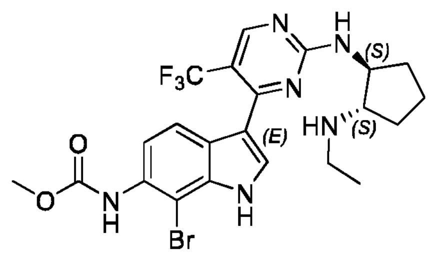 T-115 | LC-MS[M+1]:541.1,543.1 |

(continued)

| Example | Compound structure | Characterization data |
|---|---|---|
| T-116 | 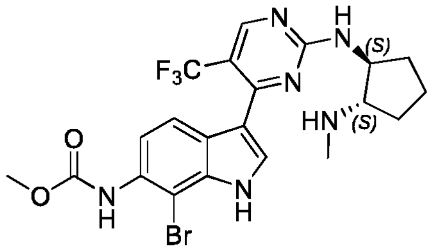<br>T-116 | LC-MS[M+1]:527.0,529.0 |
| T-117 | 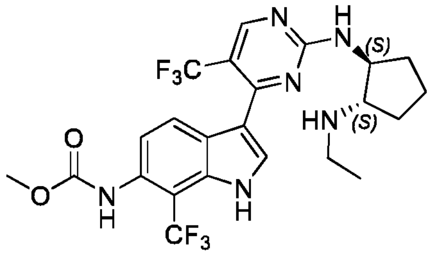<br>T-117 | LC-MS[M+1]:531.1 |
| T-118 | 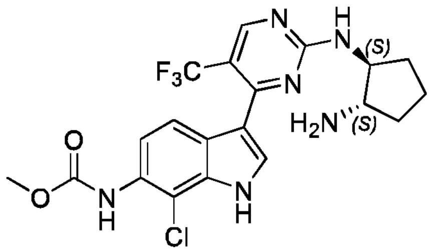<br>T-118 | LC-MS[M +1]:469.1,471.1 |
| T-119 | 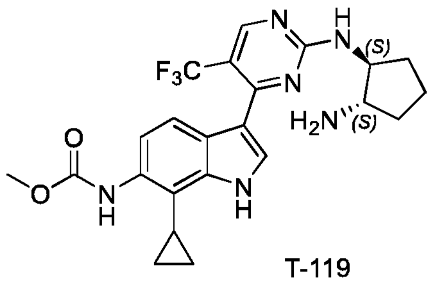<br>T-119 | LC-MS[M+1]:475.1 |
| T-120 | 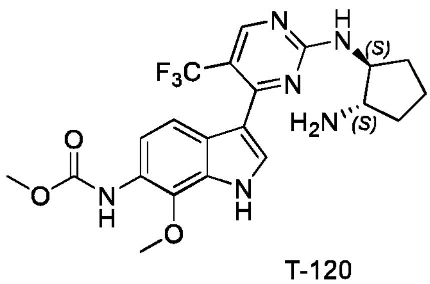<br>T-120 | LC-MS[M+1]:465.1 |

**Example T-60**

The compound synthesized by the invention:

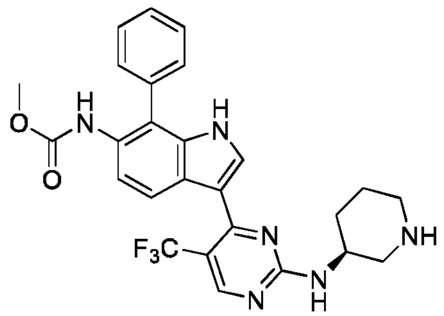

T-60

The experimental process was as follows:
The synthetic route is as follows:

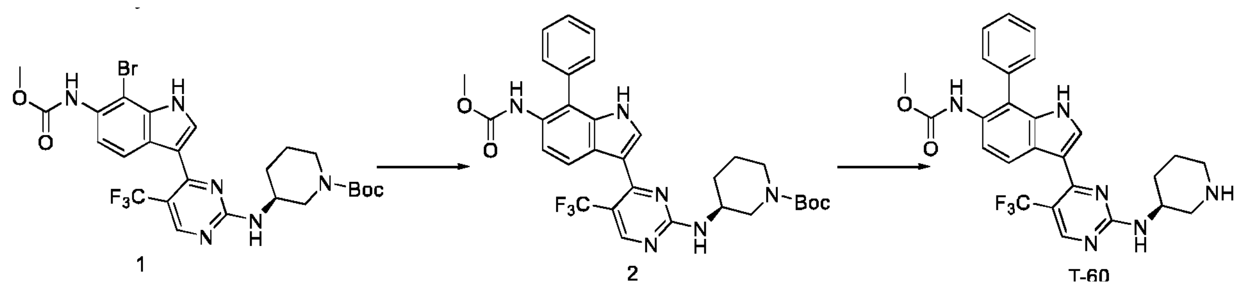

Synthesis step:

(1) Synthesis of Compound 2
0.2g(1eq) compound 1 was added to a mixed solvent of 4ml dioxane and 0.4ml water, then 0.2g(5eq) of phenylboronic acid, 0.05g(0.2eq) of Pd(dppf)$Cl_2$ and 0.14g(3eq) of potassium carbonate were added to react at 100 °C under $N_2$ protection until the reaction was completed. Water was added to quench, extracted with EA and dried, spin-dried, and purified by column to obtain 0.2g of compound 2.
(2) Synthesis of T-60
0.2g(1eq) compound 2 was dissolved into 5mL EA, 2ml dioxane hydrochloride was added, and the reaction was completed at room temperature. The solution was filtered to obtain 0.17g of compound T-60 hydrochloride. HPLC:95.4%. $^1$H NMR (400 MHz, Methanol-$d_4$) δ 8.60 (s, 1H), 8.35 (s, 1H), 8.14 (s, 1H), 7.57 m, 6H), 3.62 (s, 3H), 3.37 (m, 2H), 3.06 (m, 2H), 2.41 - 1.74 (m, 5H).

**Example T-62**

The compound synthesized by the invention:

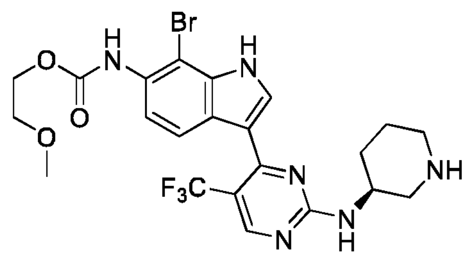

T-62

The experimental process was as follows:
The synthetic route is as follows:

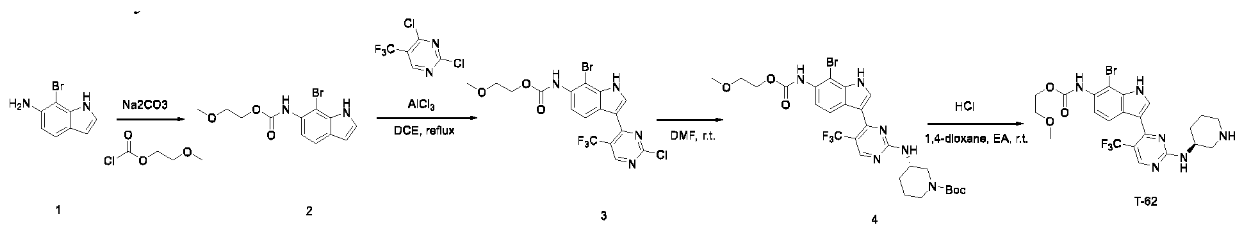


Synthesis step:

(1) Synthesis of Compound 2

Under anhydrous and oxygen-free condition, 5 mL of 2-methoxyethyl chloroformate and sodium carbonate(2.0 e.q.) were added into a 25 mLthree-necked flask in sequence in ice water bath, the temperature was kept to react under stirring for half an hour, 500 mg of compound **1** was added, the temperature was gradually raised to room temperature, and the reaction was continued. After the reaction was completed, water was added to quench, the mixture was extracted and separated by water and EA, and the organic layer was dried over anhydrous sodium sulfate, concentrated, separated and purified by silica gel column chromatography to obtain 597 mg of compound **2** with a yield of 80.5%.

(2) Synthesis of Compound 3

Under nitrogen condition, 2,4-dichloro-5-trifluoromethylpyrimidine (1.5 e.q.) and aluminum trichloride (2.0 e.q.) were added to a three-necked flask containing 150 mL dichloroethane, the temperature was raised to reflux, 2.34g of compound **2** was added after being reacted for half an hour. The temperature was kept to react overnight. After the reaction was completed, ice water was added for quenching, the mixture was extracted and separated by DCM and water, concentrated to remove most DCM, recrystallized with EA, suction filtered to obtain 1.5g of compound **3** as a solid with a yield of 40.8%.

(3) Synthesis of Compound 4

500 mg of compound **3,** (S)-1-Boc-3-aminopiperidine(2.0 e.q.) and 15 mL DMF as solvent were added to a 100 mL three-necked flask to react at room temperature under nitrogen. After the reaction was completed, DMF solvent was removed by spin evaporation, separated and purified by silica gel column chromatography to obtain 430 mg of compound **4** with a yield of 64.6%. LC-MS[M +1]:657.1, 659.1.

(4) Synthesis of Compound T-62 Hydrochloride and T-62

420 mg compound **4** was added into a 50 mL three-necked flask, dissolved to be clear with 10 mL ethyl acetate, then 3 mL 1 M hydrochloric acid dioxane solution was added to react under stirring for 1 hour at room temperature under nitrogen. After the reaction was completed, suction filtered, solid was washed with an appropriate amount of ethyl acetate and ether in turn to obtain 320 mg of compound T-62 hydrochloride as a yellow solid with a yield of 84.4%, LC-MS[M +1]:557.1, 559.1, HPLC purity: 97.9%.

The compound T-62 hydrochloride (100mg) was added with $NaHCO_3$ aqueous solution to be alkaline (pH = 8.0), extracted with EA and dried, spin-dried to obtain compound T-62. HPLC purity: 97.7%. LC-MS[M +1]:557.1, 559.1.

**Examples T-63, T-65 and T-66**

The compound synthesized by the invention:

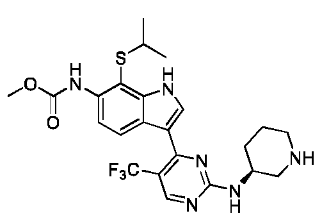

T-66

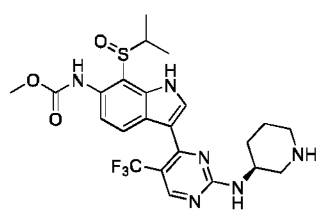

T-65

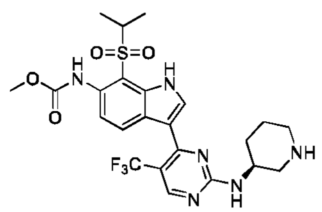

T-63

The experimental process was as follows:
The synthetic route is as follows:

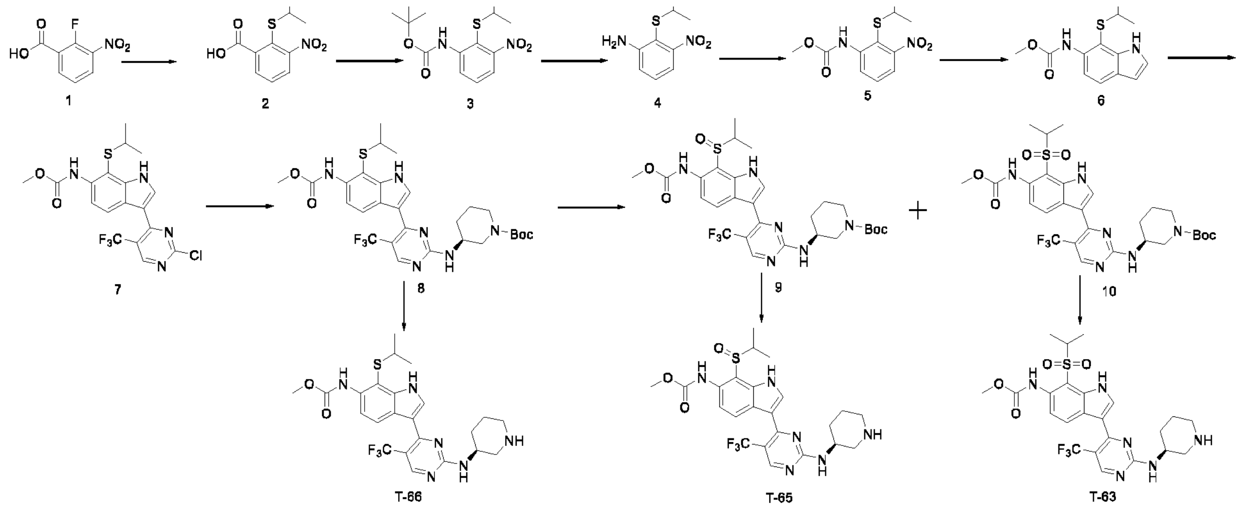

1 2 3 4 5 6 7 8 9 10 T-66 T-65 T-63

Synthesis step:

(1) Synthesis of Compound 2

Compound 1(7.4g,1.0eq) was dissolved into N,N-dimethylformamide (80mL), then sodium 2-propanethiolate (8.0g,2.5eq) was added, and then the temperature was raised to 50°C to react for 2 hours. TLC monitored until the reaction was completed, cooled to room temperature, then 80mL of ice water was added, the pH was adjusted to 5 with 2mol/L hydrochloric acid, then extracted with ethyl acetate, and the organic phases were combined, dried, and spin-dried to obtain 11.5g of crude compound 2.

(2) Synthesis of Compound 3

Under nitrogen condition, compound 2(9.64g,1.0eq) was dissolved into tert-butanol (100mL), and then triethylamine (10mL,1.8eq) and diphenylphosphoryl azide (13.2g,1.2eq) were added. Then, the temperature was slowly heated to 80°C (the heating rate was controlled to prevent spraying due to the release of a large amount of gas) to react for 12 hours. TLC monitored until the reaction was completed. The reaction solution was cooled to room temperature, then 100mL of water was added, extracted with ethyl acetate, the organic phase was dried, spin-dried, and purified by column to obtain 8.5g of compound 3 with a yield of 68%.

(3) Synthesis of Compound 4

Compound 3(8.5g,1.0eq) was added to 120mL of 4mol/L dioxane hydrochloride solution to react overnight at room temperature. TLC monitored until the reaction was completed, filtered, the filter cake was collected, dried to obtain 6.7g of compound 4 with a yield of 100%.

(4) Synthesis of Compound 5

Compound 4(6.7g,1.0eq) was added to methyl chloroformate (90mL), then, sodium carbonate (17.4g,4.0eq) was added in batches at 0°C. After the addition, the temperature was naturally increased to room temperature to react overnight. TLC monitored until the reaction was completed, filtered, the filter cake was washed with 120mL ethyl acetate, the organic phase was collected, and 8g of compound 5 crude product was obtained by direct spin-drying. Yield: 94%.

(5) Synthesis of Compound 6

Under nitrogen, compound 5(4.0g,1.0eq) was added to 80mL tetrahydrofuran, then the temperature was reduced to -78°C, vinyl magnesium bromide (113mL,6.0eq) was slowly added dropwise. After the addtion, the reaction was carried out at this temperature for 1 hour, then the temperature was slowly increased to room temperature to react for 1 hour, TLC monitored until the reaction was completed. The system was cooled to -10 °C, then ammonium chloride aqueous solution was added to quench the reaction, extracted with ethyl acetate, and the organic phases were combined, spin-dried, and purified by column to obtain 1.2g of compound 6 with a yield of 30%.

(6) Synthesis of Compound 7

Under nitrogen condition, 2,4-dichloro-5-trifluoromethylpyrimidine (1.72g,3.0eq) and aluminum trichloride (0.706g,2.0eq) were added into a three-necked flask containing 50 mL dichloroethane, the temperature was raised to reflux, 0.7g compound **6** was added after being reacted for half an hour, and the temperature was kept to react overnight. After the reaction was completed, ice water was added for quenching, the reaction mixture was extracted and separated by DCM and water, concentrated, and purified by column to obtain 0.4g of compound 7with a yield of 34%.

(7) Synthesis of Compound 8

175mg of compound 7, (S)-1-Boc-3-aminopiperidine(158mg,2.0eq), 2mL DMF and 2mL acetonitrile as solvent were added into a 50 mL three-necked flask. The reaction was carried out for 14 hours at room temperature under nitrogen. After the reaction was completed, the acetonitrile solvent was removed by spin evaporation, then the remaining reaction solution was poured into 10mL of water, the filter cake was collected, and then the filter cake was passed through a column to obtain 72mg of compound **8** with a yield of 30%.

(8) Synthesis of Compound 9

Compound 8(100mg,1.0eq) was added to dichloromethane (2mL), then m-chloroperoxybenzoic acid (45.2mg,0.99eq) was added at -10°C to react at this temperature for 2 hours, and then the temperature was heated to room temperature to react overnight. TLC monitored until the reaction was completed. The reaction solution was filtered, the filtrate was washed with 10% sodium hydroxide solution, and then the organic phase was spin-dried, and purified by column to obtain 80mg of compound 9 with a yield of 77%.

(9) Synthesis of Compound 10

Compound 8(100mg, 1.0eq) was added to 2mL of dichloromethane, and then m-chloroperoxybenzoic acid (114mg,2.5eq) was added at room temperature to react overnight. TLC monitored until the reaction was completed. The reaction solution was filtered, the filtrate was washed with 10% sodium hydroxide aqueous solution, and the organic phase was spin-dried, and purified by column to obtain 90mg of compound 10 with a yield of 86%.

(10) Synthesis of Hydrochlorides of Compound T-63, 65 and 66

100mg of compound 8, compound 9 and compound 10 were added into three 25 mL three-necked flasks respectively, and then 3mL of dioxane hydrochloride solution was added respectively to react under stirring for 1hour at room temperature under nitrogen. After the reaction was completed, suction filtered, the solid was sequentially washed with an appropriate amount of ether to obtain50mg of compound T-63 hydrochloride, 69mg of compound T-65 hydrochloride and 48mg of compound T-66 hydrochloride.

The following compounds were synthesized with reference to the method in Example T-63:

| Example | Compound structure | Characterization data |
|---|---|---|
| T-64 | 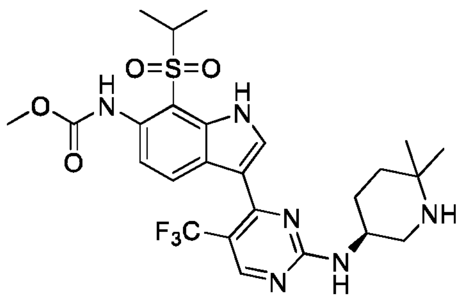 T-64 | LC-MS[M+1]:569.2 |
| T-89 | 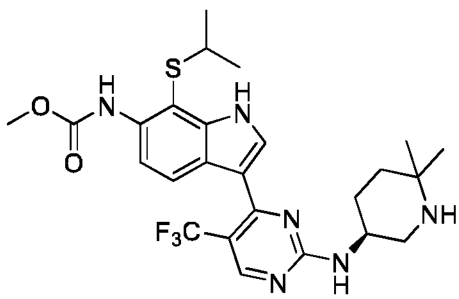 T-89 | LC-MS[M+1]:537.2 |

**Example T-69 and T-70**

The compound synthesized by the invention:

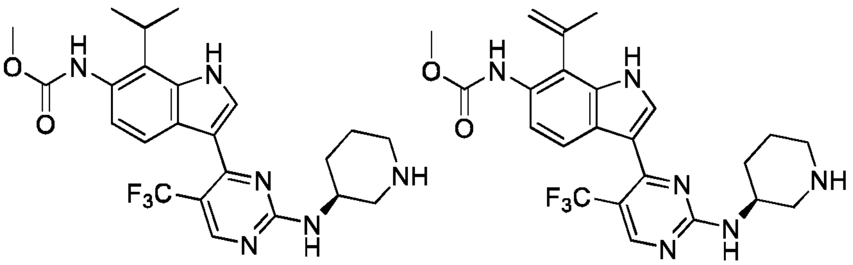

T-69 T-70

The experimental process was as follows:
The synthetic route is as follows:

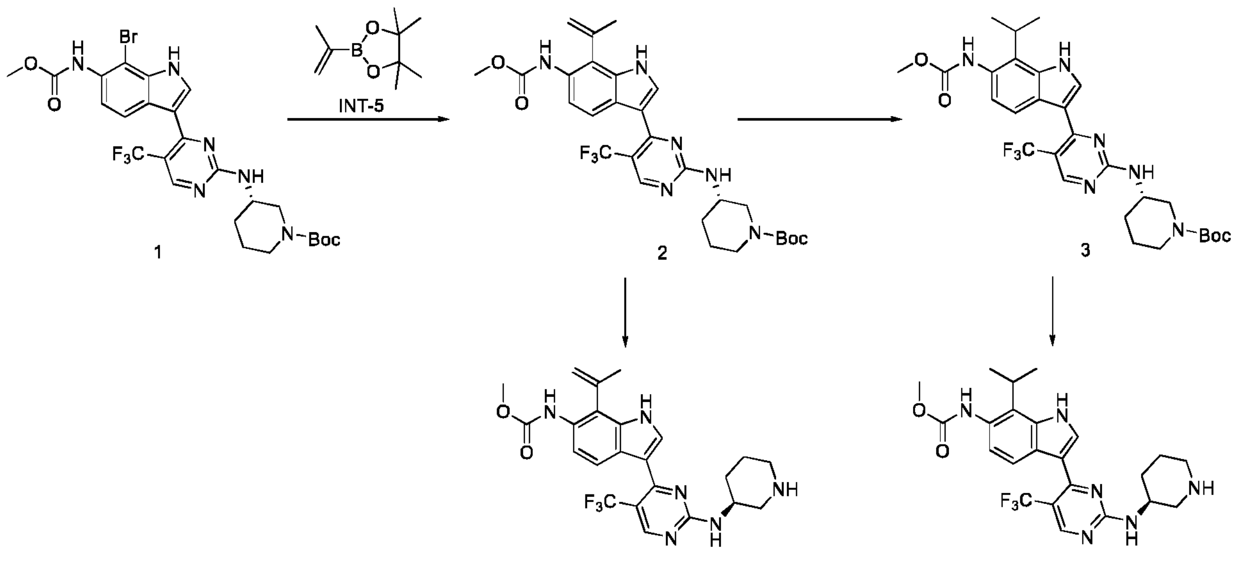


1 2 3 T-70 T-69

Synthesis step:

(1) Synthesis of Compound 2
1.0g(1.0eq) Compound 1, 1.37g(5.0eq) isopropenyl boronate, 0.24g(0.2eq) Pd(dppf)$Cl_2$, 0.67g(3.0eq) potassium carbonate, 20ml of dioxane and 2ml of water as solvent were mixed evenly. After three times of nitrogen replacement, the oil bath was heated to 100 °C to react for18h under nitrogen protection. TLC showed that there was no remaining raw materials. Water and EA were added for extraction and drying. The samples were mixed and passed through a column to obtain 0.7g of compound 2. with a yield of 75%.
(2) Synthesis of Compound 3
200mg(1eq) compound 2, 3ml methanol and 10mg Pd/C were sequentially added to the reaction system. After the air was replaced by $H_2$, the reaction was carried out overnight. The reaction was completed after 36 hours, the diatomite pad was used to remove excess palladium carbon, and the mother liquid was spin-dried to obtain 150mg of crude product 3.
(3) Synthesis of Compound T-69
150mg of compound 3, 2ml 4M HCl/dioxane solution and 4mL EA were added to the reaction system at room temperature. After 5h, TLC monitored until the reaction was completed, and suction filtered to obtain crude target product( HPLC purity: 84%), , freed with saturated sodium bicarbonate solution, and purified by preparation plate after extraction with EA to obtain 38mg of T-69 with a purity of 93.8%. $^1$H NMR (400 MHz, DMSO-$d_6$) $\delta$ 11.50 (s, 1H), 8.60 (d, $J$ = 8.9 Hz, 1H), 8.21 (d, $J$ = 8.6 Hz, 1H), 8.06 - 7.86 (m, 1H), 7.80 - 7.63 (m, 1H), 6.95 (t, $J$ = 10.9 Hz, 1H), 4.20 (s, 1H), 3.62 (s, 2H), 3.08 (d, $J$ = 13.0 Hz, 1H), 2.80 (dd, $J$ = 20.8, 11.1 Hz, 2H), 1.99 (s, 1H), 1.38 (d, $J$ = 7.1 Hz, 4H), 1.23 (s, 2H).
(4) Synthesis of Compound T-70
400mg(1eq) compound 2, 3mL 4M hydrochloric acid/dioxane solution and 5mL EA were sequentially added to the

reaction system and reacted overnight at room temperature. After the reaction was completed, freed with saturated sodium bicarbonate solution, extracted and spin-dried. 53mg of T-70 was obtained by preparation liquid phase separation. $^1H$ NMR (400 MHz, DMSO-$d_6$) δ 11.51 (s, 1H), 8.65 - 8.48 (m, 1H), 7.72 (s, 1H), 7.13 (d, *J* = 8.9 Hz, 1H), 5.51 - 5.38 (m, 1H), 5.03 (s, 1H), 3.60 (s, 2H), 3.35 (s, 8H), 3.06 (d, *J*= 11.8 Hz, 1H), 2.80 (d, *J*= 12.5 Hz, 1H), 2.07 (s, 2H), 1.99 (s, 1H), 1.72 - 1.53 (m, 1H), 1.52 - 1.36 (m, 1H), 1.23 (s, 1H).

**Example T-71**

The compound synthesized by the invention:

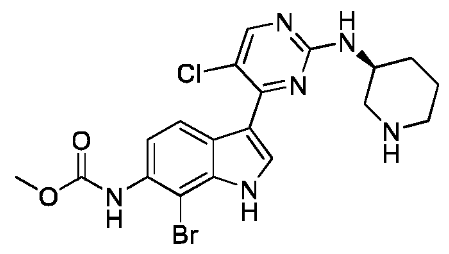

T-71

The experimental process was as follows:
The synthetic route is as follows:

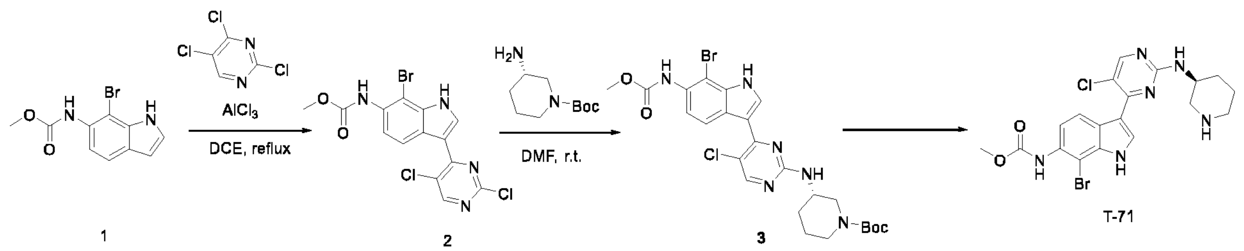


Synthesis step:

(1) Synthesis of Compound 2
Under nitrogen condition, 2,4,5-trichloropyrimidine (1.5 e.q.) and aluminum trichloride (2.0 e.q.) were added into a three-necked flask containing 100 mL dichloroethane, the temperature was raised to reflux, 2.78g of compound **1** was added after being reacted for half an hour, the temperature was kept to react overnight. After the reaction was completed, ice water was added for quenching, the reaction solution was extracted and separated by DCM and water, concentrated to remove most DCM, recrystallized with EA, suction filtered to obtain 1.16 g of compound 2 as a solid with a yield of 27.2%.
(2) Synthesis of Compound 3
400 mg of compound **2,** (S)-1-Boc-3-aminopiperidine(2.0 e.q.), DIPEA(5.0 e.q.) and 15 mL NMP as solvent were added into 100 mL of three-necked flask to react at room temperature under nitrogen. After the reaction was completed, the reaction solution was extracted and separated by EA and water , and the organic phase was dried over anhydrous sodium sulfate, separated and purified by silica gel column chromatography to obtain 230 mg of compound **3** with a yield of 41.3%.
(3) Synthesis of Compound T-71
100 mg of compound **3** was added into a 50 mL three-necked flask, 5 mL of dichloromethane was added, after being dissolved to be clear, and then 2 mL of trifluoroacetic acid was added to react under stirring at room temperature for half an hour. After the reaction was completed, concentrated to remove excess solvent, the pH value was adjusted to be alkaline, and the mixture was extracted and spin-dried, separated and purified by a medium pressure preparation reverse column to obtain 40 mg of compound **4** with a yield of 48.4%, HPLC purity: 96.6%, LC-MS[M +1]:479.0, 481.0.

The following compounds were synthesized with reference to the methods in Examples T-01 and T-71:

| Example | Compound structure | Characterization data |
|---|---|---|
| T-72 | 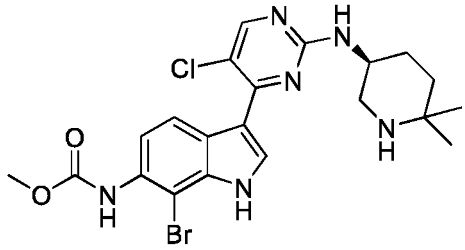 T-72 | LC-MS[M+1]:507.0,509.0 |
| T-75 | 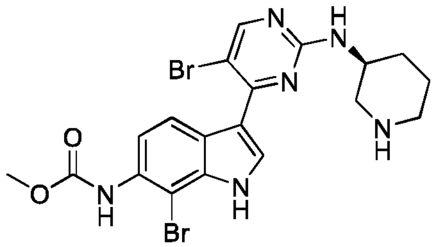 T-75 | LC-MS[M+1]:524.9,526.9 |
| T-76 | 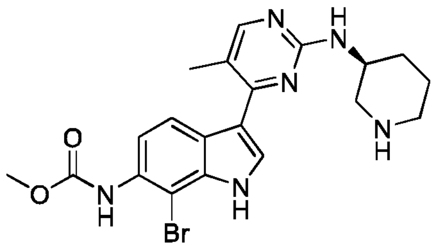 T-76 | LC-MS[M+1]:459.1,461.1 |
| T-77 | 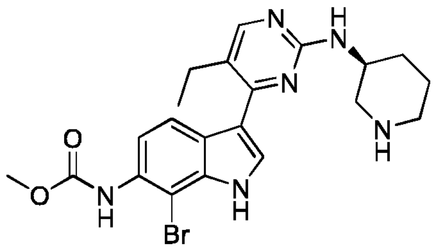 T-77 | LC-MS[M+1]:473.1,475.1 |
| T-78 | 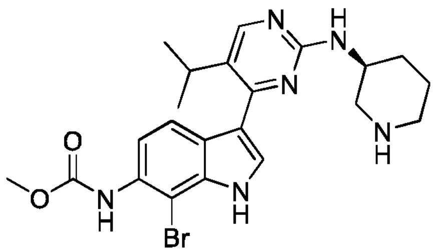 T-78 | LC-MS[M+1]:487.1,489.1 |

**Example T-79**

The compound synthesized by the invention:

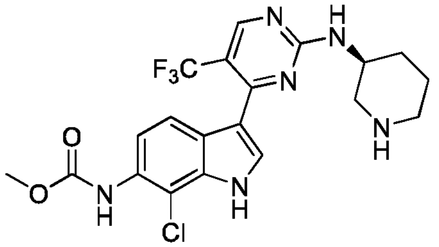

T-79

The experimental process was as follows:
The synthetic route is as follows:

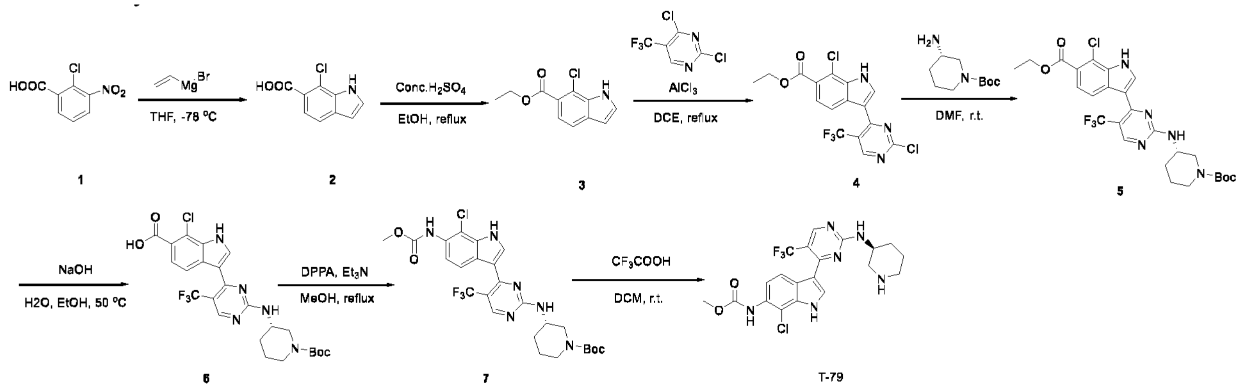


Synthesis step:

(1) Synthesis of Compound 2

Under nitrogen condition, 20g of compound **1 and** 200 mL of anhydrous tetrahydrofuran were added to a 500 mL three-necked flask respectively, cooled to -78 °C, a solution of vinyl magnesium bromide (10 e.q.) in THF was added dropwise, and the temperature was controlled for reaction. After the reaction was completed, water was added for quenching, extracted with EA and water, and the organic phase was dried over anhydrous sodium sulfate, concentrated, and separated and purified by silica gel column chromatography to obtain 3.56g of compound **2.**

(2) Synthesis of Compound 3

3.56g of compound 2 and 35 mL of anhydrous ethanol were added into 150 mL of three-necked flask respectively, concentrated sulfuric acid (1.0 e.q.) was used as catalyst, reacted at reflux overnight under nitrogen condition. After the reaction was completed, cooled to room temperature, concentrated to remove excess solvent, separated and purified by silica gel column chromatography to obtain 1.6g of compound **3.**

(3) Synthesis of Compound 4

Under nitrogen condition, 2,4-dichloro-5-trifluoromethylpyrimidine (2.0 e.q.) and aluminum trichloride (3.0 e.q.) were added to a three-necked flask containing 50 mL dichloroethane, the temperature was raised to reflux to react for half an hour, 1.7 g of compound 3 was added . The temperature was kept to react overnight. After the reaction was completed, ice water was added for quenching, the reaction solution was extracted and separated by DCM and water, concentrated to remove most DCM, recrystallized with EA, suction filtered to obtain 500 mg of compound 4 as a solid with **a** yield of 16.3%, LC-MS[M +1] :404.0, 406.0.

(4) Synthesis of Compound 5

500 mg compound 4, (S)-1-Boc-3-aminopiperidine (2.0 e.q.) and 10 mL DMF as solvent were added into a 100 mL three-necked flask to react at room temperature under nitrogen. After the reaction was completed, DMF solvent was removed by spin evaporation, separated and purified by silica gel column chromatography to obtain 384 mg of compound 5 with a yield of 54.6%, LC-MS[M +1]:568.1, 570.1.

(5) Synthesis of Compound 6

384 mg of compound **5 was added** into a 50 mL three-necked flask, dissolved to be clear with 10 mL of ethanol, then 5 M of sodium hydroxide aqueous solution (5.0 e.q.) was added dropwise, the temperature was kept at 50 °C to react overnight to obtain 353.1 mg of hydrolysate **6** with a yield of 97.0%.

(6) Synthesis of Compound 7

Under nitrogen condition, 150 mg of compound 6 and 5 mL of anhydrous methanol were added into 10 mL of reaction branch pipe successively. After being dissolved to be clear, DPPA(10.0 e.q.) and $Et_3N$(5.0 e.q.) were added respectively. The temperature was raised until reflux to react for two hours. After the reaction was completed, cooled to room temperature, quenched with water, the reaction solution was extracted and separated by EA and water, and the organic phase was dried over anhydrous sodium sulfate, concentrated, separated and purified by silica gel column chromatography to obtain 100 mg of compound 7 with a yield of 63.3%, LC-MS[M +1]:569.2, 571.2.

(7) Synthesis of Compound T-79

Under nitrogen condition, 100mg of compound 7, 6 mL of dichloromethane and 2 mL of trifluoroacetic acid were added into a 50 mL three-necked flask respectively to react under stirring at room temperature. After the reaction was completed, concentrated, the pH value was adjusted to be 8, then extracted, dried and spin-dried, and purified by column to obtain 40 mg of compound T-79 with a yield of 48.8%, HPLC purity: 93%,LC-MS[M +1]:469.1, 471.1.

Example T-80

The compound synthesized by the invention:

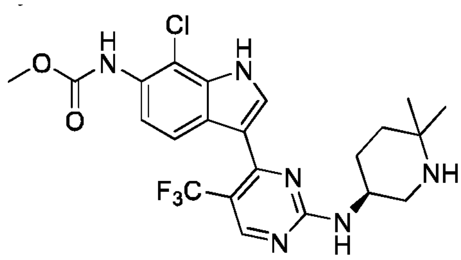

T-80

The experimental process was as follows:
The synthetic route is as follows:

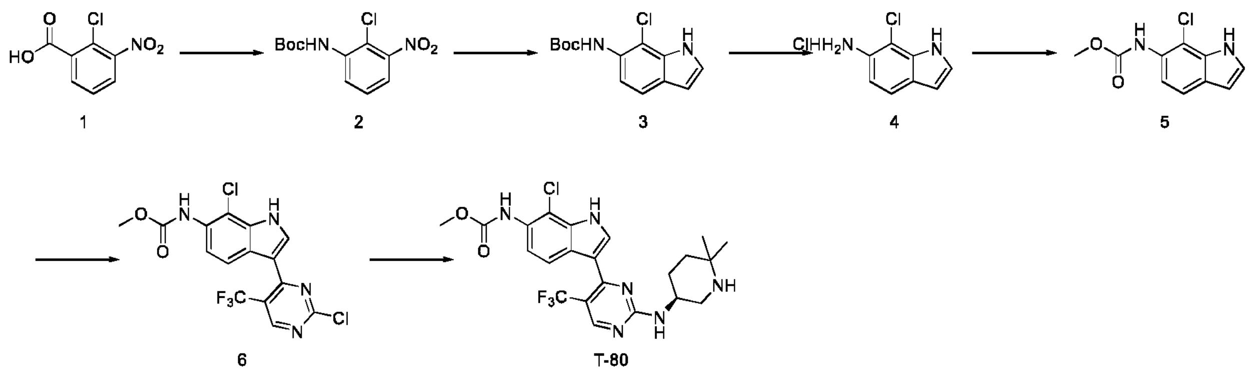

Synthesis step:

(1) Synthesis of Compound 2

20g of compound 1 was dissolved in tert-butanol (300mL), triethylamine (20.2g,0.2mol) was added into the reaction solution under stirring at room temperature, the solution changed from clear yellow to clear reddish brown. Under nitrogen protection, DPPA(41.3g,0.15mol) was slowly added dropwise, the reaction temperature was controlled to prevent spraying. The thermometer monitored the temperature to be stabilized at 80°C to react for 3-5 hours. Ammonium chloride aqueous solution was added to quench the reaction, then extracted with EA. After extraction, the organic phases were combined, washed with saturated sodium chloride once, dried over anhydrous sodium sulfate, spin-dried, and purified by column( pure EA/PE = 10: 1) to obtain 26g of compound 2.

(2) Synthesis of Compound 3

Under nitrogen protection, vinyl magnesium bromide (670mL, 1mol/L,0.67moL) was added into the reaction flask, stirred in a liquid nitrogen bath until the temperature was dropped to -70 °C to -80 °C, then compound 2(26g) was dissolved in THF(100mL), slowly dripped into the reaction solution under nitrogen protection. After addition, the mixture was naturally heated to room temperature to react overnight. Under the ice bath, the reaction solution was quenched with saturated ammonium chloride. At the beginning, a large number of bubbles were generated. The temperature was raised quickly. Pay attention to the dripping speed to prevent spraying. After quenching with saturated ammonium chloride, a large number of solids were precipitated, and then EA was added for extraction. The insoluble solids can be dissolved with water. After extraction, the combined organic phase was washed with saturated sodium chloride once, dried over anhydrous sodium sulfate, spin-dried and purified by column (PE:EA = 5:1) to obtain 13g of compound 3.

(3) Synthesis of Compound 4

Compound 3(13g) was added to the reaction flask in an ice bath, then 50mL of 1,4-dioxane was added, then 4mol/L of hydrochloric acid/1,4-dioxane solution (80mL) was added dropwise under stirring, and then reacted for 5 hours. After the reaction was completed, a large amount of solid was precipitated, filtered to obtain 12g of compound 4.

(4) Synthesis of Compound 5

Under nitrogen condition, compound 4(12g) was added to the reaction flask in the ice bath, then 100mL dichloromethane was added, then sodium carbonate (40.7g,0.3mol) was added, finally, methyl chloroformate (11.3g,0.12mol) was slowly added dropwise. After the addition, the ice bath was maintained for 3 hours. Then ice water was added to quench the reaction, and then EA was used for extraction. After extraction, the organic phase was washed with saturated sodium chloride once, dried over anhydrous sodium sulfate, spin-dried, and purified by column (PE:EA = 2:1) to obtain 4g of compound 5.

(5) Synthesis of Compound 6

Under nitrogen condition, aluminum trichloride (0.03mol) was added to a solution of 2,4-dichloro-5-trifluoromethylpyrimidine (0.034mol) in DCE(150mL), then heated to 80 °C to react for 30min, then 4g of compound 5 was slowly added, and continued to react overnight at 80 °C. The reaction was quenched with water, and then extracted with dichloromethane. After extraction, the organic phase was washed with saturated sodium chloride once, dried over anhydrous sodium sulfate, after spin-drying was completed, the solid was slurried in EA(50mL) to obtain 3g of compound 6.

(6) Synthesis of Compound T-80

400 mg of compound 2, (S)-6,6 -dimethyl -3-aminopiperidine hydrochloride(2.0 e.q.), DIPEA(5.0 e.q.) and 15 mL NMP as solvent were added into a 100 mL three-necked flask to react at room temperature under nitrogen. After the reaction was completed, the reaction solution was extracted and separated by EA and water , and the organic phase was dried over anhydrous sodium sulfate, separated and purified by silica gel column chromatography to obtain 130 mg of compound T-80 with a yield of 26.1%.

The following compounds were synthesized with reference to the methods in Examples T-79 and T-80:

| Example | Compound structure | Characterization data |
|---|---|---|
| T-81 | 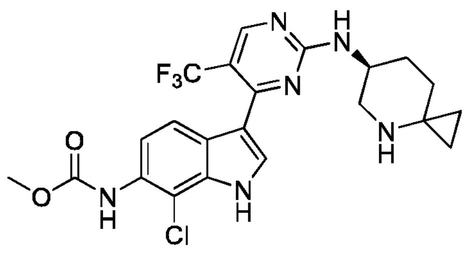 T-81 | LC-MS[M +1]:495.1,497.1 |

(continued)

| Example | Compound structure | Characterization data |
|---|---|---|
| T-82 | 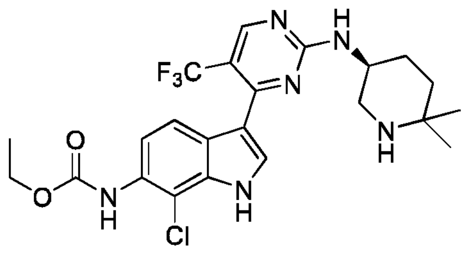 T-82 | LC-MS[M+ 1]:511.1,513.1 |
| T-126 | 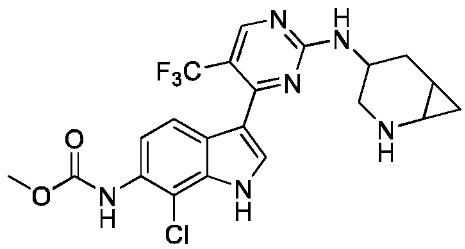 T-126 | LC-MS[M+1]:481.1,483.1 |
| T-145 | 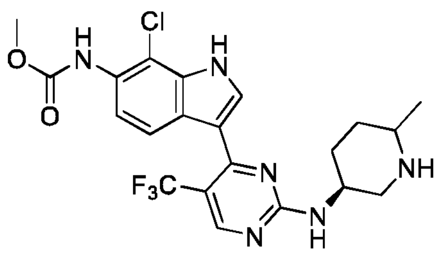 T-145 | LC-MS[M+1]:483.1,485.1 |
| T-146 | 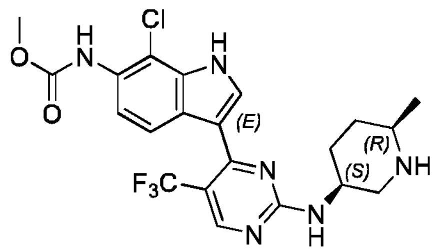 T-146 | LC-MS[M+1]:483.1,485.1 |
| T-147 | 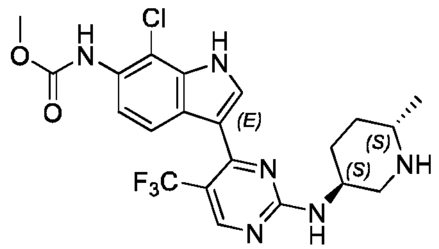 T-147 | LC-MS[M+1]:483.1,485.1 |

(continued)

| Example | Compound structure | Characterization data |
|---|---|---|
| T-148 | 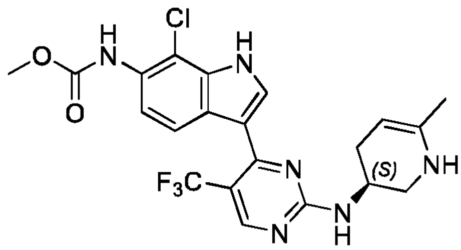 T-148 | LC-MS[M+1]:481.1,483.1 |
| T-168 | 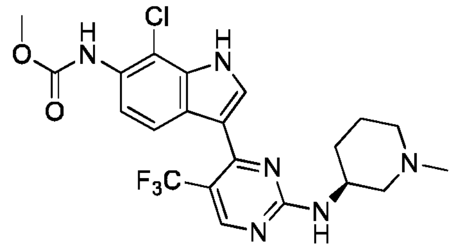 T-168 | LC-MS[M+1]:483.1,485.1 |

**Example T-98**

The compound synthesized by the invention:

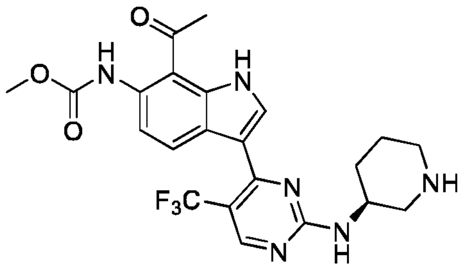

T-98

The experimental process was as follows:
The synthetic route is as follows:

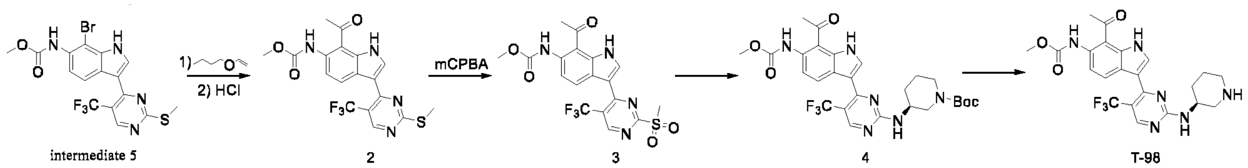


intermediate 5 2 3 4 T-98

Synthesis step:

(1) Synthesis of Compound 2
17g(1 eq) of intermediate 5 dissolved in 170ml of n-butanol was added to a 250 ml three-necked flask, stirred to dissolve, 10.1g(2eq) of potassium carbonate, 7.4g(2.0eq) of vinyl butyl ether, 3.3g(0.4eq) of Xantphos and

1.6g(0.2eq) of palladium acetate were added in turn, replaced with nitrogen for 3 times, and reacted overnight at 95 °C. TLC monitored until the reaction was completed. Water was added, extracted with ethyl acetate for 2 times, the ethyl acetate phase was transferred to 250ml one-necked flask, 5ml 1.5eq concentrated hydrochloric acid was added, stirred at room temperature for 3 hours. The reaction was monitored by TLC to be completed. 13.4g compound 2 was obtained by direct purification through the column.

(2) Synthesis of Compound 3

8g compound 2 was dissolved in 160 mL DCM in a 250 ml one-necked flask, cooled to 0 °C in the ice bath, 9.7g(3 eq) m-CPBA was added under stirring, a large amount of solid was precipitated after being reacted for 3 h at room temperature, and TLC monitored until the reaction was completed. 5.4g of compound 3 was obtained by direct suction filtration, rinsing with DCM and drying.

(3) Synthesis of Compound 4

500mg of compound 3 was dissolved in 5ml DMF in a 50 ml one-necked flask, 440mg(2eq) (S)-1-Boc-3-aminopiperidine and 0.28g(2eq) diisopropyl ethylamine were added to react for 5h at room temperature, TLC monitored until the reaction was completed. Water was added, extracted with ethyl acetate, washed with saturated brine, dried over anhydrous sodium sulfate, spin-dried, separated by column chromatography to obtain 300mg of compound 4.

(4) Synthesis of T-98

300mg compound 4 and 10ml 4M/L dioxane hydrochloride were added into a 50 ml round bottom flask to react for 3h at room temperature, and TLC monitored until the reaction was completed. Saturated sodium bicarbonate solution was added, extracted with ethyl acetate, dried over anhydrous sodium sulfate, spin-dried, and purified by preparative plate to obtain 100mg of compound T-98. $^1$H NMR (400 MHz, DMSO-$d_6$) δ 11.59 (s, 1H), 9.80 (s, 1H), 8.60 (d, J = 8.4 Hz, 1H), 8.55 - 8.37 (m, 1H), 7.80 (m, 2H), 7.22 (d, J = 8.6 Hz, 1H), 3.96 - 3.81 (m, 1H), 3.69 (s, 3H), 3.15 - 3.00 (m, 1H), 2.84 - 2.77 (m, 1H), 2.53 (d, J = 1.5 Hz, 3H), 1.99 (s, 1H), 1.70 - 1.60 (m, 1H), 1.47 (m, 2H), 1.24 (m, 1H), 0.89 - 0.79 (m, 2H).

**Example T-99**

The compound synthesized by the invention:

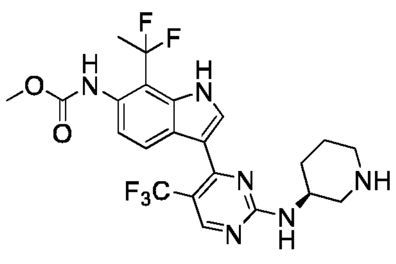

T-99

The experimental process was as follows:
The synthetic route is as follows:

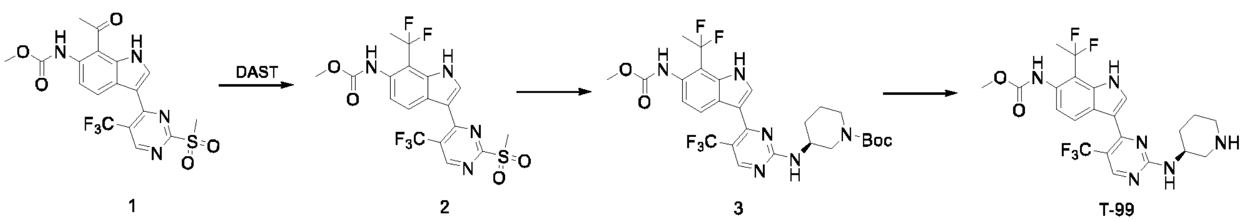


Synthesis step:

(1) Synthesis of Compound 2

1g(1 eq) compound 1 was added into a 50 ml one-necked flask, 20ml tetrahydrofuran was added and dissolved under stirring , 3ml(3eq) DAST was added dropwise in an ice bath to react overnight at room temperature. The reaction was monitored by TLC to be completed. Water was added in the ice bath, extracted with ethyl acetate twice, purified by passing through the column to obtain 150mg of compound 2.

(2) Synthesis of Compound 3:

150mg of compound 2 was dissolved in 2ml of tetrahydrofuran in a 25 ml one-necked flask, 125mg(2eq) (S)-1-Boc-3-aminopiperidine and 81mg(2eq) diisopropyl ethylamine were added to react at room temperature for 3h, and TLC monitored until the reaction was completed. Water was added, extracted with ethyl acetate, washed with saturated brine, dried over anhydrous sodium sulfate, spin-dried, separated by column chromatography to obtain 100mg of compound 3.

(3) Synthesis of T-99:

100mg compound 3 and 3ml 4M/L dioxane hydrochloride were added into a 25 ml round bottom flask to react for 3h at room temperature, and TLC monitored until the reaction was completed. Saturated sodium bicarbonate solution was added, extracted with ethyl acetate, dried over anhydrous sodium sulfate, spin-dried, and purified by preparative plate to obtain 40mg of compound T-99. $^1$H NMR (400 MHz, DMSO-$d_6$) $\delta$ 11.54 (s, 1H), 8.89 (d, J = 18.0 Hz, 1H), 8.64 (d, J = 5.6 Hz, 1H), 8.46 (d, J = 8.4 Hz, 1H), 8.07 (s, 1H), 7.79 (d, J = 8.4 Hz, 1H), 7.10 (t, J = 10.0 Hz, 1H), 4.22 (s, 1H), 3.63 (s, 3H), 3.15 (s, 1H), 2.82 (s, 2H), 2.08 (t, J = 19.2 Hz, 3H), 1.89 (d, J = 12.0 Hz, 1H), 1.64 (s, 2H), 1.24 (s, 1H), 0.99 - 0.74 (m, 2H).

The following compounds were synthesized with reference to the methods in examples T-98 and T-99 :

| Example | Compound structure | Characterization data |
|---|---|---|
| T-134 | 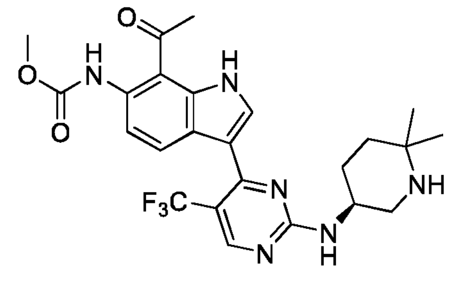 T-134 | LC-MS[M+1]:505.2 |
| T-135 | 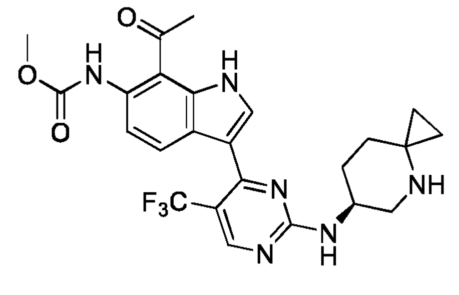 T-135 | LC-MS[M+1]:503.2 |
| T-136 | 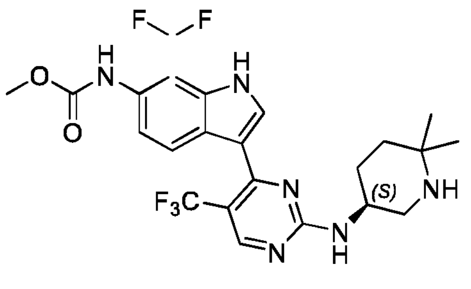 T-136 | LC-MS[M+1]:527.2 |

(continued)

| Example | Compound structure | Characterization data |
|---|---|---|
| T-137 | 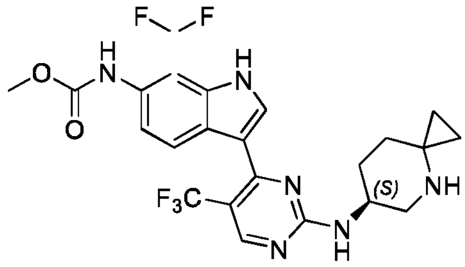 T-137 | LC-MS[M+1]:525.2 |
| T-153 | 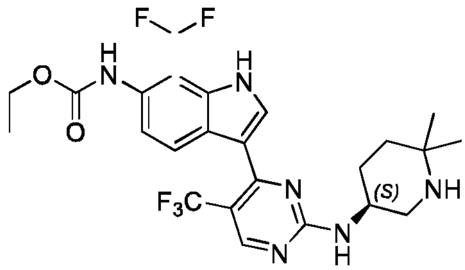 T-153 | LC-MS[M+1]:541.2 |

**Example T-102**

The compound synthesized by the invention:

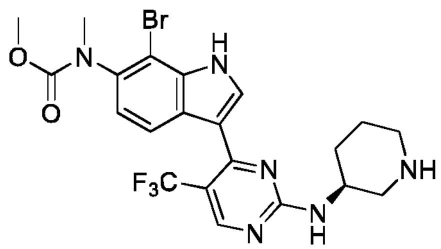

T-102

The experimental process was as follows:
The synthetic route is as follows:

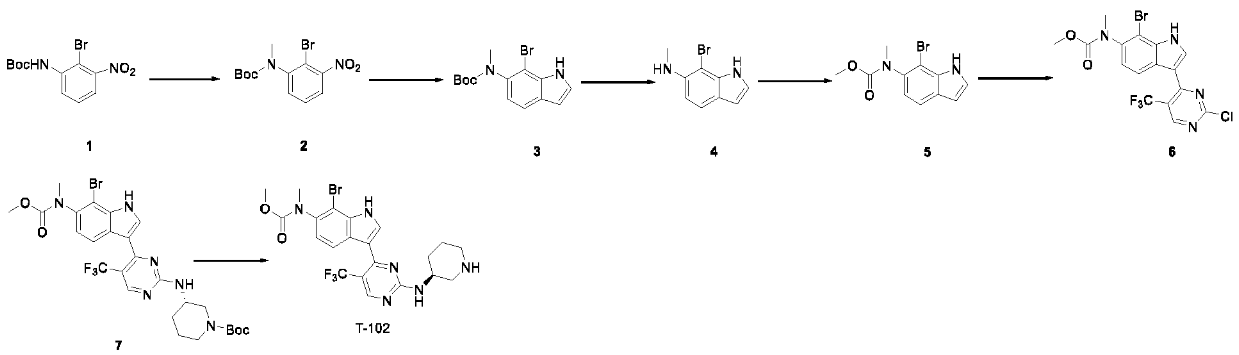

1 2 3 4 5 6 7 T-102

Synthesis step:

(1) Synthesis of Compound 2

4.7g(1eq) compound 1 was dissolved in 10mL THF, 1.8g(6eq) of NaH was added in a salt ice bath, stirred for 5min, then 1.6g(5eq) of $CH_3I$ was added to react at room temperature. After the reaction was completed, 50mL of water was added, extracted with EA and dried, spin-dried to obtain 4.9g of crude compound 2.

(2) Synthesis of Compound 3

4.9g(1eq) compound 1 was dissolved in 40mL THF. Under the condition of -78 °C and $N_2$ protection, vinyl magnesium bromide (6eq) was slowly added dropwise, and the temperature was raised to room temperature for reaction after the addition. After the reaction was completed, 50mL of water was added, extracted with EA and dried, spin-dried, and purified by column to obtain 1.5g of compound 3.

(3) Synthesis of Compound 4

1.5g(1eq) compound 5 was taken and added into 10mL 4M/L dioxane hydrochloride to react at room temperature. After the reaction was completed, $NaHCO_3$ aqueous solution was added to adjust the solution to be alkaline, extracted with EA and dried, spin-dried, 1.0g of compound 4 was obtained by preparation separation. LC-MS[M +1]:224.9, 226.9.

(4) Synthesis of Compound 5

1.0g(1eq) compound 4 was dissolved in 6ml of methyl chloroformate, 300mg of $Na_2CO_3$ was added to react at room temperature. After the reaction was completed, water was added extracted with EA and dried, spin-dried to obtain 0.86g of crude compound 5 .

(5) Synthesis of Compound 6

In a 50 mL round bottom flask, 1.5g(3eq) 2,4-dichloro-5-trifluoromethylpyrimidine, 25 mL DCE and 0.5g(1.7eq) $AlCl_3$ were added, the temperature was heated to 80 °C to react for 30min under nitrogen protection, 0.5g(1eq) compound 2 was added, and the reaction was continued at 80 °C under nitrogen protection. After the reaction was completed, water was added, extracted with EA, and the organic layer was evaporated, and EA was added for slurrying to obtain 0.3g of compound 6. LC-MS[M +1]:462.9, 464.9.

(6) Synthesis of Compound 7

0.3g(1.0eq) compound 3 was dissolved in 6mL DMF, 0.3g(2.2eq) of (S)-1-Boc-3-aminopiperidine was added to react overnight under stirring at room temperature. After the reaction was completed, water was added, extracted with EA and dried, spin-dried, and purified by column to obtain 0.3g of compound 7.

(7) Synthesis of Compound T-102

250mg(1eq) compound 7 was taken and added into 6 mL 4M/L dioxane hydrochloride and 4mL EA to react at room temperature. After the reaction was completed, filtered, the filter cake was washed with methyl tert-butyl ether to obtain 200mg of compound T-102. HPLC:97.4%. LC-MS[M +1]:527.0, 529.0. $^1H$ NMR (400 MHz, DMSO-$d_6$) δ 12.40 - 12.06 (m, 1H), 8.66 (d, *J* = 7.2 Hz, 1H), 8.27 (m, 2H), 7.84 (s, 1H), 7.34 - 6.92 (m, 1H), 4.31 (s, 1H), 4.03 (q, *J* = 7.2 Hz, 1H), 3.71 (s, 1H), 3.55 (d, *J* = 18.3 Hz, 3H), 3.21 (s, 3H), 3.00 - 2.72 (m, 2H), 1.95 (d, *J* = 36.9 Hz, 2H), 1.67 (d, *J* = 61.6 Hz, 2H).

The following compounds were synthesized with reference to the method in Example T-102:

| Example | Compound structure | Characterization data |
|---|---|---|
| T-162 | 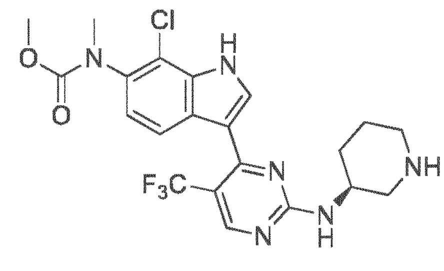 T-162 | LC-MS[M+1]:483.1,485.1 |

(continued)

| Example | Compound structure | Characterization data |
|---|---|---|
| T-163 | 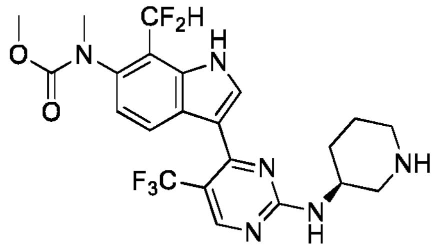 T-163 | LC-MS[M+1]:499.2 |
| T-164 | 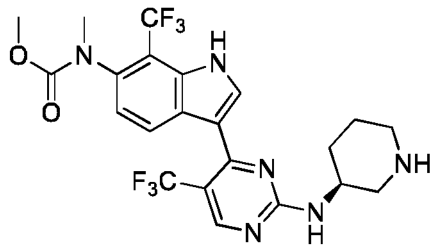 T-164 | LC-MS[M+1]:517.1 |
| T-165 | 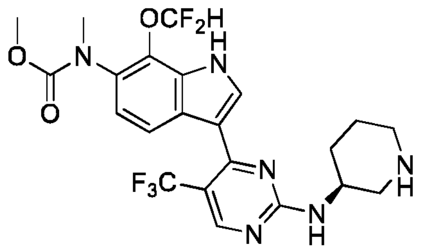 T-165 | LC-MS[M+1]:515.1 |
| T-166 | 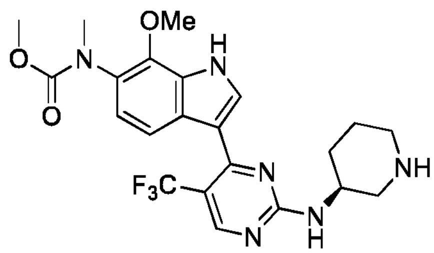 T-166 | LC-MS[M+1]:479.2 |
| T-170 | 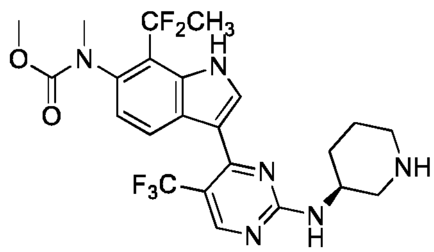 T-170 | LC-MS[M+1]:513.2 |

(continued)

| Example | Compound structure | Characterization data |
|---|---|---|
| T-175 | 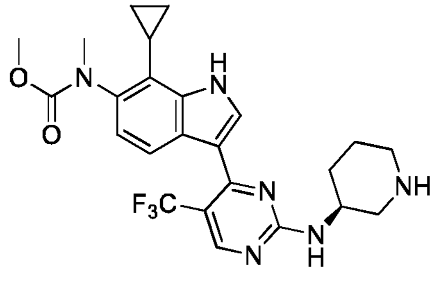 T-175 | LC-MS[M+1]:489.2 |

Compound 101 in WO2020093006A1 was synthesized by reference to the patent application, as a comparative compound for the present invention, and the structural formula thereof is as follows:

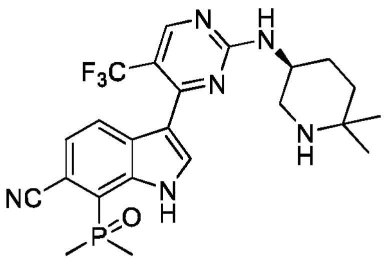

compound101

**Test Example 1 Enzyme Activity Test**

The biological activity test of some of the compounds above and the comparative compound was carried out.

The biological activity test experiment process was as follows:
$IC_{50}$ values of the compounds for CDK7 kinase were detected in Wuxi Biortus Biosciences Co. Ltd..

1. Compound preparation:
The compound powder was dissolved in 100% DMSO to prepare a 10mM storage solution. The storage solution was further diluted to 0.5mM as the initial concentration, and continuously diluted 3 times to obtain 10 compound solutions with different concentrations. The compound and enzyme were pre-incubated for 0 minutes and 60 minutes, respectively, using duplicate detection. Compound Staurosporine having a structural formula of

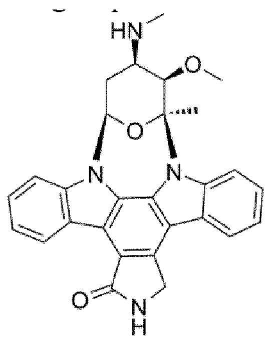

was used as a positive control, and Mobility shift assay method was used to detect CDK kinase (such as CDK7, CDK9, CDK12 and CDK2) activity of the compounds.
2. Kinase reaction process:

1) The compound solution and the positive control were diluted 8.3 times with double distilled water and then added to the 384 well plate, each concentration was 2uL/well;

(2) 6nM CDK7/Cyclin H/MAT1 kinase solution was added to the compound well and the positive control well respectively;

(3) incubating at room temperature for 0 and 60 minutes;

(4) 2mM ATP and 2pM 5-FAM-CDK7 peptide substrate (5-FAM-YSPTSPSYSPTSPSYSPTSPSKKKK) solution (8 nM CDK9/Cyclin Tl polymer and 2 pM 5-FAM GSRTPMY-NH2 peptide substrate were used in CDK9 inhibition reaction; 50 nM CDK12 (aa686-1082)/Cyclin K polymer and 2 pM 5-FAM GSRTPMY-NH2 peptide substrate were used in CDK12 inhibition reaction; 0.5 nM CDK2/Cyclin El polymer and 2 pM 5-FAM-YSPTSPSYSPTSPSYSPTSPSKKKK peptide substrate were used in CDK2 inhibition reaction) were added;

(5) Incubating the 384 well plate at 25°C for 30 minutes;

(6) 4uL 120 mM EDTA was added to stop the kinase reaction;

(7) the conversion rate was read with Caliper/LabChip EZ Reader (Perkin Elmer);

(8) $IC_{50}$ value was obtained by curve fitting using GraphPad Prism 8 software.

Through the above detection, $IC_{50}$ (nM) value of the test samples for the inhibitory activity of CDK7 kinase was obtained and showed in Table 1, wherein A≤ 50nM; 50nM<B<500 nM; C≥ 500nM.

Table 1

| Compound | CDK7 kinase $IC_{50}$ (nM) | CDK2 kinase $IC_{50}$(nM) | CDK9 kinase $IC_{50}$(nM) | CDK12 kinase $IC_{50}$(nM) |
|---|---|---|---|---|
| Staurosporine | C | A | B | C |
| T-01 | A | C | C | C |
| T-02 | B | C | C | C |
| T-03 | A | C | C | C |
| T-04 | A | C | C | C |
| T-05 | A | C | C | C |
| T-06 | A | C | C | C |
| T-07 | A | C | C | C |
| T-08 | A | C | C | C |
| T-09 | A | C | C | C |
| T-10 | A | C | C | C |
| T-11 | A | C | C | C |
| T-12 | A | C | C | C |
| T-13 | A | C | C | C |
| T-14 | A | C | C | C |
| T-15 | A | C | C | C |
| T-16 | A | C | C | C |
| T-17 | A | C | C | C |
| T-18 | A | C | C | C |
| T-19 | A | C | C | C |
| T-20 | B | C | C | C |
| T-21 | B | C | C | C |
| T-22 | B | C | C | C |
| T-23 | B | C | C | C |
| T-24 | A | C | C | C |
| T-25 | B | C | C | C |

(continued)

| Compound | CDK7 kinase $IC_{50}$ (nM) | CDK2 kinase $IC_{50}$(nM) | CDK9 kinase $IC_{50}$(nM) | CDK12 kinase $IC_{50}$(nM) |
|---|---|---|---|---|
| T-26 | B | C | C | C |
| T-27 | A | C | C | C |
| T-28 | B | C | C | C |
| T-29 | C | C | C | C |
| T-30 | C | NT | NT | NT |
| T-31 | A | C | C | C |
| T-32 | B | NT | NT | NT |
| T-33 | B | NT | NT | NT |
| T-34 | A | C | C | C |
| T-35 | A | C | C | C |
| T-36 | A | C | C | C |
| T-37 | A | B | C | C |
| T-38 | A | NT | NT | NT |
| T-39 | B | NT | NT | NT |
| T-40 | B | NT | NT | NT |
| T-41 | B | NT | NT | NT |
| T-42 | A | C | C | C |
| T-43 | A | C | C | C |
| T-44 | A | C | C | C |
| T-45 | B | NT | NT | NT |
| T-46 | B | NT | NT | NT |
| T-47 | B | NT | NT | NT |
| T-48 | A | C | C | C |
| T-49 | A | NT | NT | NT |
| T-50 | C | NT | NT | NT |
| T-51 | C | NT | NT | NT |
| T-52 | A | C | C | C |
| T-53 | C | NT | NT | NT |
| T-54 | B | NT | NT | NT |
| T-55 | C | NT | NT | NT |
| T-56 | B | NT | NT | NT |
| T-57 | B | NT | NT | NT |
| T-58 | B | NT | NT | NT |
| T-59 | B | NT | NT | NT |
| T-60 | A | C | C | C |
| T-61 | B | NT | NT | NT |
| T-62 | A | C | C | C |
| T-63 | A | C | C | C |

(continued)

| Compound | CDK7 kinase $IC_{50}$ (nM) | CDK2 kinase $IC_{50}$(nM) | CDK9 kinase $IC_{50}$(nM) | CDK12 kinase $IC_{50}$(nM) |
|---|---|---|---|---|
| T-64 | A | C | C | C |
| T-65 | A | C | C | C |
| T-66 | A | C | C | C |
| T-67 | A | C | C | C |
| T-68 | A | C | C | C |
| T-69 | A | C | C | C |
| T-70 | A | C | C | C |
| T-71 | A | NT | NT | NT |
| T-72 | A | NT | NT | NT |
| T-73 | C | NT | NT | NT |
| T-74 | C | NT | NT | NT |
| T-75 | A | C | C | C |
| T-76 | A | NT | NT | NT |
| T-77 | A | NT | NT | NT |
| T-78 | B | NT | NT | NT |
| T-79 | A | C | C | C |
| T-80 | A | C | C | C |
| T-81 | A | C | C | C |
| T-82 | A | C | C | C |
| T-83 | A | NT | NT | NT |
| T-84 | A | C | C | C |
| T-85 | A | C | C | C |
| T-86 | A | C | C | C |
| T-87 | A | NT | NT | NT |
| T-88 | A | C | C | C |
| T-89 | A | NT | NT | NT |
| T-90 | A | NT | NT | NT |
| T-91 | A | NT | NT | NT |
| T-92 | B | NT | NT | NT |
| T-93 | A | NT | NT | NT |
| T-94 | A | NT | NT | NT |
| T-95 | A | NT | NT | NT |
| T-96 | A | C | C | C |
| T-97 | A | NT | NT | NT |
| T-99 | A | NT | NT | NT |
| T-100 | A | C | C | C |
| T-101 | A | NT | NT | NT |
| T-102 | A | C | C | C |

(continued)

| Compound | CDK7 kinase $IC_{50}$ (nM) | CDK2 kinase $IC_{50}$(nM) | CDK9 kinase $IC_{50}$(nM) | CDK12 kinase $IC_{50}$(nM) |
|---|---|---|---|---|
| T-102 | A | C | C | C |
| T-103 | A | NT | NT | NT |
| T-105 | A | C | C | C |
| T-106 | A | C | C | C |
| T-107 | A | C | C | C |
| T-108 | A | C | C | C |
| T-109 | A | C | C | C |
| T-110 | A | NT | NT | NT |
| T-111 | A | NT | NT | NT |
| T-115 | A | NT | NT | NT |
| T-117 | A | NT | NT | NT |
| T-121 | A | NT | NT | NT |
| T-122 | A | NT | NT | NT |
| T-123 | A | NT | NT | NT |
| T-126 | A | NT | NT | NT |
| T-127 | A | C | C | C |
| T-128 | A | C | C | C |
| T-129 | B | C | C | C |
| T-130 | B | C | C | C |
| T-131 | B | C | C | C |
| T-132 | A | C | C | C |
| T-133 | B | NT | NT | NT |
| T-134 | A | C | C | C |
| T-135 | A | C | C | C |
| T-136 | A | C | C | C |
| T-137 | A | C | C | C |
| T-138 | A | C | C | C |
| T-139 | A | C | C | C |
| T-140 | B | C | C | C |
| T-141 | B | NT | NT | NT |
| T-142 | A | C | C | C |
| T-143 | B | C | C | C |
| T-144 | B | NT | NT | NT |
| T-145 | B | NT | NT | NT |
| T-146 | B | C | C | C |
| T-147 | B | NT | NT | NT |
| T-148 | B | NT | NT | NT |
| T-149 | B | C | C | C |

(continued)

| Compound | CDK7 kinase $IC_{50}$ (nM) | CDK2 kinase $IC_{50}$(nM) | CDK9 kinase $IC_{50}$(nM) | CDK12 kinase $IC_{50}$(nM) |
|---|---|---|---|---|
| T-150 | A | C | C | C |
| T-151 | B | C | C | C |
| T-152 | B | NT | NT | NT |
| T-153 | A | C | C | C |
| T-154 | A | C | C | C |
| T-155 | A | C | C | C |
| T-156 | A | C | C | C |
| T-157 | A | NT | NT | NT |
| T-158 | A | NT | NT | NT |
| T-160 | A | C | C | C |
| T-161 | A | C | C | C |
| T-162 | A | C | C | C |
| T-163 | A | C | C | C |
| T-164 | A | C | C | C |
| T-165 | A | C | C | C |
| T-166 | A | NT | NT | NT |
| T-167 | A | NT | NT | NT |
| T-168 | A | C | C | C |
| T-169 | A | C | C | C |
| T-170 | A | NT | NT | NT |
| T-171 | A | NT | NT | NT |
| T-172 | A | NT | NT | NT |
| T-173 | A | NT | NT | NT |
| T-174 | A | NT | NT | NT |
| T-175 | A | NT | NT | NT |
| Note: NT represents no test | | | | |

From the above table, it can be seen that, the compound synthesized in the present application has a good inhibitory ability on CDK7 kinase, and has a good selectivity through in vitro biological activity screening, using Staurosporine as the reference substance. Therefore, it is expected to be further developed into a drug for regulating CDK7 kinase activity or treating CDK7 related diseases.

**Test Example 2 Cell Anti-proliferation Experiment**

I. Experimental materials and equipment:
Human breast cancer cells MDA-MB 231, MDA-MB453, ovarian cancer cells OVCAR3 and A2780, colorectal cancer cells HCT-116 and WiDr, and lung cancer cell Calu-6 were purchased from BNCC. DMEM medium (Bio-Channel), DMSO (dimethyl sulfoxide), MTT (thiazole blue), 0.25% EDTA-Tripsin (pancreatin digestive juice), 1xPBS (phosphate buffer, PH7.2), 96-well plate (Corning), fetal bovine serum (FBS), 10,000 U/mL penicillin-G/streptomycin, high-speed freezing centrifuge (EPPENDORF 5810R), enzyme-linked immunosorbent assay (Tecan Spark).
Two: Preparation for experiments

1. Cell plating

A) Tumor cells were cultured to 80-90% density in DMEM (high sugar, containing 10% FBS and 100U/mL penicillin-G/streptomycin) at 37°C, 5% CO2 and saturated humidity.
B) The culture medium was removed from the 10cm culture dish.
C) Cells were washed with 10 ml 1xPBS.
D) 4 ml of 0.25% EDTA-Tripsin was added to an incubator to digest pancreatin for 5 minutes at 37°C and 5% CO2, transferred to a 15 ml centrifuge tube, centrifuged for 5 minutes at 200g, and supernatant was discarded to obtain cell precipitation.
E) The cell precipitation was resuspended with 4 ml DMEM medium, counted and adjusted to 50,000 cells/ml.
F) Cell suspension was added to a 96-well plate with a volume of 100 μL per well and cultured overnight in a 5% CO2 incubator at 37°C.

2. Compound Treatment

Compound dilution

A) Preparing the tested compound gradient dilution solution: the tested compounds were prepared into 10mM and 1mM storage solution respectively. Firstly, 1μl and 0.5 μl of 10mM storage solution were dissolved in 1ml of DMSO-free culture solution, respectively, to obtain diluted compounds with concentrations of 10 and 5 μM. Then, 1.5 μl of 1mM storage solution was dissolved in 1.5ml of DMSO-free culture solution to obtain a dilution compound with a concentration of 1 μM, and then diluted with a 3-fold continuous gradient with 0.025% DMSO culture solution for a total of 7 concentrations. The concentrations of all 10 diluted compounds mentioned above were as follows:
10 μM, 5 μM, 1 μM, 333.33 nM, 111.11 nM, 37.03 nM, 12.35 nM, 4.15 nM, 1.37 nM, 0.46 nM
B) After full mixing, 100 μL of culture compound solution was taken to replace the culture solution in the cell culture plate, with 4 duplicated wells at each concentration;
C) The cells were transferred to incubator to incubate for 3 days.

3. MTT detection

A) The cell culture plate was taken out and added with 5 mg/ml MTT 10 μL in the biosafety cabinet;
B) The cell culture plate was put back into the incubator and continued to incubate for 3 hours;
C) The cell culture plate was taken out to remove the culture solution, 100 μL isopropanol (containing 0.4 mM HCl, 0.1% NP-40) was added, shaking at room temperature for 30 minutes;
D) The absorbance value was measured at 570nm on TECAN enzyme linked immunosorbent assay.

4. Data Analysis

The following formula was used to calculate the viability (% Cell Viability) :

$$\%\text{Cell Viability}=100\%\times(\text{Lum_Sample}-\text{Lum_LC})/(\text{Lum_HC}-\text{Lum_LC})$$

Lum_HC: Cell readings for 0.1%DMSO control group
Lum_Sample: Cell readings for sample added with compounds
Lum_LC: Readings for blank medium

IC50 value was obtained by curve fitting using GraphPad Prism 8 software. As shown in Table 2.

Table 2

| Compound | MDA-MB 231 (nM) | MDA-MB453 (nM) | OVCAR3 (nM) | A2780 (nM) | HCT-116 (nM) | WiDr (nM) | Calu-6 (nM) |
|---|---|---|---|---|---|---|---|
| T-01 | 144.9 | 51.87 | 41.79 | 7.97 | 62.45 | 12.21 | 68.89 |
| T-17 | NT | NT | NT | 4.76 | NT | 26.77 | NT |
| T-34 | 573 | 120.2 | 215 | 8.73 | NT | NT | NT |
| T-36 | 470.8 | 115.9 | 197.4 | NT | NT | NT | 208.8 |
| T-37 | 52.67 | 10.84 | 25.15 | 7.65 | 47.81 | 13.76 | 40.09 |
| T-42 | 181.1 | 77.88 | 98.37 | 12.31 | 136.8 | 22.22 | 109.8 |
| T-52 | 119.3 | 17.73 | 51.06 | 16.15 | 89.18 | 28.34 | 106.8 |
| T-62 | 75.5 | 23.76 | 38.76 | 12.5 | 55.43 | 6.35 | 57.01 |
| T-70 | NT | NT | NT | 4.47 | NT | 45.49 | NT |
| T-79 | 144.1 | 53.14 | 69.77 | 14.18 | 35.3 | 16.34 | 113.8 |
| T-81 | NT | NT | 55.5 | 1.26 | NT | NT | NT |
| T-91 | NT | NT | 80.2 | 8.75 | NT | NT | NT |
| T-98 | NT | NT | 127.0 | 5.55 | NT | NT | NT |
| T-99 | NT | NT | 53.4 | 1.54 | NT | NT | NT |
| T-122 | NT | NT | NT | 0.54 | NT | NT | NT |
| T-128 | NT | NT | 9.08 | 8.68 | NT | NT | NT |
| T-132 | NT | NT | 100.3 | 4.52 | NT | NT | NT |
| T-135 | NT | NT | 161.4 | 4.61 | NT | NT | NT |
| T-142 | NT | NT | 120.1 | 5.05 | NT | NT | NT |
| T-150 | NT | NT | 160.7 | 13.5 | NT | NT | NT |
| T-156 | NT | NT | NT | 1.23 | NT | NT | NT |
| Compound 101 | 1662 | 112.5 | 245.8 | 8.76 | 88.8 | 11.17 | 207.4 |
| Note: NT represents no test | | | | | | | |

As can be seen from Table 2, the compound of the present invention has a very good inhibitory effect on human breast cancer cells MDA-MB 231, MDA-MB453, ovarian cancer cells OVCAR3 and A2780, colorectal cancer cells HCT-116 and WiDr, and lung cancer cell Calu-6, and has better activity than the best compound 101 in WO2020093006A1.

**Test Example 3 Pharmacokinetic Experiment of Compound**

The head-to-head pharmacokinetics comparison between Compound T-01 of the invention and the control compound (compound 101 in WO2020093006A1) for oral administration was conducted.

Experimental protocol of rat pharmacokinetics

(1) Required animals: healthy adult SD rats, male, 3 rats, 6-8 weeks old; Weight 200-300g.
(2) Required equipment: analytical balance, animal weight scale, magnetic stirrer, frozen centrifuge, single-channel manual pipette, etc.
(3) Required reagents: EDTA-Na2 anticoagulant, etc. 11.2g of EDTA-Na2 was weighed and placed in a reagent flask, 100 mL of normal saline was added, and shaken to completely dissolve it. After the preparation was completed, it was divided into 1.5 mL centrifuge tubes with 20 uL each for whole blood sample collection.

(3) About 10 mg of the sample to be tested was accurately weighed, 5% DMSO after conversion was added to dissolve, then 30% PEG400 and 65% (10% Hp-β-CD in PBS) were added for ultrasound, vortex-mixed evenly to obtain a solution with a concentration of 1 mg/mL. Fresh preparation before use.

(4) 0.1mL of sample was absorbed into 1.5 mL centrifuge tube and stored at -80°C for concentration analysis of drug administration solution.

(5) Animals were kept in rat cages, and fasting (not less than 10 h) was started the day before the test, but water was allowed. The animals were separately weighed and marked at the tail on the day of the test. Blank blood was collected before administration. The blood was collected from tail vein.

(6) Route of administration: gavage (p.o.); dosage: 10 mg/kg; administration volume: 10mL/kg.

(7) Operation process: The rat was caught in the left hand with anti-bite gloves, held upright, the 16th gastric lavage needle was inserted through the throat of the mouth, tested to feel the needle without obvious resistance, and then the drug was injected into the stomach.

(8) 0.2 ml of whole blood was collected from the tested animals and added into EDTA-Na2 anticoagulant tubes before administration and at 0.5 h, 1 h, 2h, 4 h, 6 h, 8h, 12h and 24 h after administration respectively, reversed up and down for 3 -4 times and mixed evenly, centrifuged at 4 °C at 10000g for 5 min to separate plasma, and stored at -80°C for testing. The blood was collected from tail vein.

(9) LC-MS/MS method was established to determine the original drug concentration in plasma, the blood drug concentration-time curve was drawn, and the main pharmacokinetic parameters were calculated by non-compartmental model.

(10) The pharmacokinetic parameters of compound T-01 and the reference compound were specifically shown in FIG. 1, wherein, the ordinate was the concentration of the drug in plasma.

As can be seen from Figure 1, the compound of the present invention has better pharmacokinetic parameters than the reference compound (compound 101), both in terms of plasma concentration Cmax and AUC, it is about 5 times better than that of the reference compound.

**Preparation Example 1**

The compound synthesized by the invention:

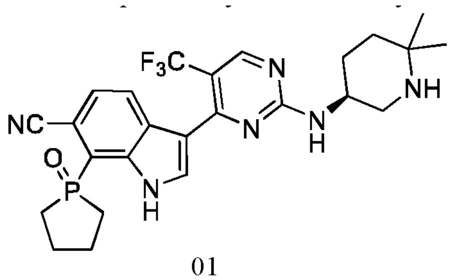

01

The experimental process was as follows:

I. Synthesis of Intermediate SM1

The synthetic route is as follows:

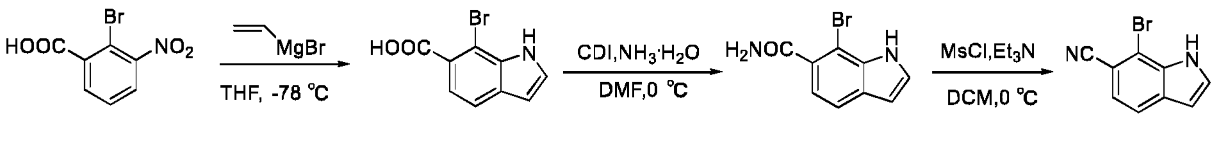


1. Synthesis of Compound 2

730 mL(6 eq) vinyl magnesium bromide was added to a 2 L three-necked flask under nitrogen condition, liquid nitrogen-ethanol was used to cool to -78 °C, 30g compound 1 was dissolved in 700 mL anhydrous tetrahydrofuran, and added dropwise to the reaction flask slowly under stirring, the temperature was kept not higher than -70 °C, slowly heated to room temperature after dropwise addition to stir overnight. TLC showed that there was no raw

materials, and the target product point was the main point. Ammonium chloride aqueous solution was added to quench, pH was adjusted to be 2 with diluted hydrochloric acid, extracted with ethyl acetate, dried over anhydrous sodium sulfate, evaporated under reduced pressure until a large amount of solid was precipitated, and suction filtered under reduced pressure to obtain 16g of compound 2 as a filter cake. LC-MS[M +1]:240, 242.

2. Synthesis of Compound 3

8g of compound 2 was dissolved in 15 mL DMF in a 100 mL three-necked flask, the temperature was lowered to 0 °C in an ice bath, 11g(2 eq) CDI was slowly added under stirring to react under stirring for 1 h. 50 mL of ammonia (10 eq) was slowly added in the ice bath, and TLC monitored until the reaction was completed. Water was added, extracted with ethyl acetate, dried over anhydrous sodium sulfate, spin-dried, purified by column chromatography to obtain 6g of compound 3. LC-MS[M +1]:239, 241.

3. Synthesis of Intermediate SM1

6g of compound 3 and 25.5g(10 eq) of triethylamine were dissolved in DCM in a 100 mL three-necked flask, cooled to 0 °C in ice bath, 23g(8 eq) methanesulfonyl chloride was added under stirring to react overnight at room temperature, TLC monitored until the reaction was completed. Extracted with ethyl acetate, dried over anhydrous sodium sulfate, spin-dried, separated by column chromatography to obtain 3.3g of compound 4. LC-MS[M +1]:221, 223.

II. Synthesis of Compound 01

The synthetic route is as follows:

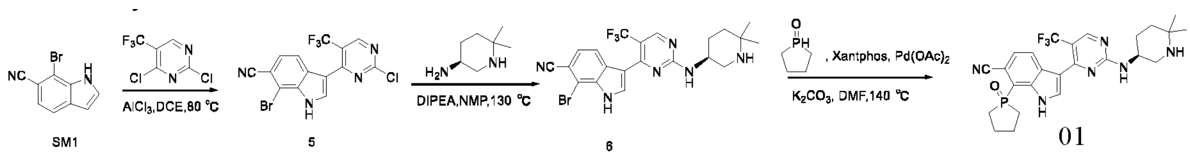


SM1 5 6 01

1. Synthesis of Compound 5

9g(3 eq) 2,4-dichloro-5 trifluoromethylpyrimidine was dissolved in 150 mL DCE in a 100 mL round bottom flask, 3g(1.5 eq) anhydrous aluminum trichloride was added under stirring, the temperature was raised to 80 °C under nitrogen protection, and stirred for half an hour. Cooled to room temperature, 3g compound SM1 was added, the temperature was raised to 80 °C, and TLC monitored until the reaction was completed. The reaction product had two positional isomers, and the content ratio thereof was close to 3:1. Water was added, extracted with ethyl acetate, evaporated under reduced pressure until a large amount of solid was precipitated, suction filtered under reduced pressure to obtain 1.2g of filtrate cake of compound 5 with a single configuration, and the filtrate was separated by column chromatography to obtain 1.6g of compound 5 being a mixture of two configurations. LC-MS[M +1]:401, 403.

2. Synthesis of Compound 6

1.2g of compound 5, 1g(1.2 eq) (S)-6, 6-dimethylpiperidine -3-amine, 1.5g(4 eq) DIPEA and 6 mL N-methylpyrrolidone were added to a 100 mL round bottom flask, stirred and heated to 130 °C to react for 3 h under nitrogen protection, and TLC monitored until the reaction was completed. Water was added, extracted with ethyl acetate, and the organic phase was dried over anhydrous sodium sulfate, separated by column chromatography to obtain 1.2g of compound 6. LC-MS[M +1]:493, 495.

3. Synthesis of Compound 01

0.1g compound 6, 0.02g(1.2 eq) dimethylphosphine oxide, 0.03g(0.2 eq) Xantphos, 0.09g(2 eq) tripotassium phosphate, 0.005g(0.1 eq) palladium acetate, and 1 mL DMF were added into a 100mL three-necked flask, heated to 140 °C under nitrogen protection, and TLC monitored until the reaction was completed. Water and ethyl acetate were added for extraction, and the organic phase was dried over anhydrous sodium sulfate, separated by column chromatography to obtain 5 mg of compound 01. LC-MS[M+1]:517.

The following compounds were synthesized by reference to method I or method II in Preparation Example 1:

| Compound number | Compound structure | Characterization data |
|---|---|---|
| 02 | 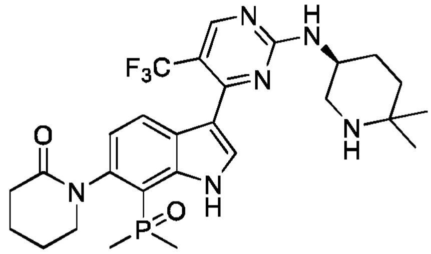 | LC-MS[M+1]:563 |
| 03 | 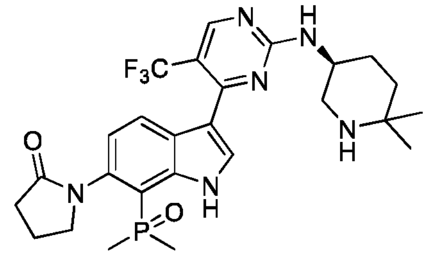 | LC-MS[M+1]:549 |
| 04 | 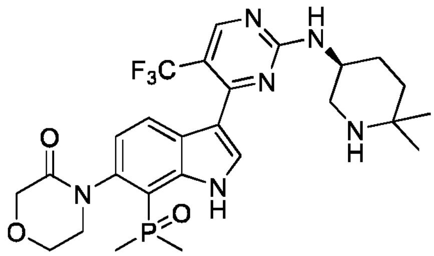 | LC-MS[M+1]:565 |
| 08 | 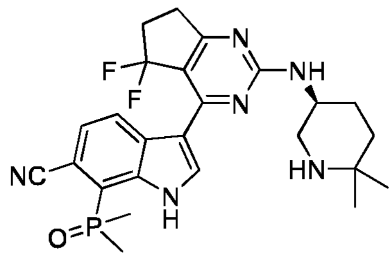 | LC-MS[M+1]:499 |
| 09 | 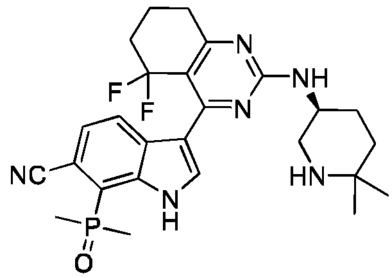 | LC-MS[M+1]:513 |
| 10 | 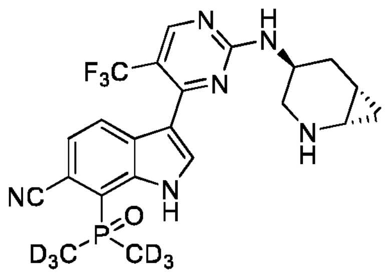 | LC-MS[M+1]:481 |

(continued)

| Compound number | Compound structure | Characterization data |
|---|---|---|
| 11 | 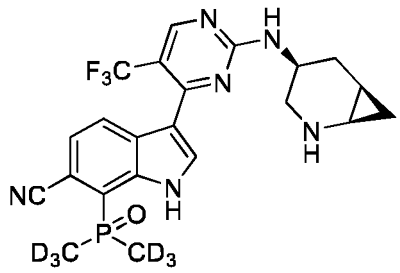 | LC-MS[M+1]:481 |
| 12 | 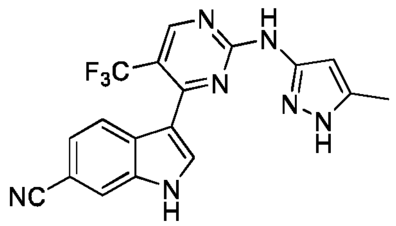 | LC-MS[M+1]:384 |
| 13 | 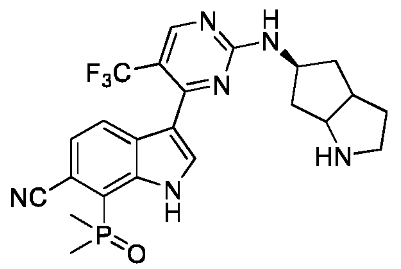 | LC-MS[M+1]:489 |
| 14 | 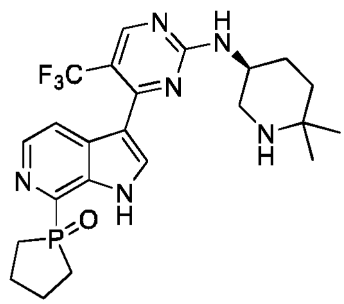 | LC-MS[M+1]:493 |
| 18 | 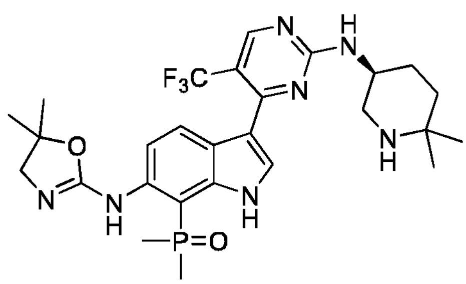 | LC-MS[M+1]:578 |

(continued)

| Compound number | Compound structure | Characterization data |
|---|---|---|
| 20 | 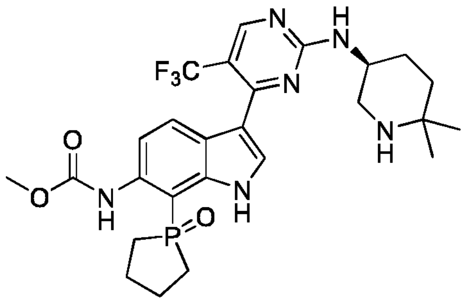 | LC-MS[M+1]:565 |
| 23 | 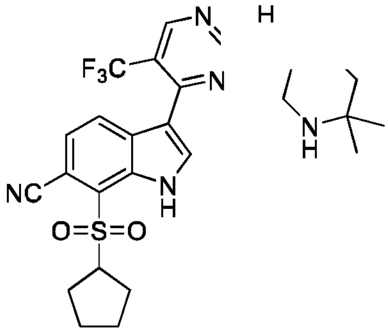 | LC-MS[M+1]:547 |
| 24 | 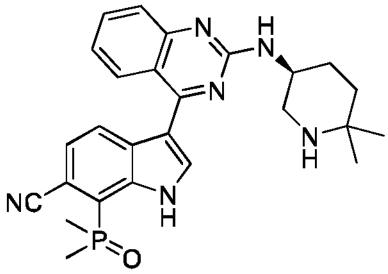 | LC-MS[M+1]:473 |
| 28 | 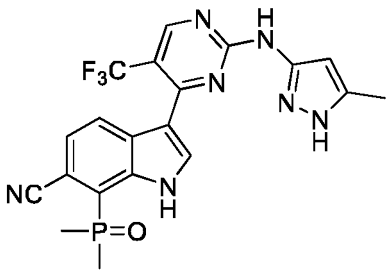 | LC-MS[M+1]:460 |
| 31 | 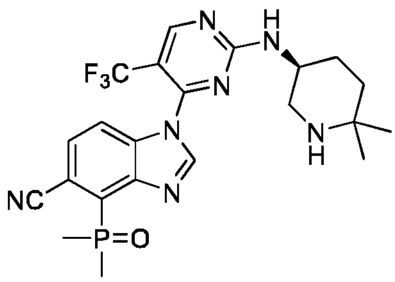 | LC-MS[M+1]:492 |

(continued)

| Compound number | Compound structure | Characterization data |
|---|---|---|
| 32 | 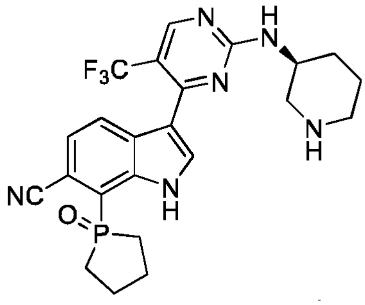 | LC-MS[M+1]:489 |
| 36 | 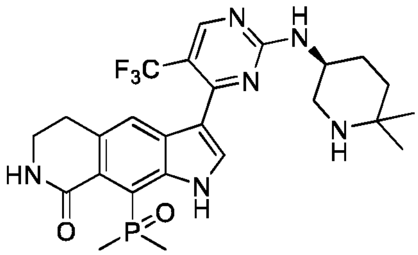 | LC-MS[M+1]:535 |
| 38 | 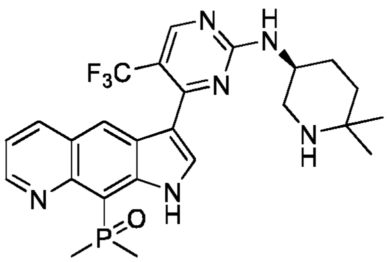 | LC-MS[M+1]:517 |
| 48 | 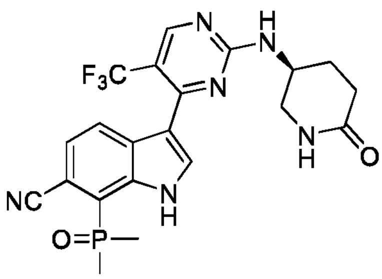 | LC-MS[M+1]:477 |
| 65 | 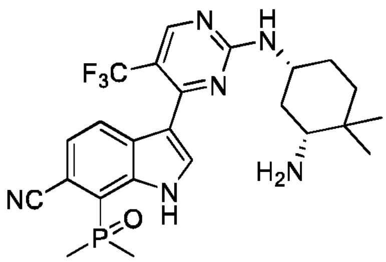 | LC-MS[M+1]:505 |

(continued)

| Compound number | Compound structure | Characterization data |
|---|---|---|
| 66 | 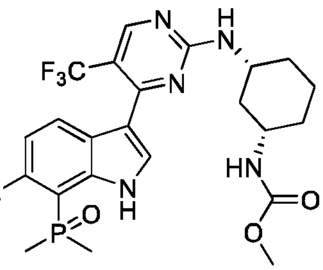 | LC-MS[M+1]:535 |

**Test Example 1 Enzyme Activity Test**

The biological activity test of some of the compounds above and the comparative compound was carried out.

The biological activity test experiment process was as follows:

$IC_{50}$ values of the compounds for CDK7 kinase were detected in Wuxi Biortus Biosciences Co. Ltd..

1. Compound preparation:

The compound powder was dissolved in 100% DMSO to prepare a 10mM storage solution. The storage solution was further diluted to 0.5mM as the initial concentration, and continuously diluted 3 times to obtain 10 compound solutions with different concentrations. The compound and enzyme were pre-incubated for 0 minutes and 60 minutes, respectively, using duplicate detection. The Staurosporine compound having a structural formula of

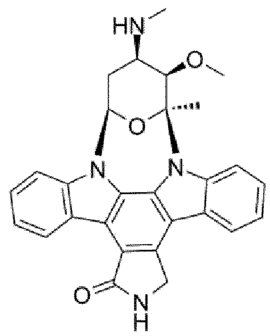

was used as a positive control, using Mobility shift say method, to detect CDK kinase (such as CDK7, CDK9, CDK12 and CDK2 ) activity of the compound .

2. Kinase reaction process:

1) The compound solution and the positive control were diluted 8.3 times with double distilled water and then added to the 384 well plate, each concentration was 2uL/well;

(2) 6nM CDK7/Cyclin H/MAT1 kinase solution was added to the compound well and the positive control well respectively;

(3) incubating at room temperature for 0 and 60 minutes;

(4) 2mM ATP and 2pM 5-FAM-CDK7 peptide substrate (5-FAM-YSPTSPSYSPTSPSYSPTSPSKKKK) solution (8 nM CDK9/Cyclin TI polymer and 2 pM 5-FAM GSRTPMY-NH2 peptide substrate were used in CDK9 inhibition reaction; 50 nM CDK12 (aa686-1082)/Cyclin K polymer and 2 pM 5-FAM GSRTPMY-NH2 peptide substrate were used in CDK12 inhibition reaction; 0.5 nM CDK2/Cyclin EI polymer and 2 pM 5-FAM-YSPTSPSYSPTSPSYSPTSPSKKKK peptide substrate were used in CDK2 inhibition reaction) were added;

(5) Incubating the 384 well plate at 25°C for 30 minutes;

(6) 4uL 120 mM EDTA was added to stop the kinase reaction;

(7) The conversion rate was read with Caliper/LabChip EZ Reader (Perkin Elmer);

(8) $IC_{50}$ value was obtained by curve fitting using GraphPad Prism 8 software.

Through the above detection, $IC_{50}$ (nM) value of the test samples for the inhibitory activity of CDK7 kinase was obtained and showed in Table 3, wherein A≤ 10nM; 10nM<B<500 nM; C≥ 500nM.

Table 3

| Compound | CDK7 kinase $IC_{50}$ (nM) |
|---|---|
| Staurosporine | A |
| 01 | A |
| 02 | A |
| 12 | B |
| 14 | A |
| 20 | A |
| 23 | A |
| 24 | B |
| 28 | A |
| 31 | A |
| 48 | A |
| 66 | A |

From the above table, it can be seen that the compound synthesized in the present application has a good inhibitory ability on CDK7 kinase through in vitro biological activity screening, using Staurosporine as the reference substance. Therefore, it is expected to be further developed into a drug for regulating CDK7 kinase activity or treating CDK7 related diseases.

**Test Example 2 Cell Anti-proliferation Experiment**

I. Experimental materials and equipment:
Human breast cancer cells MDA-MB 231, MDA-MB453, ovarian cancer cells OVCAR3, colorectal cancer cells HCT-116 and lung cancer cells NCI-H209 were purchased from BNCC. DMEM medium (Bio-Channel), DMSO (dimethyl sulfoxide), MTT (thiazole blue), 0.25% EDTA-Tripsin (pancreatin digestive juice), 1xPBS (phosphate buffer, PH7.2), 96-well plate (Corning), fetal bovine serum (FBS), 10,000 U/mL penicillin-G/streptomycin, high-speed freezing centrifuge (EPPENDORF 5810R), enzyme-linked immunosorbent assay (Tecan Spark).
Two: Preparation for experiments

1. Cell plating

A) Tumor cells were cultured to 80-90% density in DMEM (high sugar, containing 10% FBS and 100U/mL penicillin-G/streptomycin) at 37°C, 5% CO2 and saturated humidity.
B) The culture medium was removed from the 10cm culture dish.
C) Cells were washed with 10 ml 1xPBS.
D) 4 ml of 0.25% EDTA-Tripsin was added to an incubator to digest pancreatin for 5 minutes at 37°C and 5% CO2, transferred to a 15 ml centrifuge tube, centrifuged for 5 minutes at 200g, and supernatant was discarded to obtain cell precipitation.
E) The cell precipitation was resuspended with 4 ml DMEM medium, counted and adjusted to 50,000 cells/ml.
F) Cell suspension was added to a 96-well plate with a volume of 100 μL per well and cultured overnight in a 5% CO2 incubator at 37°C.

2. Compound Treatment

Compound dilution

A) Preparing the tested compound gradient dilution solution: TY 2600 1 mM, TY 2601 1 mM, TY-2648a 1 mM, TY-2648b 1 mM and TY-2650 1 mM storage solutions were prepared. Then 1.5 μl of storage solution was dissolved in 1.5ml of DMSO-free culture solution, and then performed 3-fold continuous gradient dilution with 0.1% DMSO culture solution for a total of 9 concentrations. The concentrations of the diluted compound were as follows:
333.33 nM, 111.11 nM, 37.03 nM, 12.35 nM, 4.15 nM, 1.37 nM, 0.46 nM, 0.15 nM
B) After full mixing, 100 μL of culture compound solution was taken to replace the culture solution in the cell culture plate, with 4 duplicated wells at each concentration;
C) The cells were transferred to incubator to incubate for 3 days.

3. MTT detection

A) The cell culture plate was taken out and added with 5 mg/ml MTT 10 μL in the biosafety cabinet;
B) The cell culture plate was put back into the incubator and continued to incubate for 3 hours;
C) The cell culture plate was taken out to remove the culture solution, 100 μL isopropanol (containing 0.4 mM HCl, 0.1% NP-40) was added, shaking at room temperature for 30 minutes;
D) The absorbance value was measured at 570nm on TECAN enzyme linked immunosorbent assay.

4. Data Analysis

The following formula was used to calculate the viability (% Cell Viability) :

$$\%\text{Cell Viability}=100\%\times(\text{Lum_Sample- Lum_LC })/(\text{Lum_HC}-\text{Lum_LC})$$

Lum_HC: Cell readings for 0.1% DMSO control group
Lum_Sample: Cell readings for sample added with compounds
Lum_LC: Readings Blank medium

IC50 value was obtained by curve fitting using GraphPad Prism 8 software. As shown in Table 4, wherein A ≤ 10nM; 10nM<B<500 nM; C≥ 500nM.

Table 4

| Compound | MDA-MB 231(nM) | MDA-MB453(nM) | OVCAR3(nM) | HCT-116(nM) |
|---|---|---|---|---|
| 01 | B | B | B | B |
| 14 | B | B | B | B |
| 20 | B | B | B | B |
| 31 | B | B | B | B |
| 48 | B | B | B | B |

As can be seen from Table 4, the compound of the present invention has a very good inhibitory effect on human breast cancer cells MDA-MB 231, MDA-MB453, ovarian cancer cell OVCAR3, and colorectal cancer cell HCT-116.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

**Claims**

**1.** A compound used as a CDK7 kinase inhibitor, wherein the compound is a compound of formula I, or its pharmaceutically acceptable salt, stereoisomer, tautomer, hydrate, solvate, isotope compound or prodrug,

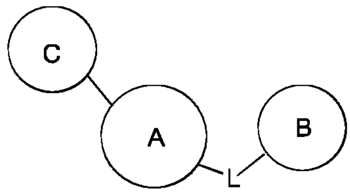

I

wherein:

ring A is

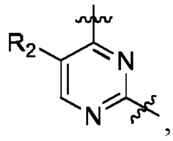

,

wherein $R_2$ is selected from the group consisting of halogen, C1-C6 alkyl and C1-C6 haloalkyl;

ring B is selected from the substituted or unsubstituted group consisting of 4-7 membered heterocyclyl containing 1-3 groups selected from N, O, S, S(O) or $S(O)_2$, 5-9 membered bridged heterocycloalkyl containing 1-3 groups selected from N, O, S, S(O) or $S(O)_2$, 5-9 membered bridged cycloalkyl, 6-10 membered spiro heterocycloalkyl containing 1-3 groups selected from N, O, S, S(O) or $S(O)_2$, 6-10 membered spiro cycloalkyl, 6-10 membered fused heterocycloalkyl containing 1-3 groups selected from N, O, S, S(O) or $S(O)_2$, 6-10 membered fused cycloalkyl, 5-6 membered heteroaryl containing 1-3 groups selected from N, O, S, S(O) or $S(O)_2$ and C6-C10 aryl;

L is selected from the group consisting of O, $NR_1$ and $-NR_1$-(C1-C6 alkylene)-;

ring C is

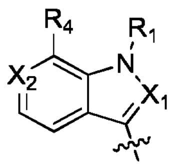

; wherein,

$X_1$ is selected from the group consisting of N and CH;

$X_2$ is selected from the group consisting of N and $CR_2$';

$R_2$' is independently selected from the substituted or unsubstituted group consisting of hydrogen, amino, $-NR_1$-C(=O)-O-(C1-C6 alkyl), $-NR_1$-C(=O)-$R_5$, $-NR_1$-C(=O)-O-C1-C6 alkylene-Xs-C1-C6 alkyl, -O-C(=O)-$NR_1R_5$,

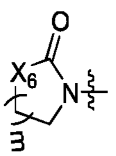

,

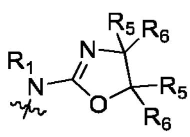

,

and hydroxyl;

$R_4$ is selected from the substituted or unsubstituted group consisting of H, halogen, C1-C6 alkyl, C1-C6 haloalkyl, C2-C6 alkenyl, C2-C6 alkynyl, C1-C6 alkoxy, C1-C6 haloalkoxy, C1-C6 heteroalkyl, C3-C6 cycloalkyl, C3-C6 cycloalkoxy, C3-C6 halocycloalkyl, C6-C10 aryl, 5-10 membered heteroaryl containing 1, 2 or 3 heteroatoms

selected from N, O or S, 3-8 membered heterocycloalkyl containing 1 2 or 3 heteroatoms selected from N, O or S,

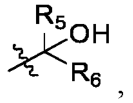,

$-NR_1-C(=O)-O-(C1-C6\ alkyl)$, -O-C(=O)-Rs, $-C(=O)-R_5$,

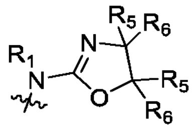,

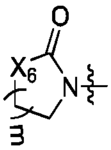, 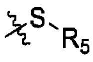, 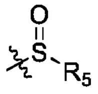, 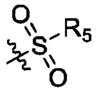, 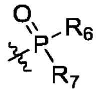, 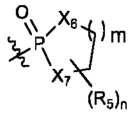,

and cyano;

$R_1$ is independently selected from the substituted or unsubstituted group consisting of hydrogen, amino, -C(=O)-O-(C1-C6 alkyl), C1-C6 alkyl, C1-C6 haloalkyl, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1, 2 or 3 heteroatoms selected from N, O or S, and $-C(=O)-NR_8R_9$;

each $R_5$, $R_6$ and $R_7$ is independently selected from the group consisting of hydrogen, C1-C6 alkyl, C1-C6 haloalkyl, C1-C6 alkyl containing 1, 2 or 3 heteroatoms selected from N, O or S, C3-C6 cycloalkyl, C3-C6 halocycloalkyl, C1-C6 alkylamino, C3-C6 cycloalkylamino, 3-8 membered heterocycloalkyl containing 1, 2 or 3 heteroatoms selected from N, O or S, C6-C10 aryl, 5-10 membered heteroaryl containing 1, 2 or 3 heteroatoms selected from N, O or S, and $NR_8R_9$;

each $R_8$ and $R_9$ is independently selected from the group consisting of hydrogen and C1-C6 alkyl;

each $X_6$ and $X_7$ is independently selected from the group consisting of O, $CR_5R_6$ and $NR_1$;

each $X_8$ is independently selected from the group consisting of N, O and S;

m is selected from the group consisting of 0, 1, 2 and 3;

each n is independently selected from the group consisting of 0, 1, 2, 3, 4 and 5;

the substituted independently means to be substituted by a group selected from the group consisting of -NH-C(=O)-O-(C1-C6 alkyl), amino, halogenated or unsubstituted C1-C6 alkyl, =O, hydroxyl, and -NH(C1-C6 alkyl).

**2.** The compound of claim 1, wherein the compound is a compound of formula II, or its pharmaceutically acceptable salt, stereoisomer, tautomer, hydrate, solvate, isotope compound or prodrug,

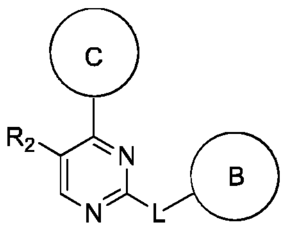 II

$R_2$ is selected from trifluoromethyl, chlorine, bromine, methyl, ethyl or isopropyl;

ring B is selected from the substituted or unsubstituted group consisting of 4-7 membered heterocyclyl containing 1-3 groups selected from N, O, S, S(O) or $S(O)_2$, 5-9 membered bridged heterocycloalkyl containing 1-3 groups selected from N, O, S, S(O) or $S(O)_2$, 5-9 membered bridged cycloalkyl, 6-10 membered spiro heterocycloalkyl containing 1-3 groups selected from N, O, S, S(O) or $S(O)_2$, 6-10 membered spiro cycloalkyl, 6-10 membered fused heterocycloalkyl containing 1-3 groups selected from N, O, S, S(O) or $S(O)_2$, 6-10 membered fused

cycloalkyl, 5-6 membered heteroaryl containing 1-3 groups selected from N, O, S, S(O) or S(O)z and 5-6 membered aryl;
L is selected from the group consisting of O, $NR_1$ and $-NR_1$-(C1-C6 alkylene)-;
ring C is

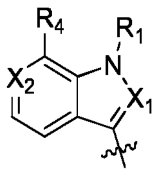;

wherein, $R_1$, $R_4$, $X_1$, and $X_2$ are as defined in claim 1.

**3.** The compound of claim 1, wherein,

ring B is selected from the group consisting of

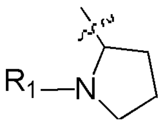, 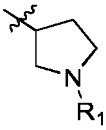, 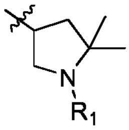,

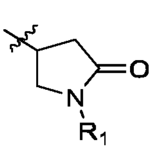, 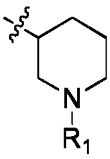, 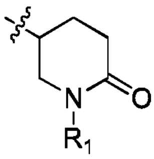, 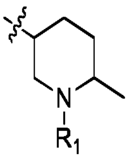, 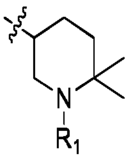, 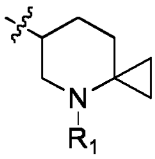, 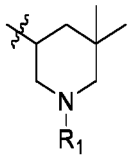,

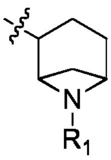, 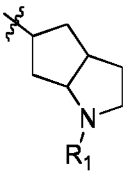, 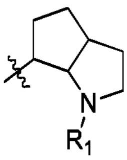, 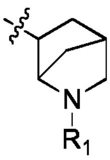, 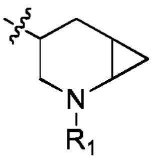, 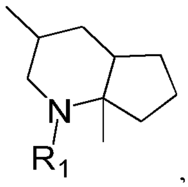, 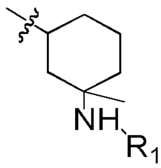,

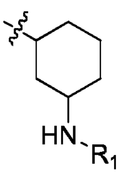, 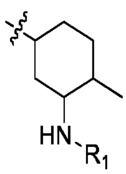, 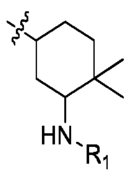, 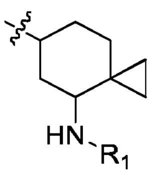, 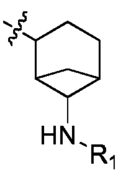, 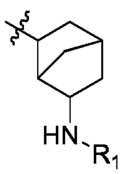, 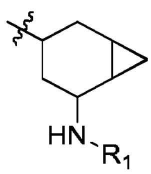, 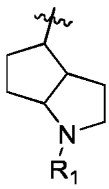,

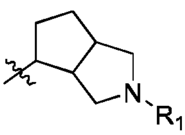, 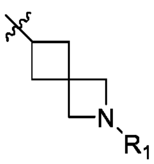, 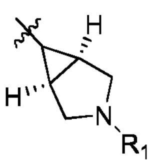, 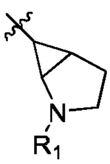, 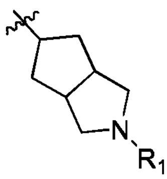, 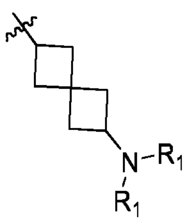,

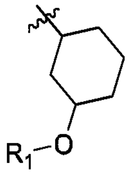, 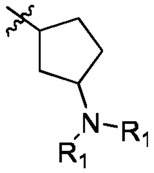, and 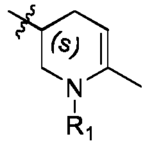;

ring C is selected from the group consisting of

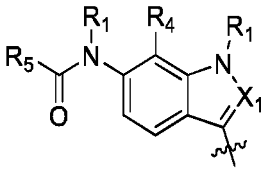,

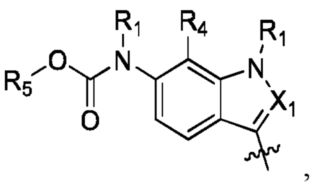, 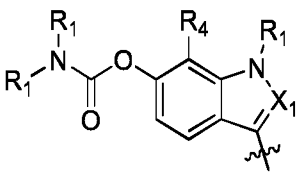, 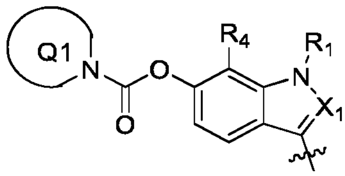,

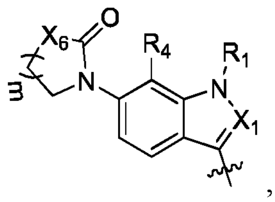, and 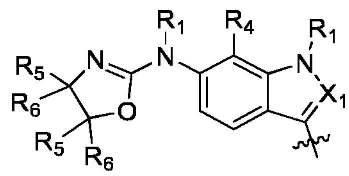;

wherein, $R_1$, $R_4$, $R_5$, $R_6$, $X_1$, $X_2$, $X_6$, and m are as defined in claim 1, and $Q_1$ is a 3-7 membered ring.

**4.** The compound of claim 1, wherein,

ring A is selected from the group consisting of

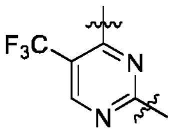, 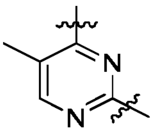, 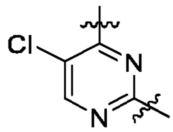,

and

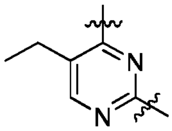;

ring C is selected from the group consisting of

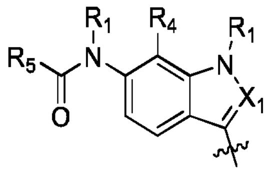,

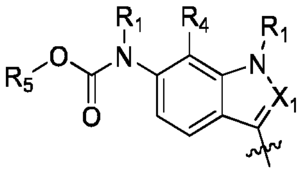, 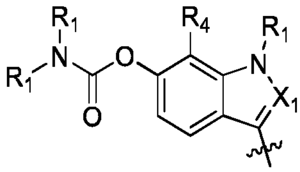, 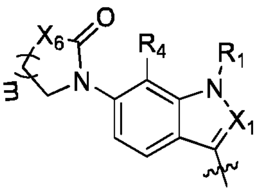;

and

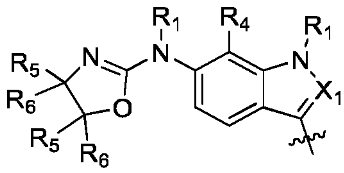;

R4is selected from the group consisting of H, halogen, C1-C6 alkyl, C1-C6 haloalkyl, C2-C6 alkenyl, C1-C6 alkoxy, C1-C6 haloalkoxy, C1-C6 heteroalkyl, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1, 2 or 3 heteroatoms selected from N, O or S,

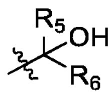, 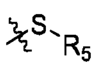, 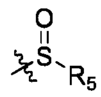, 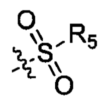, 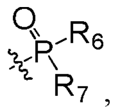,

and

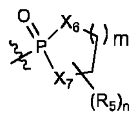;

wherein, $R_1$, $R_5$, $R_6$, $R_7$, $X_1$, $X_6$, $X_7$, m, and n are as defined in claim 1.

**5.** The compound of claim 1, wherein,

ring A is selected from the group consisting of

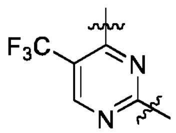, 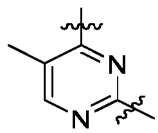,

and

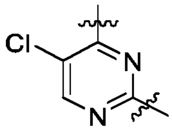;

ring C is selected from the group consisting of

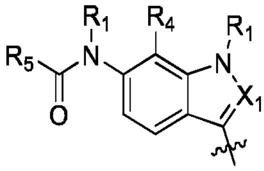,

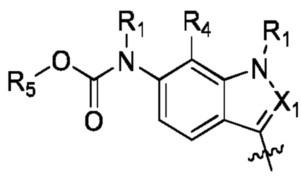, 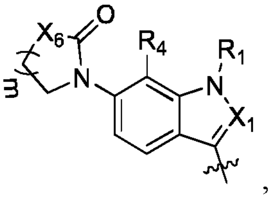, and 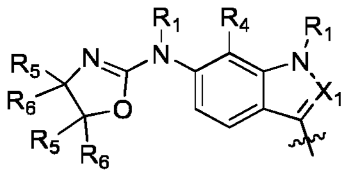;

$R_4$ is selected from the group consisting of H, halogen, C1-C6 alkyl, C1-C6 haloalkyl, C2-C6 alkenyl, C1-C6 alkoxy, C1-C6 haloalkoxy, C3-C6 cycloalkyl, C3-C6 halocycloalkyl, C6-C10 aryl, 5-10 membered heteroaryl containing 1, 2 or 3 heteroatoms selected from N, O or S, 3-8 membered heterocycloalkyl containing 1, 2 or 3 heteroatoms selected from N, O or S, 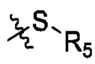, 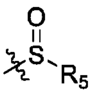, 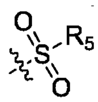, and 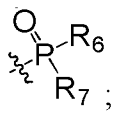;

L is selected from the group consisting of O and $NR_1$;
wherein, $R_1$, $R_5$, $R_6$, $R_7$, $X_1$, $X_6$, and m are as defined in claim 1.

**6.** The compound of claim 5, wherein $R_4$ is selected from the group consisting of H, halogen, C1-C6 alkyl, C1-C6 haloalkyl, C2-C6 alkenyl, C1-C6 alkoxy, C1-C6 haloalkoxy, C3-C6 cycloalkyl, C3-C6 halocycloalkyl, C6-C10 aryl, 5-10 membered heteroaryl containing 1, 2 or 3 heteroatoms selected from N, O or S, and 3-8 membered heterocycloalkyl containing 1, 2 or 3 heteroatoms selected from N, O or S;
L is NH.

**7.** The compound of claim 1, wherein, the compound is selected from the group consisting of

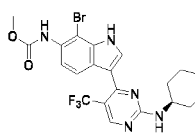
T-01

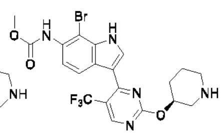
T-02

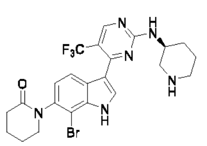
T-03

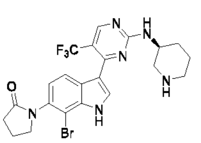
T-04

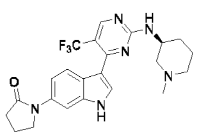
T-05

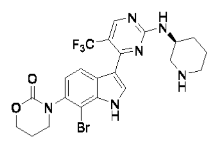
T-06

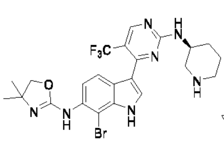
T-07

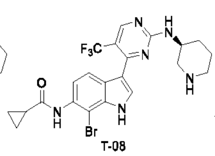
T-08

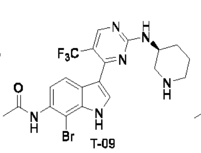
T-09

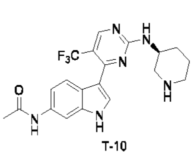
T-10

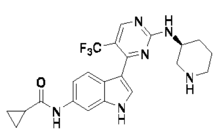
T-11

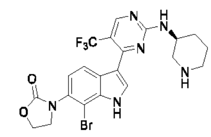
T-12

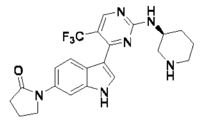
T-13

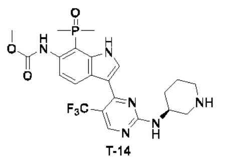
T-14

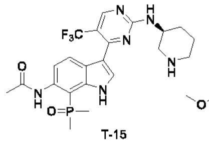
T-15

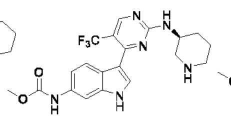
T-16

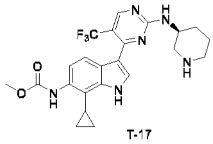
T-17

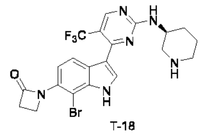
T-18

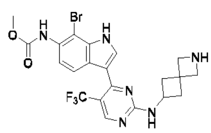
T-19

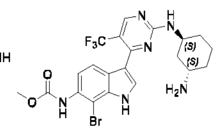
T-20

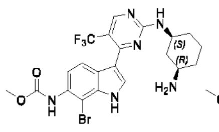
T-21

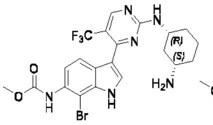
T-22

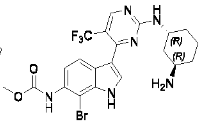
T-23

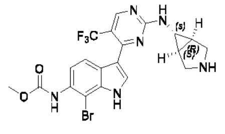
T-24

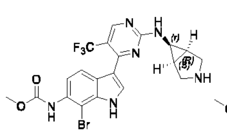
T-25

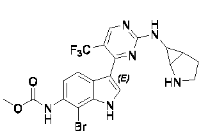
T-26

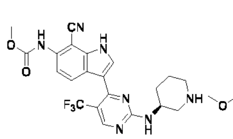
T-27

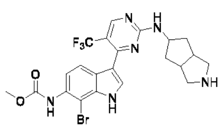
T-28

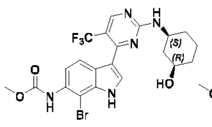
T-29

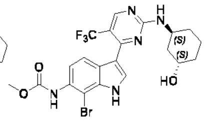
T-30

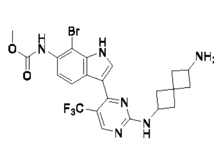
T-31

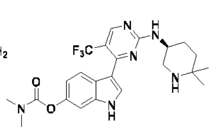
T-32

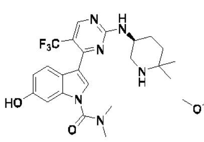
T-33

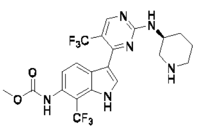
T-34

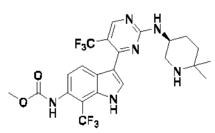
T-35

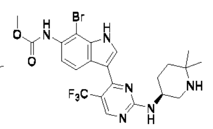
T-36

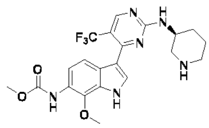
T-37

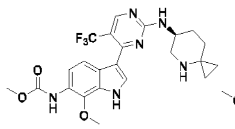
T-38

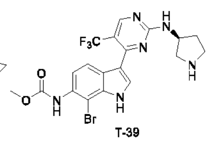
T-39

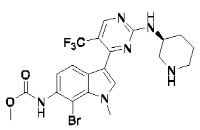
T-40

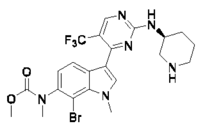
T-41

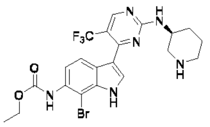
T-42

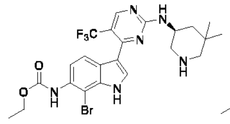
T-43

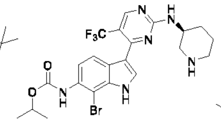
T-44

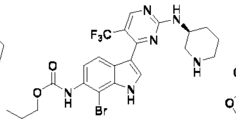
T-45

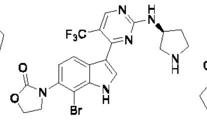
T-46

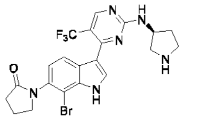
T-47

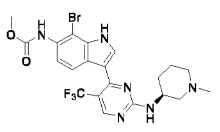
T-48

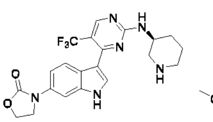
T-49

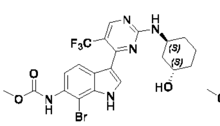
T-50

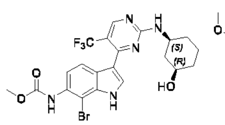
T-51

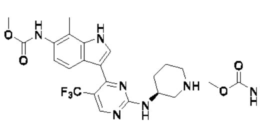
T-52

T-53

T-54

(cis)
T-55

T-56

T-57

T-58

T-59

T-60

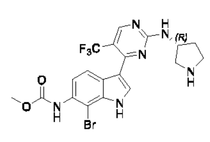
T-61

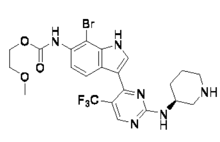
T-62

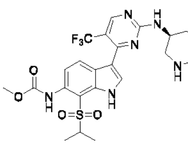
T-63

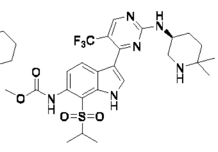
T-64

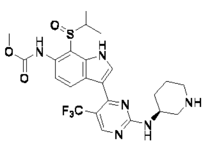
T-65

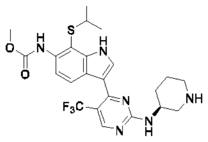
T-66

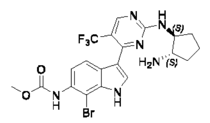
T-67

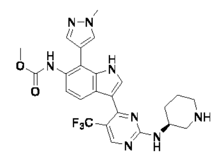
T-68

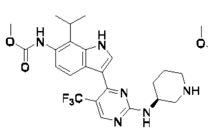
T-69

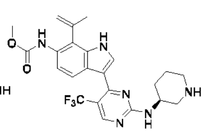
T-70

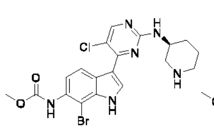
T-71

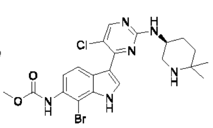
T-72

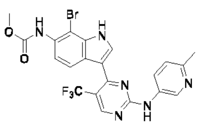
T-73

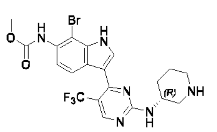
T-74

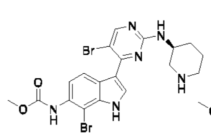
T-75

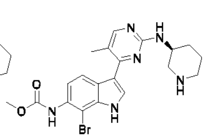
T-76

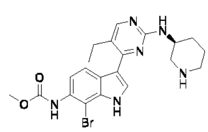
T-77

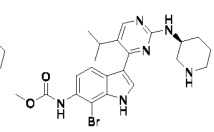
T-78

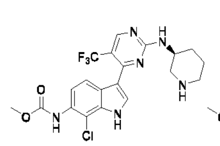
T-79

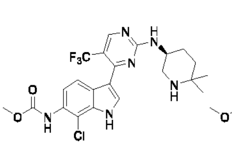
T-80

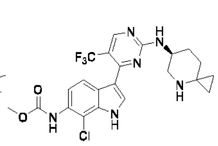
T-81

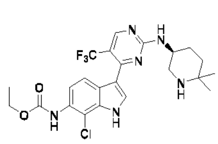
T-82

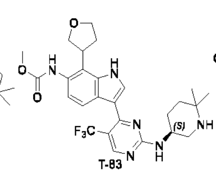
T-83

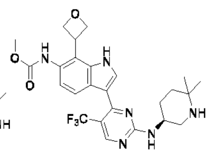
T-84

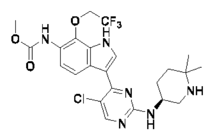
T-85

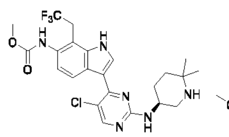
T-86

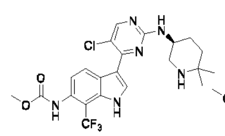
T-87

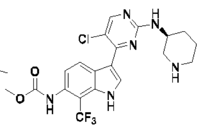
T-88

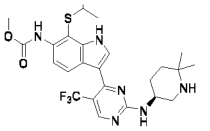
T-89

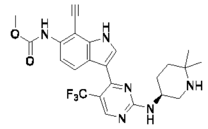
T-90

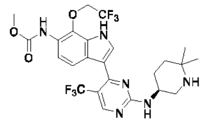
T-91

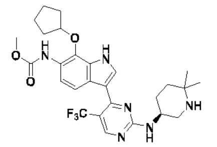
T-92

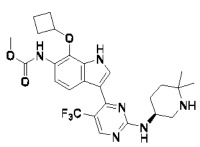
T-93

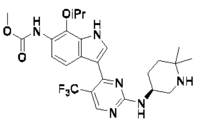
T-94

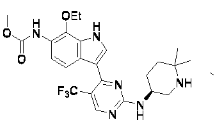
T-95

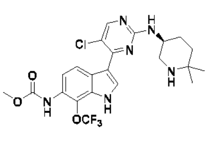
T-96

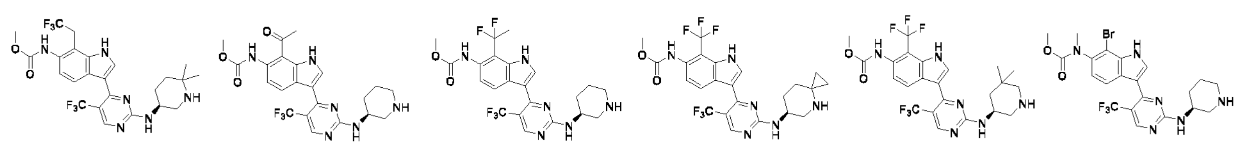
T-97 T-98 T-99 T-100 T-101 T-102

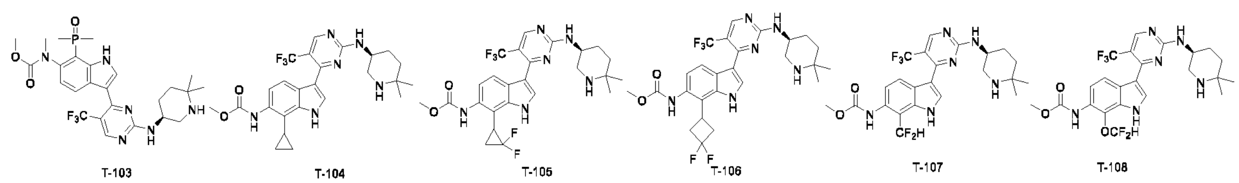
T-103 T-104 T-105 T-106 T-107 T-108

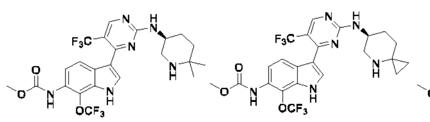
T-109 T-110
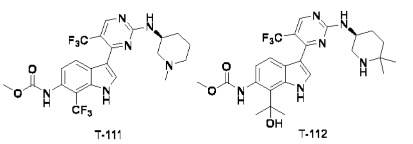
T-111 T-112
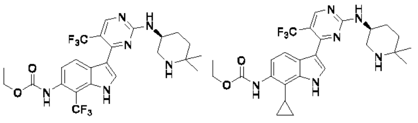
T-113 T-114

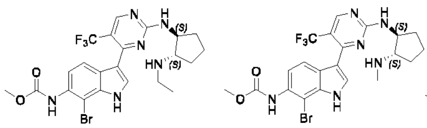
T-115 T-116
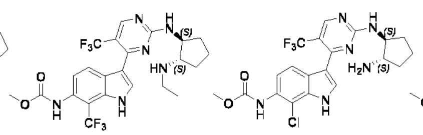
T-117 T-118
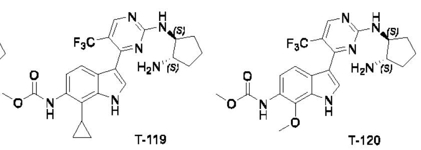
T-119 T-120

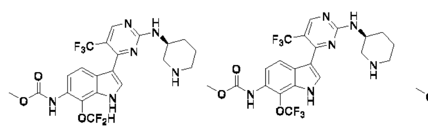
T-121 T-122
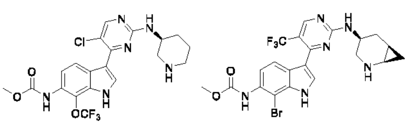
T-123 T-124
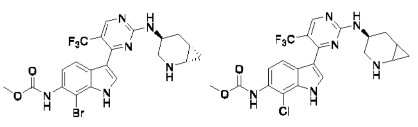
T-125 T-126

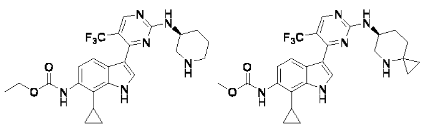
T-127 T-128
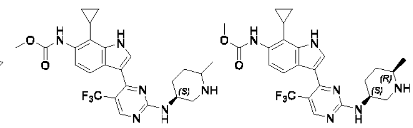
T-129 T-130
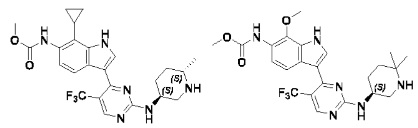
T-131 T-132

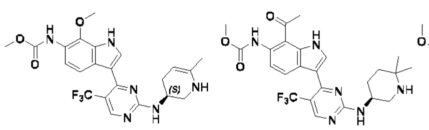
T-133 T-134
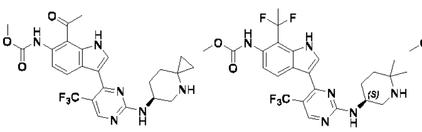
T-135 T-136
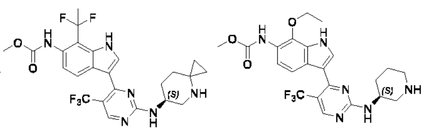
T-137 T-138

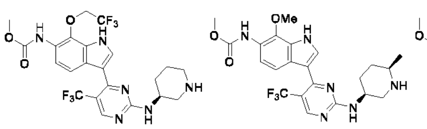
T-139 T-140
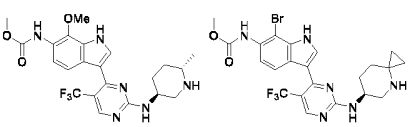
T-141 T-142
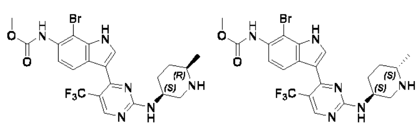
T-143 T-144

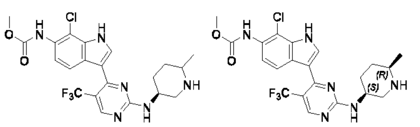
T-145 T-146
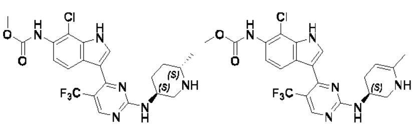
T-147 T-148
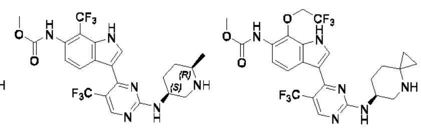
T-149 T-150

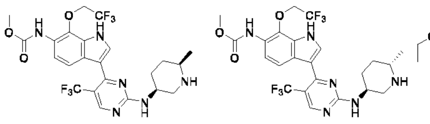
T-151 T-152
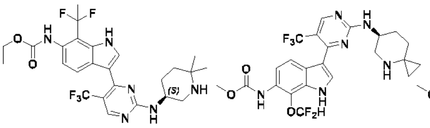
T-153 T-154
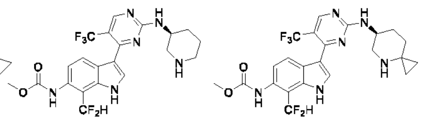
T-155 T-156

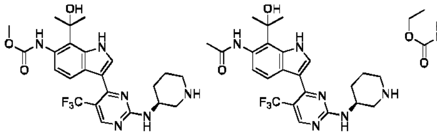
T-157 T-158
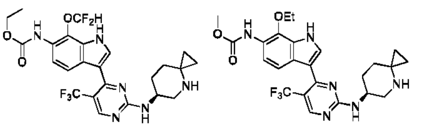
T-159 T-160
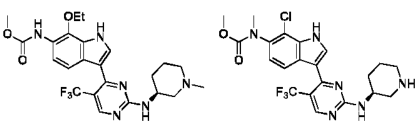
T-161 T-162

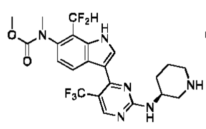
T-163

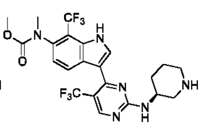
T-164

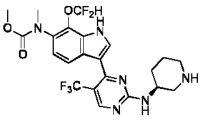
T-165

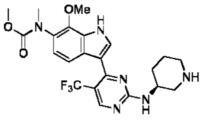
T-166

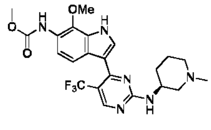
T-167

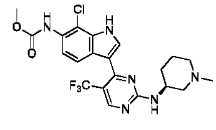
T-168

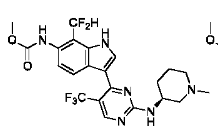
T-169

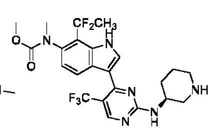
T-170

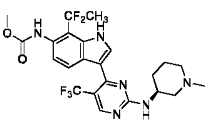
T-171

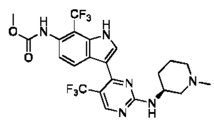
T-172

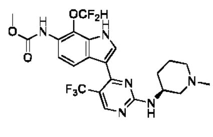
T-173

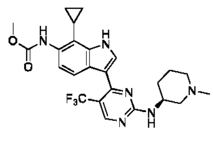
T-174

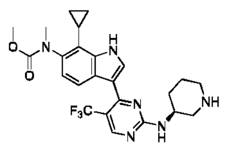
T-175

.

**8.** The compound of claim 1, wherein the pharmaceutically acceptable salt is an inorganic acid salt or an organic acid salt;

the inorganic acid salt is selected from the group consisting of hydrochloride, hydrobromide, hydroiodate, sulfate, bisulfate, nitrate, phosphate, and acid phosphate;
the organic acid salt is selected from the group consisting of formate, acetate, trifluoroacetate, propionate, pyruvate, hydroxyacetate, oxalate, malonate, fumarate, maleate, lactate, malate, citrate, tartrate, methanesulfonate, ethanesulfonate, benzene sulfonate, p-toluene sulfonate, salicylate, picrate, glutamate, ascorbate, camphorate, and camphor sulfonate.

**9.** A pharmaceutical composition comprising a prophylactically and/or therapeutically effective amount of the compound of claim 1 and a pharmaceutically acceptable carrier.

**10.** Use of the compound of claim 1 for the preparation of a medicament for modulating CDK7 kinase activity or for the prevention and/or treatment of CDK7-related diseases.

**11.** The use of claim 10, wherein the CDK7-related disease is selected from the group consisting of inflammation, cancer, cardiovascular disease, infection, immune disease, and metabolic disease.

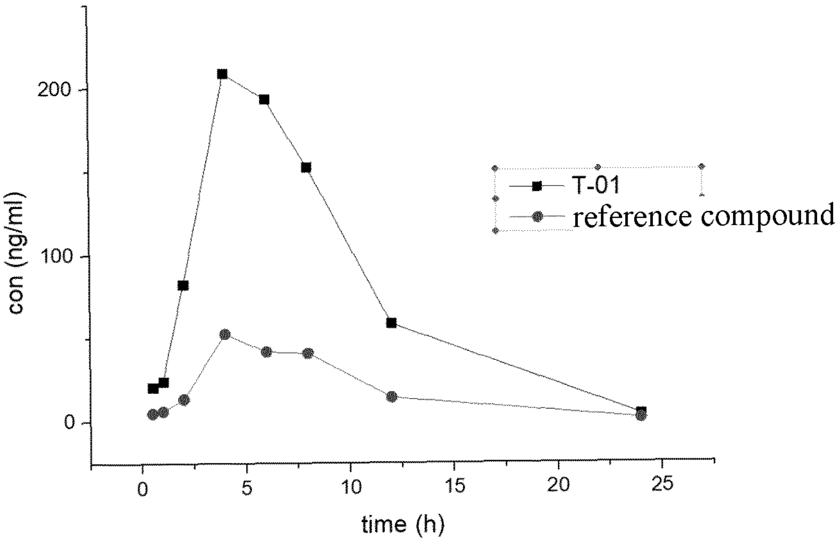


Figure 1

## INTERNATIONAL SEARCH REPORT

International application No.
**PCT/CN2021/108429**

**A. CLASSIFICATION OF SUBJECT MATTER**

C07D 401/14(2006.01)i； C07D 403/14(2006.01)i； C07D 413/14(2006.01)i； C07D 487/04(2006.01)i； C07F 9/6558(2006.01)i； C07F 9/6561(2006.01)i； C07F 9/6568(2006.01)i； C07F 9/6574(2006.01)i； A61P 3/00(2006.01)i； A61P 9/00(2006.01)i； A61P 29/00(2006.01)i； A61P 31/00(2006.01)i； A61P 35/00(2006.01)i； A61P 35/02(2006.01)i； A61P 37/02(2006.01)i； A61K 31/675(2006.01)i； A61K 31/5025(2006.01)i； A61K 31/506(2006.01)i

According to International Patent Classification (IPC) or to both national classification and IPC

**B. FIELDS SEARCHED**

Minimum documentation searched (classification system followed by classification symbols)

C07D C07F A61P A61K

Documentation searched other than minimum documentation to the extent that such documents are included in the fields searched

Electronic data base consulted during the international search (name of data base and, where practicable, search terms used)

CNABS, CNTXT, VEN, USTXT, EPTXT, WOTXT, CNKI, 万方, Web of Science, 百度学术, Registry, Caplus, Marpat, 浙江同源康医药股份有限公司, 梁阿朋, 李钧, 董胜利, 吴豫生, 李美华, 牛成山, 徐宇, 细胞周期蛋白依赖性激酶, 嘧啶, 吲哚, 吲唑, 苯并吡唑, CDK-7, cyclin-dependent kinase, pyrimidine, indole, indazole.

**C. DOCUMENTS CONSIDERED TO BE RELEVANT**

| Category* | Citation of document, with indication, where appropriate, of the relevant passages | Relevant to claim No. |
|---|---|---|
| PX | CN 112661745 A (ZHEJIANG TONGYUANKANG MEDICINE CO., LTD.) 16 April 2021 (2021-04-16)<br>paragraphs 0006-0070, 0073, 0079-0085 | 1-11 |
| PX | WO 2021087138 A1 (SYROS PHARMACEUTICALS INC.) 06 May 2021 (2021-05-06)<br>pages 1-4, page 50, description figure 1 | 1-11 |
| X | CN 110036004 A (SYROS PHARMACEUTICALS, INC.) 19 July 2019 (2019-07-19)<br>paragraphs 0048, 0071-0089, 0177, 0202-204, pages 40, 43-47, 50, 52, 157, description figure 1 | 1-11 |
| X | WO 2016058544 A1 (SYROS PHARMACEUTICALS INC. et al.) 21 April 2016 (2016-04-21)<br>paragraphs 378, 397, 435, 440, 445, 452, paragraphs 50, 72-76, 101, 127, 130-135 | 1-11 |
| X | CN 105849099 A (DANA-FARBER CANCER INSTITUTE, INC.) 10 August 2016 (2016-08-10)<br>paragraphs 951, 1020 | 1-2 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 October 2021** | **03 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/108429**

**C. DOCUMENTS CONSIDERED TO BE RELEVANT**

| Category* | Citation of document, with indication, where appropriate, of the relevant passages | Relevant to claim No. |
|---|---|---|
| X | Mahbub Alam et al. "Synthesis and SAR of aminopyrimidines as novel c-Jun N-terminal kinase (JNK) inhibitors" *Bioorganic Medicinal Chemistry Letters,* Vol. 17, No. 12, 30 March 2007 (2007-03-30), ISSN: 0960-894X, pages 3463-3467, particularly page 3465, table 1 | 1-2 |
| X | WO 2020093011 A1 (SYROS PHARMACEUTICALS INC. et al.) 07 May 2020 (2020-05-07) PP. 1-4, 20 and 30 | 1-2, 8-11 |
| A | WO 2019143719 A1 (SYROS PHARMACEUTICALS INC.) 25 July 2019 (2019-07-25) entire document | 1-11 |
| A | WO 2019143730 A1 (SYROS PHARMACEUTICALS INC.) 25 July 2019 (2019-07-25) entire document | 1-11 |
| A | CN 101723936 A (SHANGHAI GENOMICS, INC.) 09 June 2010 (2010-06-09) entire document | 1-11 |
| A | CN 111393415 A (SUZHOU XINNUOWEI PHARMACEUTICAL TECHNOLOGY CO., LTD.) 10 July 2020 (2020-07-10) entire document | 1-11 |

Form PCT/ISA/210 (second sheet) (January 2015)

**INTERNATIONAL SEARCH REPORT**
**Information on patent family members**

International application No.
**PCT/CN2021/108429**

| Patent document cited in search report | Publication date (day/month/year) | Patent family member(s) | Publication date (day/month/year) |
|---|---|---|---|
| CN 112661745 A | 16 April 2021 | None | |
| WO 2021087138 A1 | 06 May 2021 | None | |
| CN 110036004 A | 19 July 2019 | MX 2019000527 A | 12 August 2019 |
| | | KR 20190038841 A | 09 April 2019 |
| | | SG 11201900328 W A | 27 February 2019 |
| | | EP 3484871 A1 | 22 May 2019 |
| | | RU 2019103870 A | 14 August 2020 |
| | | RU 2019103870 A3 | 09 November 2020 |
| | | AU 2017295863 A1 | 07 February 2019 |
| | | BR 112019000716 A2 | 07 May 2019 |
| | | WO 2018013867 A1 | 18 January 2018 |
| | | WO 2018013867 A8 | 15 November 2018 |
| | | JP 2019527217 A | 26 September 2019 |
| | | IL 264222 D0 | 28 February 2019 |
| | | CA 3030795 A1 | 18 January 2018 |
| WO 2016058544 A1 | 21 April 2016 | US 2017327496 A1 | 16 November 2017 |
| | | US 10308648 B2 | 04 June 2019 |
| | | US 2021061803 A1 | 04 March 2021 |
| | | US 2019337940 A1 | 07 November 2019 |
| | | US 10865206 B2 | 15 December 2020 |
| CN 105849099 A | 10 August 2016 | WO 2015058140 A1 | 23 April 2015 |
| | | CA 2927920 A1 | 23 April 2015 |
| | | US 2019031642 A1 | 31 January 2019 |
| | | US 10906889 B2 | 02 February 2021 |
| | | JP 2016533379 A | 27 October 2016 |
| | | JP 6491202 B2 | 27 March 2019 |
| | | AU 2014337044 A1 | 05 May 2016 |
| | | US 2016264554 A1 | 15 September 2016 |
| | | US 10047070 B2 | 14 August 2018 |
| | | EP 3057956 A1 | 24 August 2016 |
| | | EP 3057956 B1 | 05 May 2021 |
| | | AU 2019200372 A1 | 07 February 2019 |
| | | AU 2019200372 B2 | 23 July 2020 |
| WO 2020093011 A1 | 07 May 2020 | US 2020190126 A1 | 18 June 2020 |
| | | US 10738067 B2 | 11 August 2020 |
| | | SG 11202104438V A | 28 May 2021 |
| | | IL 282737 D0 | 30 June 2021 |
| | | CA 3118324 A1 | 07 May 2020 |
| | | AU 2019374142 A1 | 27 May 2021 |
| | | AU 2019371454 A1 | 27 May 2021 |
| | | CA 3118330 A1 | 07 May 2020 |
| | | EP 3873477 A1 | 08 September 2021 |
| | | EP 3873462 A1 | 08 September 2021 |
| WO 2019143719 A1 | 25 July 2019 | AU 2019209470 A1 | 13 August 2020 |
| | | EP 3740207 A1 | 25 November 2020 |
| | | CA 3088526 A1 | 25 July 2019 |
| WO 2019143730 A1 | 25 July 2019 | CA 3088529 A1 | 25 July 2019 |
| | | AU 2019209475 A1 | 20 August 2020 |
| | | EP 3740206 A1 | 25 November 2020 |

Form PCT/ISA/210 (patent family annex) (January 2015)

**INTERNATIONAL SEARCH REPORT**
**Information on patent family members**

International application No.
**PCT/CN2021/108429**

| Patent document cited in search report | Publication date (day/month/year) | Patent family member(s) | Publication date (day/month/year) |
|---|---|---|---|
| CN 101723936 A | 09 June 2010 | CN 101723936 B | 15 January 2014 |
| | | US 2011263541 A1 | 27 October 2011 |
| | | US 8309550 B2 | 13 November 2012 |
| | | WO 2010051781 A1 | 14 May 2010 |
| CN 111393415 A | 10 July 2020 | CN 111393415 B | 20 November 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020093006 A1


### Non-patent literature cited in the description


- Handbook of Chemistry and Physics
- **THOMAS SORRELL.** Organic Chemistry. University Science Books, 1999
- **SAMBROOK et al.** Molecular Cloning: Laboratory Manual. Cold Spring Harbor Laboratory Press, 1989
- *CHEMICAL ABSTRACTS,* 1788036-28-1